# EUROPEAN PATENT APPLICATION

(11) **EP 4 389 152 A1**
(43) Date of publication of application: **26.06.2024**
(21) Application number: 22216669.6
(22) Date of filing: 23.12.2022
(51) Int. Cl.: A61K 47/68, A61P 35/00

(54) **CONJUGATES OF PBD PRODRUGS**

(71) Applicant: Synaffix B.V., 5349 AB Oss (NL)
(72) Inventor: The designation of the inventor has not yet been filed
(74) Representative: Nederlandsch Octrooibureau

(57) **Abstract**

The inventions concerns conjugates of masked PBD dimers, which are suitable in the treatment of cancers. The inventors found that coupling a masked PBD dimer via a glycan of a cell-binding agent increases the efficiency and tolerability of a PBD-payload. These findings allow for more effective cancer treatment with less adverse side-effects. The invention therefore concerns a conjugate of structure (1):

AB-[L-(D)ₓ]_{y} (1)

wherein AB is a cell-binding agent; x is 1 or 2; y is 1 or 2; D is a masked PBD dimer payload; and L is a linker that connects AB with D.

## Description

### Field of the invention

The present invention is in the field of medicine. More specifically, the present invention relates to masked pyrrolobenzodiazepine dimers and antibody-drug conjugates prepared therewith, in particular to antibody-drug conjugates for the treatment of cancer based on masked pyrrolobenzodiazepine dimers for release in the tumor microenvironment.

### Background

Antibody-drug conjugates (ADC), considered as one of the major classes of targeted therapy, are comprised of an antibody to which is attached a pharmaceutical agent. The antibodies (also known as binding agents or ligands) can be small protein formats (scFv's, Fab fragments, DARPins, Affibodies, etc.) but are generally monoclonal antibodies (mAbs) of IgG type which have been selected based on their high selectivity and affinity for a given antigen, their long circulating half-lives, and little to no immunogenicity. Thus, mAbs as ligands for a carefully selected biological receptor provide an ideal targeting platform for selective delivery of pharmaceutical drugs. For example, a monoclonal antibody known to bind selectively with a specific cancer-associated antigen can be used for delivery of a chemically conjugated cytotoxic agent to the tumour, via binding, internalization, intracellular processing and finally release of active catabolite. The cytotoxic agent may be small molecule toxin, a protein toxin or other formats, like oligonucleotides. As a result, the tumour cells can be selectively eradicated, while sparing normal cells which have not been targeted by the antibody. Similarly, chemical conjugation of an antibacterial drug (antibiotic) to an antibody can be applied for treatment of bacterial infections, while conjugates of antiinflammatory drugs are under investigation for the treatment of autoimmune diseases and for example attachment of an oligonucleotide to an antibody is a potential promising approach for the treatment of neuromuscular diseases. Hence, the concept of targeted delivery of an active pharmaceutical drug to a specific cellular location of choice is a powerful approach for the treatment of a wide range of diseases, with many beneficial aspects versus systemic delivery of the same drug.

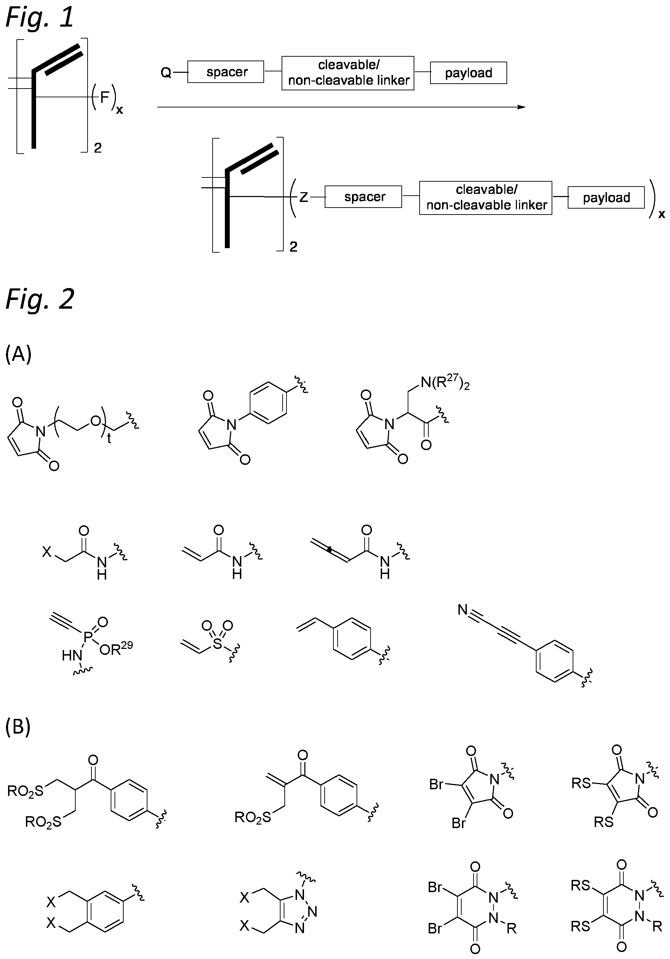

ADCs are prepared by conjugation of a linker-drug to a protein, a process known as bioconjugation. Many technologies are known for bioconjugation, as summarized in G.T. Hermanson, "Bioconjugate Techniques", Elsevier, 3rd Ed. 2013**,** incorporated by reference. Conceptually, the method the preparation of an ADC by bioconjugation entails the reaction of x number of reactive moieties F present on the antibody with a complementary reactive moiety Q present on the pharmaceutical drug (the payload), see Figure 1.

Typically, a chemical linker is present between Q and the payload. This linker needs to possess a number of key attributes, including the requirement to be stable in plasma after drug administration for an extended period of time. A stable linker enables localization of the ADC to the projected site or cells in the body and prevents premature release of the payload in circulation, which would indiscriminately induce undesired biological response of all kinds, thereby lowering the therapeutic index of the ADC. Upon internalization, the ADC should be processed such that the payload is effectively released so it can exert its mode-of-action inside the cell. The linker can also contain a spacer element. There are two families of linkers, non-cleavable and cleavable. Non-cleavable linkers consist of a chain of atoms between the antibody and the payload, which is fully stable under physiological conditions, irrespective of which organ or biological compartment the antibody-drug conjugate resides in. As a consequence, liberation of the payload from an ADC with a non-cleavable linker relies on the complete (lysosomal) degradation of the antibody after internalization of the ADC into a cell. As a result of this degradation, the payload will be released, still carrying the linker, as well as a peptide fragment and/or the amino acid from the antibody the linker was originally attached to. Cleavable linkers utilize an inherent property of a cell or a cellular compartment for selective release of the payload from the ADC, which generally leaves no trace of linker after processing. For cleavable linkers, there are three commonly used mechanisms: (1) susceptibility to specific enzymes, (2) pH-sensitivity, and (3) sensitivity to redox state of a cell (or its microenvironment). The cleavable linker may also contain a self-immolative unit, for example based on a para-aminobenzyl alcohol group and derivatives thereof. A linker may also contain an additional element, often referred to as spacer or stretcher unit, to connect the linker with a reactive group for attachment to the antibody via a reactive moiety F present on the antibody.

The reactive moiety F can be naturally present in the antibody, for example the reactive moiety can be the side chain of lysine or cysteine, which can be employed for acylation (lysine side chain) or alkylation (cysteine side chain).

Acylation of the e-amino group in a lysine side-chain is typically achieved by subjecting the protein to a reagent based on an activated ester or activated carbonate derivative, for example SMCC is applied for the manufacturing of Kadcyla^{®}.

Various reagents are known for alkylation of the thiol group in cysteine side-chain, see Figure 2. Amongst the cysteine alkylation strategies, the vast majority is based on the use of maleimide reagents, as is for example applied in the manufacturing of Adcetris^{®}, Polivy^{®} and Padcev^{®}. Besides standard maleimide reagents, a range of maleimide variants are also applied for more stable cysteine conjugation, as for example demonstrated by James Christie et al., J. Contr. Rel. 2015, 220, 660-670 and Lyon et al., Nat. Biotechnol. 2014, 32, 1059-1062, both incorporated by reference. Other approaches for cysteine alkylation involve for example nucleophilic substitution of haloacetamides (typically bromoacetamide or iodoacetamide), see for example Alley et al., Bioconj. Chem. 2008, 19, 759-765, incorporated by reference, or various approaches based on nucleophilic addition on unsaturated bonds, such as reaction with acrylate reagents, see for example Bernardim et al., Nat. Commun. 2016, 7, 13128 and Ariyasu et al., Bioconj. Chem. 2017, 28, 897-902, both incorporated by reference, reaction with phosphonamidates, see for example Kasper et al., Angew. Chem. Int. Ed. 2019, 58, 11625-11630, incorporated by reference, reaction with allenamides, see for example Abbas et al., Angew. Chem. Int. Ed. 2014, 53, 7491-7494, incorporated by reference, reaction with cyanoethynyl reagents, see for example Kolodych et al., Bioconj. Chem. 2015, 26, 197-200, incorporated by reference, reaction with vinylsulfones, see for example Gil de Montes et al., Chem. Sci. 2019, 10, 4515-4522, incorporated by reference, or reaction with vinylpyridines, see for example Seki et al, Chem. Sci., 2021, 12, 9060-9068 and https://iksuda.com/science/permalink/ (accessed July 26^{th}, 2020). An alternative approach to antibody conjugation without reengineering of antibody involves the reduction of interchain disulfide bridges, followed addition of a payload attached to a cysteine cross-linking reagent, such as bis-sulfone reagents, see for example Balan et al., Bioconj. Chem. 2007, 18, 61-76 and Bryant et al., Mol. Pharmaceutics 2015, 12, 1872-1879, both incorporated by reference, mono- or bis-bromomaleimides, see for example Smith et al., J. Am. Chem. Soc. 2010, 132, 1960-1965 and Schumacher et al., Org. Biomol. Chem. 2014, 37, 7261-7269, both incorporated by reference, bis-maleimide reagents, see for example WO2014114207, bis(phenylthio)maleimides, see for example Schumacher et al., Org. Biomol. Chem. 2014, 37, 7261-7269 and Aubrey et al., Bioconj. Chem. 2018, 29, 3516-3521, both incorporated by reference, bis-bromopyridazinediones, see for example Robinson et al., RSC Advances 2017, 7, 9073-9077, incorporated by reference, bis(halomethyl)benzenes, see for example Ramos-Tomillero et al., Bioconj. Chem. 2018, 29, 1199-1208, incorporated by reference or other bis(halomethyl)aromatics, see for example WO2013173391. Typically, ADCs prepared by cross-linking of cysteines have a drug-to-antibody loading of ~4 (DAR4). Another useful technology for conjugation to a cysteine side chain is by means of disulfide bonds, a bioactivatable connection that has been utilized for reversibly connecting protein toxins, chemotherapeutic drugs, and probes to carrier molecules (see for example Pillow et al., Chem. Sci. 2017, 8, 366-370, incorporated by reference).

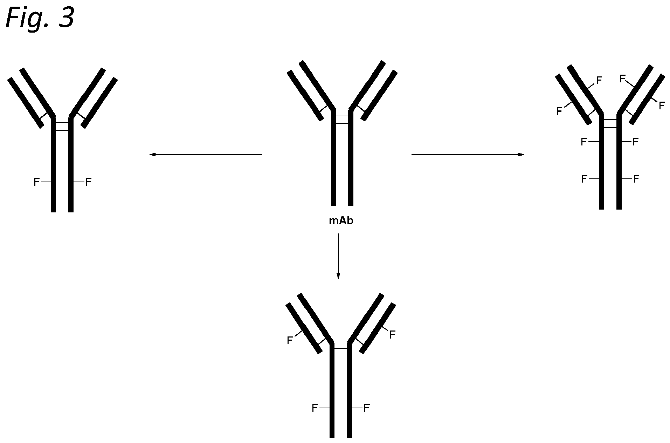

Besides conjugation to the side chains of the naturally present amino acids lysine or cysteine, a range of other conjugation technologies has been explored based on a two-stage strategy involving (a) introduction on a novel reactive group F, followed by (b) reaction with another complementary reactive group Q. For example, a method can be used to introduce a given number of reactive moieties F onto an antibody, which can be two, four or eight, see Figure 3.

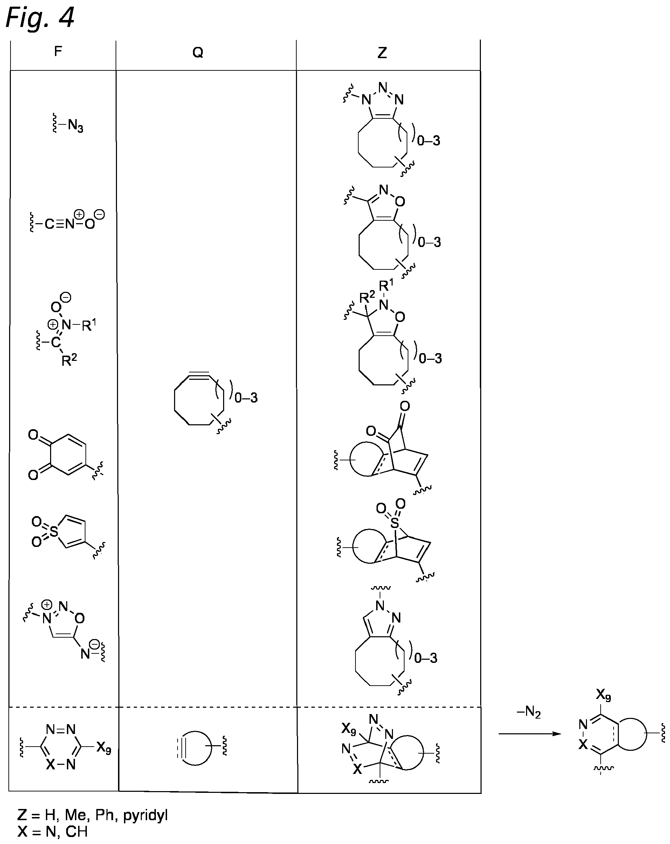

An example of an unnatural reactive functionality F that can be employed for bioconjugation of linker-drugs is the oxime group, suitable for oxime ligation or the azido group, suitable for click chemistry conjugation. The oxime can be installed in the antibody by genetic encoding of a non-natural amino acid, e.g. p-acetophenylalanine, as for example demonstrated by Axup et al. Proc. Nat. Acad. Sci. 2012, 109, 16101-16106, incorporated by reference, or by enzymatic alkylation of a cysteine present in a CAAX sequence with a prenyl group containing a remote keto group, as for example disclosed in WO2012153193. The azide can be installed in the antibody by genetic encoding of p-azidomethylphenylalanine or p-azidophenylalanine, as for example demonstrated by Axup et al. Proc. Nat. Acad. Sci. 2012, 109, 16101-16106, incorporated by reference. Similarly, Zimmerman et al., Bioconj. Chem. 2014, 25, 351-361, incorporated by reference have employed a cell-free protein synthesis method to introduce p-azidomethylphenylalanine (AzPhe) into monoclonal antibodies for conversion into ADCs by means of metal-free click chemistry. Also, it has also be shown by Nairn et al., Bioconj. Chem. 2012, 23, 2087-2097, incorporated by reference, that a methionine analogue like azidohomoalanine (Aha) can be introduced into protein by means of auxotrophic bacteria and further converted into protein conjugates by means of click chemistry. Finally, genetic encoding of aliphatic azides in recombinant proteins using a pyrrolysyl-tRNA synthetase/tRNA_{CUA} pair was shown by Nguyen et al., J. Am. Chem. Soc. 2009, 131, 8720-8721, incorporated by reference, and labelling was achieved by click chemistry, either by copper-catalyzed alkyne-azide cycloaddition (CuAAC) or strain-promoted alkyne-azide cycloaddition (SPAAC). Besides, CuAAC and SPAAC, bioconjugation of linker-drugs to antibodies (and other biomolecules such as glycans, nucleic acids) can be achieved by a range of other metal-free click chemistries, see e.g. Nguyen and Prescher, Nature Rev. Chem. 2020, 4, 476-489, incorporated by reference. For example, oxidation of a specific tyrosine in a protein can give an ortho-quinone, which readily undergoes cycloaddition with strained alkenes (e.g. TCO) or strained alkynes, see e.g. Bruins et al., Chem. Eur. J. 2017, 24, 4749-4756, incorporated by reference. Besides cyclooctyne, certain cycloheptynes are also suitable for metal-free click chemistry, as reported by Wetering et al. Chem. Sci. 2020, 11, 9011-9016, incorporated by reference. A tetrazine moiety can also be introduced into a protein or a glycan by various means, for example by genetic encoding or chemical acylation, and may also undergo cycloaddition with cyclic alkenes and alkynes. A list of pairs of functional groups F and Q for metal-free click chemistry is provided in Figure 4.

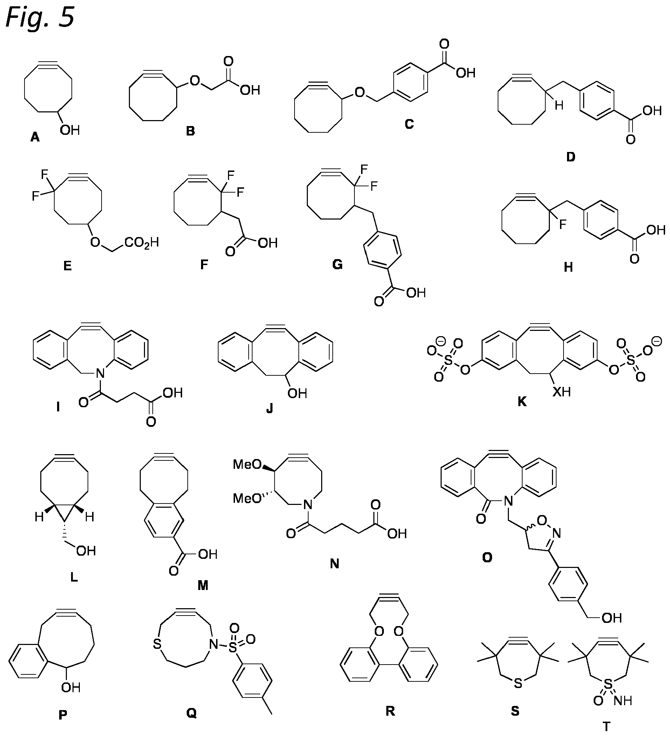

In a SPAAC bioconjugation, the linker-drug is functionalized with a cyclic alkyne and the cycloaddition with azido-modified antibody is driven by relief of ring-strain. Conversely, the linker-drug can be functionalized with azide and the antibody with cyclic alkyne. Various strained alkynes suitable for metal-free click chemistry are indicated in Figure 5.

A method of increasing popularity in the field of ADCs is based on enzymatic installation of a non-natural functionality F. For example, Lhospice et al., Mol. Pharmaceut. 2015, 12, 1863-1871, incorporated by reference, employ the bacterial enzyme transglutaminase (BTG or TGase) for installation of an azide moiety onto an antibody. A genetic method based on C-terminal TGase-mediated azide introduction followed by conversion in ADC with metal-free click chemistry was reported by Cheng et al., Mol. Cancer Therap. 2018, 17, 2665-2675, incorporated by reference.

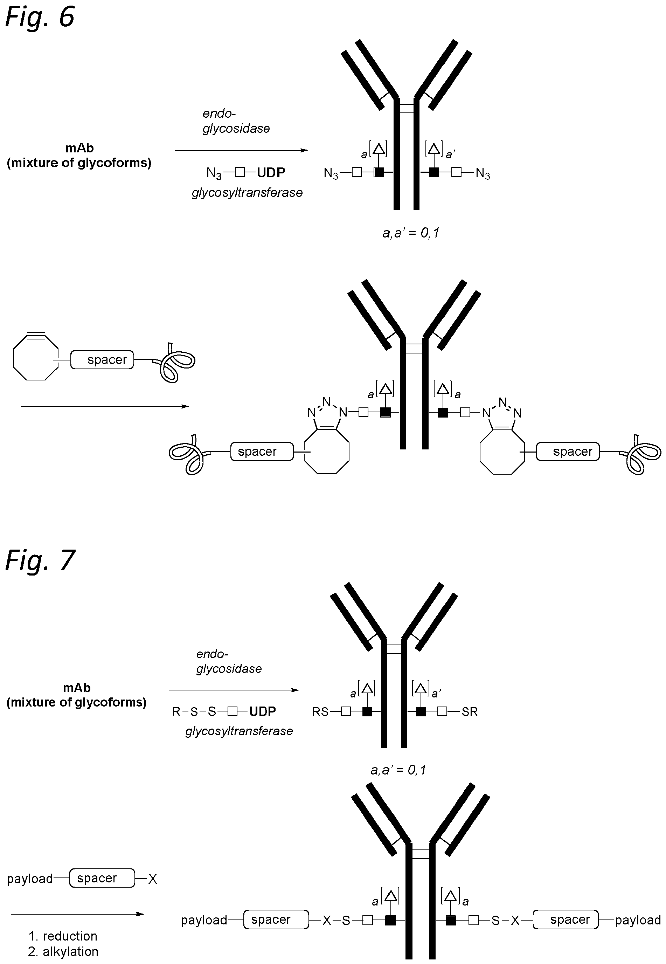

It has been shown in WO2014065661, by van Geel et al., Bioconj. Chem. 2015, 26, 2233-2242, Verkade et al., Antibodies 2018, 7, 12, and Wijdeven at al. MAbs 2022, 14, 2078466, all incorporated by reference, that enzymatic remodelling of the native antibody glycan at N297 enables introduction of an azido-modified sugar, suitable for attachment of cytotoxic payload using metal-free click chemistry, see Figure 6. Similarly, the enzymatic glycan remodelling protocol can also be employed to install a free thiol group on an antibody (see Figure 7) for conjugation based on any of the methods described above for cysteine conjugation.

Although ADCs have demonstrated clinical and preclinical activity, it has been unclear what factors determine such potency in addition to antigen expression on targeted tumour cells. For example, drug:antibody ratio (DAR), ADC-binding affinity, potency of the payload, receptor expression level, internalization rate, trafficking, multiple drug resistance (MDR) status, and other factors have all been implicated to influence the outcome of ADC treatment in vitro. In addition to the direct killing of antigen-positive tumour cells, ADCs also have the capacity to kill adjacent antigen-negative tumour cells: the so-called "bystander killing" effect, as originally reported by Sahin et al, Cancer Res. 1990, 50, 6944-6948, incorporated by reference, and for example studied by Li et al, Cancer Res. 2016, 76, 2710-2719, incorporated by reference. Generally spoken, cytotoxic payloads that are neutral will show bystander killing whereas ionic (charged) payloads do not, as a consequence of the fact that ionic species do not readily pass a cellular membrane by passive diffusion. Payloads with established bystander effect are for example MMAE and DXd. Examples of payloads that do not show bystander killing are MMAF or the active catabolite of Kadcyla^{®} (lysine-MCC-DM1).

Currently, cytotoxic payloads include for example microtubule-disrupting agents [e.g. auristatins such as monomethyl auristatin E (MMAE) and monomethyl auristatin F (MMAF), maytansinoids such as DM1 and DM4, tubulysins], DNA-damaging agents [e.g., calicheamicin, pyrrolobenzodiazepine (PBD) dimers, indolinobenzodiapine dimers, duocarmycins, anthracyclines such as PNU-159,682, topoisomerase inhibitors [e.g. DXd, exatecan, SN-38] or RNA polymerase II inhibitors [e.g. amanitin]. ADCs that have reached market approval include for example payloads MMAE, MMAF, DM1, calicheamicin, SN-38, DXd and a PBD dimer, while a BLA has been filed for an ADC based on DM4 and a pivotal trials is running for an ADC based on duocarmycin. A larger variety of payloads is still under clinical evaluation or has been in clinical trials in the past, e.g. eribulin, indolinobenzodiazepine dimer, PNU-159,682, amanitin, hemi-asterlin, doxorubicin, vinca alkaloids and others. Finally, various ADCs in early clinical or late-stage preclinical stage are conjugated to novel payloads for example, KSP inhibitors, MMAD, cryptophycins, and others.

With the exception of sacituzumab govetican (Trodelvy^{®}), all of the clinical and marketed ADCs contain cytotoxic drugs that are not suitable as stand-alone drug. Trodelvy^{®} is the exception because it features SN-38 as cytotoxic payload, which is also the active catabolite of irinotecan (an SN-38 prodrug). Several other payloads now used in clinical ADCs have been initially evaluated for chemotherapy as free drug, for example calicheamicin, PBD dimers and eribulin. but have failed because the extremely high potency of the cytotoxin (picomolar to low nanomolar IC₅₀ values) versus the typically low micromolar potency of standard chemotherapy drugs, such as paclitaxel and doxorubicin.

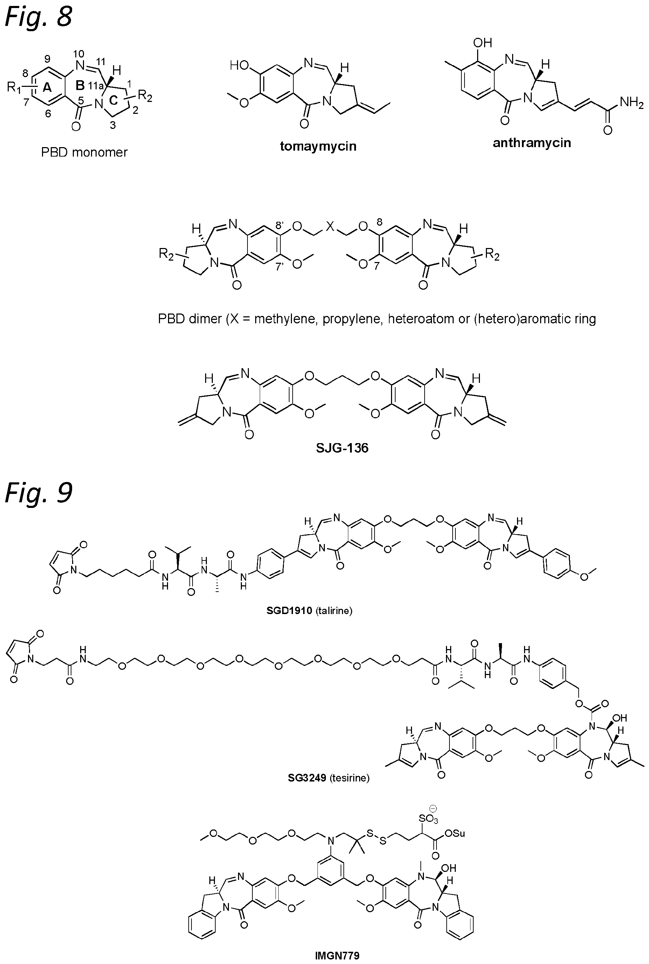

A cytotoxin that has received extensive interest for application in ADCs inspired by payloads based on pyrrolobenzodiazepine (PBD) structure (see Figure 8). Discovered in the 1960s in cultures of *Streptomyces* species (e.g. anthramycin and tomaymycin), the pyrrolobenzodiazepines are an important class of sequence-selective DNA-interactive agents that bind covalently to guanine bases within the minor groove of DNA. PBD molecules have a chiral center at their C11a(S)- position, which provides them with an appropriate 3-dimensional shape to fit perfectly within the DNA minor groove. They also possess an electrophilic N10-C11 moiety (i.e., interconvertible imine, carbinolamine, or carbinolamine methyl ether functionalities) that can form a reversible covalent aminal linkage between their C11-position and the nucleophilic C2-NH₂ group of a guanine base. The PBD monomers are remarkable in possessing a 3-dimensional shape that allows them to fit perfectly within the minor groove of DNA, partly due to the longitudinal twist created by the chiral center at their C11a-position. Once located in a position of low energy in the groove (i.e. a preferred DNA sequence), largely dictated by substituents in the A- and C-rings, the electrophilic C11-position then alkylates the C2-NH₂ group of an adjacent guanine base, thus producing a robust covalent adduct capable of blocking biological processes such as transcription factor binding and RNA polymerase progression.

Besides PBD monomers, it has been found that the chemical covalent linking of two PBD monomers gives a PBD dimer that possesses significantly enhanced cytotoxicity, see for example Gregson et al. J. Med. Chem. 2001, 44, 737 - 748, incorporated by reference. The first C7- and C8-linked examples were designed to span greater lengths of DNA than the PBD monomers, to have enhanced sequence-selectivity, and to form DNA cross-links that might be more difficult for tumor cells to repair. It is now known that PBD dimers can form both interstrand and intrastrand cross- links, as well as monoadducts under certain conditions, although the interstrand cross-linked adduct is still thought to be the most toxic in cells. One PBD dimer, SJG-136, was evaluated as a standalone agent in a phase II clinical trials in patients with leukemia or ovarian cancer, but was later discontinued.

It became apparent that the PBD dimers are ideal candidates as the cytotoxic component of an ADC. The number of potential chemical linking positions in PBDs offers great flexibility for antibody connection, and this is apparent in two PBD drug-linker molecules **SGD1910** (talirine) and **SG3249** (tesirine) which feature in numerous ADCs being evaluated in clinical trials. **SGD1910** is linked via the C2 position of the PBD and **SG3249** via the N10 position, both via a dipeptide (valine-alanine) trigger that is cleaved by cathepsin in the lysosome. A self-immolative PABC spacer is necessary to liberate the bis-imine DNA cross-linking PBD dimer form the hemi-aminal precursor upon catabolism of the ADC, while the PEG group in **SG3249** aids aqueous solubility. Moreover, both **SGD1910** and **SG3249** possess a terminal maleimide function to enable conjugation to an antibody by reaction with the cysteine side-chain thiol. A benzoannulated version of PBD, known as indolinobenzodiazepine (IBD), as reported by Reid et al., ACS Med. Chem. Lett. 2019, 10, 1193-1197, incorporated by reference, is linked to the antibody through an aryl tether in the IBD and is also in clinical development as an ADC drug-linker **(IMGN779).** Again, the linkage in **IMGN779** is cleavable (disulfide trigger) but in this case the payload is linked to the antibody through a lysine connection. Various other structural analogues of PBD dimers have also been developed over the years, including for example isoindolinopyrrolobenzodiazepine (IQB), as reported by Smith et al. ACS Med. Chem. Lett. 2018, 9, 56-60, incorporated by reference. A comprehensive review on PBD dimers was published by Mantaj et al., Angew. Chem. Int Ed. 2017, 56, 462-488, incorporated by reference..

Currently, there is one approved ADC with a PBD dimer payload (Zynlonta^{™}). Zynlonta^{™} has **SG3249**/tesirine as linker-payload, which is also the case of at least three other ADCs in development by ADC Therapeutics (ADCT-301, ADCT-602, ADCT-901) as well as by others, such as MT-8633, TR1801-ADC in development by Tanabe/Medimmune. A derivative of **SG3249**/tesirine is also in clinical development by ADC Therapeutics (ADCT-601), which is attached to the antibody not by maleimide conjugation, but by metal-free click conjugation to the antibody glycan (GlycoConnect^{™}) technology, as disclosed by Zammarchi et al. Mol. Cane. Ther. 2022, 21, 582-593, incorporated by reference. It must be noted that a large number of PBD dimer-based ADCs have entered the clinic in the past 10 years but have now been discontinued, such as ABBV-176, SC16LD6.5 (Rova-T), SC-002, SC-003, SC-004, SC-006, MED17247, RG6109 (DCLL9718S) and RG6148 (DHES0815A). One reason underlining the large number of discontinuations lies in the high potency of the PBD dimer, which leads to significant adverse events in patients, including oedema and effusion, as summarized by J.A. Hartley, Exp. Opin. Biol. Ther. 2020, DOI: 10.1080/14712598.2020.1776255, incorporated by reference.

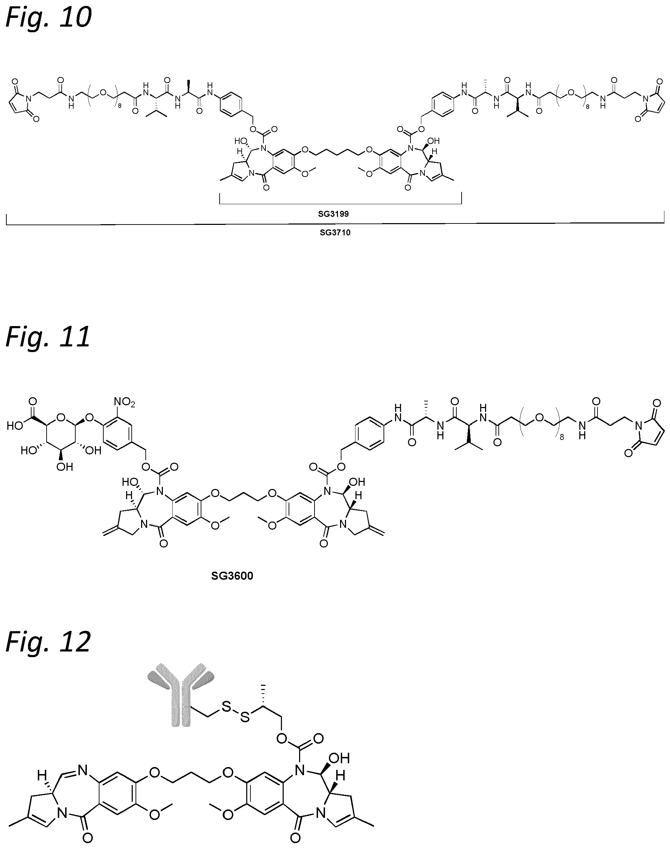

One approach to reduce the toxicity of PBD dimers for ADC application is by lowering the drug loading of the antibody for example a DAR1 format with the same payload could be preferable, as the MTD versus the similar DAR2 version will likely be two times higher. Ruddle et al., ChemMedChem 2019, 14, 1185-1195 have recently shown that DAR1 conjugates can be prepared from antibody Fab fragments by installing a maleimide-based linker attached to a Val-Ala-PABC fragment on both PBD monomer fragments of the PBD dimer (see Figure 10), thereby generating a symmetrical PBD dimer for concomitant reaction with two reduced interchain cysteines in the FAB. The resulting DAR1-type Fab fragments were shown to be highly homogeneous, stable in serum and show excellent cytotoxicity. In a follow-up publication, White et al., MAbs 2019, 11, 500-515, and also in WO2019034764, incorporated by reference, it was shown that DAR1 conjugates can also be prepared from full IgG antibodies using the same bis-maleimide functionalized PBD dimer (Figure 10, Flexmab technology). It was shown that the Flexmab-derived DAR1 ADCs was highly resistant to payload loss in serum and exhibited potent antitumor activity in a HER2-positive gastric carcinoma xenograft model. Moreover, this ADC was tolerated in rats at twice the dose compared to a site-specific DAR2 ADC prepared using a single maleimide-containing PBD dimer.

To reduce the toxicity of PBD dimer-based ADCs and to improve the therapeutic window, next-generation PBD drug-linker design has also focused on the use of lower-potency PBDs and/or the inclusion of additional tumor-selective triggers onto the PBD dimer. For example, while ignored for many years **SJG-136** is now applied also for ADCs, rather than the active catabolite **SG3199,** which is released upon proteolytic degradation of **SG3149.** In addition to decreasing the potency, the DNA-reactive imine that is normally remaining in the ADC after conjugation, it can also be charged with an additional capping moiety, such as for example a b-glucuronidase-cleavable trigger, a peptide-based trigger or a disulfide-based trigger.

b-Glucuronidase is an enzyme that plays a role in the breakdown of endogenous glucuronic acid-containing glycosaminoglycans and is normally localized to the lysosome of cells, including in the lysosome of first-pass tissues such as the liver and intestine. b-Glucuronidase concentrations in many solid tumors, including lung, breast, and gastrointestinal cancers, as well as in the tumor microenvironment, are reported to be higher than those in normal tissues, and the enzyme is not found in the general circulation. In addition, these enzyme levels are elevated in necrotic regions of tumors due to release of the lysosomal enzyme into the extracellular region from dying cells. Lysosomal concentrations of b-glucuronidase are also high in inflammatory immune cells, including neutrophils and eosinophils, and these cells can release the enzyme at sites of inflammation, such as the necrotic regions of human tumors. Exploitation of b-glucuronidase overexpression in certain tumors is a well-known approach in oncology research, where b-glucuronidase-cleavable triggers have been applied to anthracyclines, auristatins, duocarmycin and camptothecins, as well as in a multi-compound releasing prodrug approach, PBD prodrugs, and ADCs. For example Gregson et al., Eur. J. Med. Chem. 2019, 179, 591-607 have disclosed an asymmetric PBD dimer linked via Val-Ala-PABC element to the antibody (conjugated via maleimide) and a b-glucuronidase-cleavable trigger element on the other PBD monomer (Figure 11). Similar masked PBD dimers based on glucuronic acid have also been disclosed in for example US20220218830 and WO2020141923.

Other enzymes that are upregulated in the tumor microenvironment have also been considered for release of cytotoxic payloads including cathepsins, matrix metalloproteinases (MMPs), legumain and serine protease elastase, as for example summarized by M. Poreba, FEBS J., 2020, 287, 1936-1969. Investigation into these two TME-related enzymes has led to the development of cleavable linkers based on specific peptide sequences Val-Cit or Val-Ala for cathepsins, Pro-Leu-Gly (PLG) for metalloproteinases (MMPs), Asn-Asn, Ala-Asn or Asn-Ala for legumain and Asn-Pro-Val (NPV) for serine protease elastase, for example.

Finally, it is also known in the art that disulfide-based linkers can be employed as selective triggers to release the active catabolite from ADCs, including PBD dimer payloads (see Figure 12), by employing the fact that the cytoplasm is significantly more reducing than extracellular environment.

### Summary of the invention

The inventors have surprisingly found that the coupling a masked PBD dimer via a glycan of a cell-binding agent increases the efficiency and/or tolerability of a PBD-payload. These findings allow for more effective cancer treatment with less adverse side-effects.

The invention first and foremost concerns an antibody-drug conjugate, having structure (1):

AB-[L-(D)ₓ]_{y} (1)

wherein
- AB is a cell-binding agent;
- x is 1 or 2;
- y is 1 or 2;
- D is a masked PBD dimer payload;
- L is a linker that connects AB to D, wherein L comprises at least one -L⁶-Z- fragment, wherein Z¹ is a connecting group comprising the product of a cycloaddition reaction, and wherein L⁶ is -GIcNAc(Fuc)_{w}-(G)ⱼ-S-(L⁷)_{W}-, wherein L⁶ is connected to AB via GIcNAc(Fuc)_{w} and:
   o G is a monosaccharide,
   o j is an integer in the range of 0 - 6,
   o S is a sugar or a sugar derivative,
   o GicNAc is N-acetylglucosamine,
   o Fuc is fucose,
   o w is 0 or 1,
   o w' is 0 or 1, and
   o L⁷ is -N(H)C(O)CH₂-, -N(H)C(O)CF₂- or -CH₂-.

The invention further concerns a process for the synthesis of the antibody-drug conjugate according to the invention, a linker-drug construct which is suitable to be used in the process according to the invention, the medical use of the antibody-drug conjugate according to the invention and a pharmaceutical composition comprising the antibody-drug conjugate according to the invention.

### Description of the figures

Figure 1 shows the general scheme for preparation of antibody-drug conjugates by reaction of a monoclonal antibody (in most cases a symmetrical dimer) containing an x number of functionalities F. By incubation of antibody-(F)ₓ with excess of a linker-drug construct (Q-spacer-linker-payload) a conjugate is obtained by reaction of F with Q, forming connecting group Z.
Figure 2 depicts a range of reagents suitable for reaction with cysteine side-chains. Reagents may be monoalkylation type (A) or may be a cross-linker (B) for reaction with two cysteine side-chains.
Figure 3 shows the general process for non-genetic conversion of a monoclonal antibody (mAb) into an antibody containing probes for click conjugation (F). The click probe may be on various positions in the antibody, depending on the technology employed. For example, the antibody may be converted into an antibody containing two click probes (structure on the left) or four click probes (bottom structure) or eight probes (structure on the right) for click conjugation.
Figure 4 shows a representative (but not comprehensive) set of functional groups (F) that can be introduced into an antibody by engineering, by chemical modification, or by enzymatic means, which upon metal-free click reaction with a complementary reactive group Q lead to connecting group Z. Functional group F may be artificially introduced (engineered) into an antibody at any position of choice. Some functional groups F (e.g. nitrile oxide, quinone), may besides strained alkynes also react with strained alkenes, which as an example is depicted for triazine or tetrazine (bottom line). The pyridine or pyridazine connecting group is the product of the rearrangement of the tetrazabicyclo[2.2.2]octane connecting group, formed upon reaction of triazine or tetrazine with alkyne (but not alkene), respectively, with loss of N₂. Connecting groups Z depicted in Figure 4 are preferred connecting groups to be used in the present invention.
Figure 5 shows cyclic alkynes suitable for metal-free click chemistry, and preferred embodiments for reactive moiety Q. The list is not comprehensive, for example alkynes can be further activated by fluorination, by substitution of the aromatic rings or by introduction of heteroatoms in the aromatic ring.
Figure 6 depicts a specific example of site-specific conjugation of a payload based on glycan remodeling of a full-length IgG followed by azide-cyclooctyne click chemistry. The IgG is first enzymatically remodeled by endoglycosidase-mediated trimming of all different glycoforms, followed by glycosyltransferase-mediated transfer of azido-sugar onto the core GlcNAc liberated by endoglycosidase. In the next step, the azido-remodeled IgG is subjected to an immune cell-engaging polypeptide, which has been modified with a single cyclooctyne for metal-free click chemistry (SPAAC), leading to a bispecific antibody of 2:2 molecular format. It is also depicted that the cyclooctyne-polypeptide construct will have a specific spacer between cyclooctyne and polypeptide, which enables tailoring of IgG-polypeptide distance or impart other properties onto the resulting bispecific antibody.
Figure 7 depicts a specific example of site-specific conjugation of a payload based on glycan remodeling of a full-length IgG followed by thiol alkylation chemistry. The IgG is first enzymatically remodeled by endoglycosidase-mediated trimming of all different glycoforms, followed by glycosyltransferase-mediated transfer of a thiol-modified (and disulfide-protected) sugar derivative onto the core GIcNAc liberated by endoglycosidase. In the next step, the remodeled IgG is subjected to reduction (to convert the disulfide into thiol), potentially followed by oxidation, then reaction with a payload modified with a suitable thiol-reactive reagent.
Figure 8 depicts the general structure of a pyrrolobenzodiazepine (PBD) monomer (with carbon numbering) and a typical structure of a PBD dimer, as well as the specific chemical structures of tomaymycin, anthramycin and **SJG-136.**
Figure 9 shows the structures of **SGD1910** (talirine), **SG3149** (tesirine), suitable for cysteine conjugation to a protein and **IMGN779,** suitable for lysine conjugation to a protein (e.g. an antibody).
Figure 10 shows the structure of a symmetrical bis-maleimide functionalized PBD dimer **SG3199,** twice through a Val-Ala-PABC cleavable element, for double conjugation to a protein with (at least) two freely available cysteine side-chains.
Figure 11 shows the structure of a non-symmetrically modified PBD dimer, on one side via the common Val-Ala-PABC element for release by endogenous protease (e.g. cathepsin B), on the other side equipped with a glucuronic acid-functionalized p-hydroxybenzyloxycarbonyl group for release by b-glucuronidase.
Figure 12 depicts how a disulfide-based cleavable element can be installed for connection of a PBD dimer to an antibody cysteine for reductive release of the active catabolite (e.g. by glutathione).

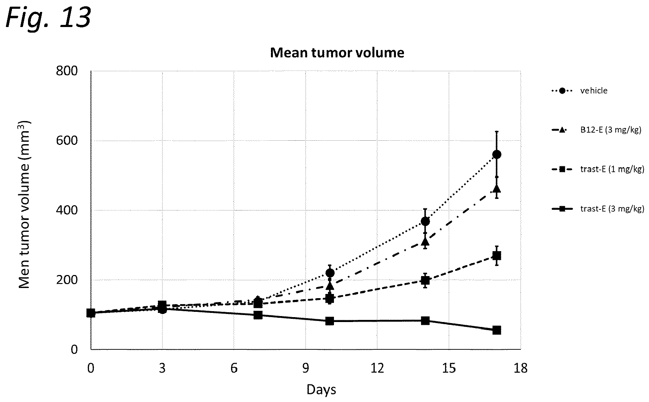

Figure 13 shows the in vivo efficacy of a mouse xenograft model (JIMT-1 cell line-derived xenograft model, HER2 1+). ADCs that were evaluated were trast-E (1 mg/kg and 3 mg/kg) and B12-E (3 mg/kg). Mice were monitored for tumor volume (top graph). Data are plotted as mean values +/- standard error of the mean.

### Detailed description of the invention

### Definitions

The verb "to comprise", and its conjugations, as used in this description and in the claims is used in its non-limiting sense to mean that items following the word are included, but items not specifically mentioned are not excluded. In addition, reference to an element by the indefinite article "a" or "an" does not exclude the possibility that more than one of the elements is present, unless the context clearly requires that there is one and only one of the elements. The indefinite article "a" or "an" thus usually means "at least one".

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof, unless stated otherwise. In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer.

The compounds disclosed in this description and in the claims may further exist as R and S stereoisomers. Unless stated otherwise, the description of any compound in the description and in the claims is meant to include both the individual R and the individual S stereoisomers of a compound, as well as mixtures thereof. When the structure of a compound is depicted as a specific S or R stereoisomer, it is to be understood that the invention of the present application is not limited to that specific S or R stereoisomer.

The compounds disclosed in this description and in the claims may further exist as R and S stereoisomers. Unless stated otherwise, the description of any compound in the description and in the claims is meant to include both the individual R and the individual S stereoisomers of a compound, as well as mixtures thereof. When the structure of a compound is depicted as a specific S or R stereoisomer, it is to be understood that the invention of the present application is not limited to that specific S or R stereoisomer.

The compounds disclosed in this description and in the claims may further exist as exo and endo diastereoisomers. Unless stated otherwise, the description of any compound in the description and in the claims is meant to include both the individual exo and the individual endo diastereoisomers of a compound, as well as mixtures thereof. When the structure of a compound is depicted as a specific endo or exo diastereomer, it is to be understood that the invention of the present application is not limited to that specific endo or exo diastereomer.

The compounds according to the invention may exist in salt form, which are also covered by the present invention. The salt is typically a pharmaceutically acceptable salt, containing a pharmaceutically acceptable anion. The term "salt thereof' means a compound formed when an acidic proton, typically a proton of an acid, is replaced by a cation, such as a metal cation or an organic cation and the like. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt.

The term "pharmaceutically acceptable" salt means a salt that is acceptable for administration to a patient, such as a mammal (salts with counter ions having acceptable mammalian safety for a given dosage regime). Such salts may be derived from pharmaceutically acceptable inorganic or organic bases and from pharmaceutically acceptable inorganic or organic acids. "Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

The term "protein" is herein used in its normal scientific meaning. Herein, polypeptides comprising about 100 or more amino acids are considered proteins. A protein may comprise natural, but also unnatural amino acids.

The term "cell-binding agent" is used herein to define an agent, typically a chemical moiety or (poly)peptide, that specifically binds to a cell. Typically, cell-binding agents bind to an epitope. Cell-binding agents include antibodies, B cells and T cells.

The term "antibody" is herein used in its normal scientific meaning. An antibody is a protein generated by the immune system that is capable of recognizing and binding to a specific antigen. An antibody is an example of a glycoprotein. The term antibody herein is used in its broadest sense and specifically includes monoclonal antibodies, polyclonal antibodies, dimers, multimers, multi-specific antibodies (e.g. bispecific antibodies), antibody fragments, and double and single chain antibodies. The term "antibody" is herein also meant to include human antibodies, humanized antibodies, chimeric antibodies and antibodies specifically binding cancer antigen. The term "antibody" is meant to include whole immunoglobulins, but also antigen-binding fragments of an antibody. Furthermore, the term includes genetically engineered antibodies and derivatives of an antibody. Antibodies, fragments of antibodies and genetically engineered antibodies may be obtained by methods that are known in the art.

An "antibody fragment" is herein defined as a portion of an intact antibody, comprising the antigen-binding or variable region thereof. Examples of antibody fragments include Fab, Fab', F(ab')2, and Fv fragments, diabodies, minibodies, triabodies, tetrabodies, linear antibodies, single-chain antibody molecules, scFv, scFv-Fc, multispecific antibody fragments formed from antibody fragment(s), a fragment(s) produced by a Fab expression library, or an epitope-binding fragments of any of the above which immunospecifically bind to a target antigen (e.g., a cancer cell antigen, a viral antigen or a microbial antigen).

An "antigen" is herein defined as an entity to which an antibody specifically binds.

The terms "specific binding" and "specifically binds" is herein defined as the highly selective manner in which an antibody or antibody binds with its corresponding epitope of a target antigen and not with the multitude of other antigens. Typically, the antibody or antibody derivative binds with an affinity of at least about 1×10-7 M, and preferably 10-8 M to 10-9 M, 10-10 M, 10-11 M, or 10-12 M and binds to the predetermined antigen with an affinity that is at least two-fold greater than its affinity for binding to a nonspecific antigen (e.g., BSA, casein) other than the predetermined antigen or a closely-related antigen.

The term "substantial" or "substantially" is herein defined as a majority, i.e. >50% of a population, of a mixture or a sample, preferably more than 50%, 55%, 60%, 65%, 70%, 75%, 80%, 85%, 90%, 91%, 92%, 93%, 94%, 95%, 96%, 97%, 98%, or 99% of a population.

The term "glycan" is herein used in its normal scientific meaning and refers to a monosaccharide or oligosaccharide chain that is linked to a protein. The term glycan thus refers to the carbohydrate-part of a glycoprotein. The glycan is attached to a protein via the C-1 carbon of one sugar, which may be without further substitution (monosaccharide) or may be further substituted at one or more of its hydroxyl groups (oligosaccharide). A naturally occurring glycan typically comprises 1 to about 10 saccharide moieties. However, when a longer saccharide chain is linked to a protein, said saccharide chain is herein also considered a glycan. A glycan of a glycoprotein may be a monosaccharide. Typically, a monosaccharide glycan of a glycoprotein consists of a single N-acetylglucosamine (GIcNAc), glucose (Glc), mannose (Man) or fucose (Fuc) covalently attached to the protein. A glycan may also be an oligosaccharide. An oligosaccharide chain of a glycoprotein may be linear or branched. In an oligosaccharide, the sugar that is directly attached to the protein is called the core sugar. In an oligosaccharide, a sugar that is not directly attached to the protein and is attached to at least two other sugars is called an internal sugar. In an oligosaccharide, a sugar that is not directly attached to the protein but to a single other sugar, i.e. carrying no further sugar substituents at one or more of its other hydroxyl groups, is called the terminal sugar. For the avoidance of doubt, there may exist multiple terminal sugars in an oligosaccharide of a glycoprotein, but only one core sugar. A glycan may be an O-linked glycan, an N-linked glycan or a C-linked glycan. In an O-linked glycan a monosaccharide or oligosaccharide glycan is bonded to an O-atom in an amino acid of the protein, typically via a hydroxyl group of serine (Ser) or threonine (Thr). In an N-linked glycan a monosaccharide or oligosaccharide glycan is bonded to the protein via an N-atom in an amino acid of the protein, typically via an amide nitrogen in the side chain of asparagine (Asn) or arginine (Arg). In a C-linked glycan a monosaccharide or oligosaccharide glycan is bonded to a C-atom in an amino acid of the protein, typically to a C-atom of tryptophan (Trp).

A "linker" is herein defined as a moiety that connects two or more elements of a compound. For example in an antibody-conjugate, an antibody and a payload are covalently connected to each other via a linker. A linker may comprise one or more linkers and spacer-moieties that connect various moieties within the linker.

A "spacer" or spacer-moiety is herein defined as a moiety that spaces (i.e. provides distance between) and covalently links together two (or more) parts of a linker. The linker may be part of e.g. a linker-construct, the linker-conjugate or a bioconjugate, as defined below.

A "self-immolative group" is herein defined as a part of a linker in an antibody-drug conjugate with a function is to conditionally release free drug at the site targeted by the ligand unit. The activatable self-immolative moiety comprises an activatable group (AG) and a self-immolative spacer unit. Upon activation of the activatable group, for example by enzymatic conversion of an amide group to an amino group or by reduction of a disulfide to a free thiol group, a self-immolative reaction sequence is initiated that leads to release of free drug by one or more of various mechanisms, which may involve (temporary) 1,6-elimination of a p-aminobenzyl group to a p-quinone methide or 1,6-elimination of a p-hydroxybenzyl group to a p-quinone, optionally with release of carbon dioxide and/or followed by a second cyclization release mechanism. The self-immolative assembly unit can part of the chemical spacer connecting the antibody and the payload (via the functional group). Alternatively, the self-immolative group is not an inherent part of the chemical spacer, but branches off from the chemical spacer connecting the antibody and the payload.

A "conjugate" is herein defined as a compound wherein a cell binding agent is covalently connected to a payload via a linker. A conjugate comprises one or more cell binding agents and/or one or more payloads.

The term "payload" refers to the moiety that is covalently attached to a targeting moiety such as an antibody, but also to the molecule that is released from the conjugate upon uptake of the protein conjugate and/or cleavage of the linker. Payload thus refers to the monovalent moiety having one open end which is covalently attached to the targeting moiety via a linker and also to the molecule that is released therefrom. In the context of the present invention, the payload is a masked PBD dimer.

A "hydrophilic group", "hydrophilic moiety" or "polar linker" is herein defined as any molecular structure containing one or more polar functional groups that imparts improved polarity, and therefore improved aqueous solubility, to the molecule it is attached to. Preferred hydrophilic groups are selected from a carboxylic acid group, an alcohol group, an ether group, a polyethylene glycol group, an amino group, an ammonium group, a sulfonate group, a phosphate group, an acyl sulfamide group or a carbamoyl sulfamide group. In addition to higher solubility other effects of the hydrophilic group include improved click conjugation efficiency, and, once incorporated into an antibody-drug conjugate: less aggregation, improved pharmacokinetics resulting in higher efficacy and in vivo tolerability.

A "PBD dimer" or "pyrrolobenzodiazepine dimer" is a class of cytotoxic agents that are used in cancer therapy, in particular as payload in antibody-drug conjugates. PBD has a chiral center at the C11a(S)- position (see Figure 8), which provides an appropriate 3-dimensional shape to fit perfectly within the DNA minor groove. A "masked PBD dimer" is defined as a pyrrolobenzodiazepine dimer derivative that comprises at least one cap, wherein the cap is hydrophilic and comprises a self-immolative group. The purpose of the cap is to mask the imine group present in the active catabolite as an hemiaminal group, thereby reducing the toxicity of the PBD until the payload has reached its target. The cap is removed by conditions that are unique for the tumor microenvironment or intracellular environment, which leads to release of the PBD active catabolite via initial liberation of the self-immolative group. Such a capped PBD dimer can be seen as prodrug of the PBD dimer. Masked PBD dimer contains at least one cap that is typically connected to the hemi-aminal or amine position on one end of the PBD dimer but may also be attached to the hemi-aminal or the amine position on other end of the PBD dimer in case the PBD dimer is linked to the antibody via the spacer connecting the two PBD monomers.

The number of payload (D) molecules attached to a single cell-binding agent, e.g. an antibody, is known in the art as the DAR (drug-antibody ratio). It will be appreciated that these are theoretical DAR values, and in practice the DAR may slightly deviate from this value. Typically, the conjugates are obtained as a stochastic mixture of antibody-drug conjugates, with DAR values varying between individual conjugates, and depending on the conjugation technique used the DAR may have a broad distribution (e.g. DAR = 0 - 10) or a narrow distribution (e.g. DAR = 3-4). In case of such mixture, DAR often refers to the average DAR of the mixture. This is well-known in the art of bioconjugation. However, in case the conjugation occurs via the glycan as in the present invention, the antibody-drug conjugates have a close-to-theoretical DAR. For example, when the theoretical DAR is 4, DAR values above 3.6 or even above 3.8 are readily obtained, indicating that most antibodies in the reaction mixture have reacted completely and have a DAR of 4. Moreover, no DAR values above 4.0 are observed.

A "DAR# conjugate" is an antibody-drug conjugate, wherein the DAR# indicates the number of payloads per antibody. In other words, a DAR1 conjugate refers to a conjugate containing one payload, a DAR2 refers to a conjugate containing two payloads and a DAR4 conjugate refers to a conjugate containing four payloads. In the context of the present invention, a DAR4 conjugate contains four masked PBD payloads, thus four pyrrolobenzodiazepines dimers.

The term "bystander killing" or "bystander effect" refers to cell death of neighbouring cells due to passive diffusion of drug away from the targeted cell and into the neighbouring cell.

### The Invention

The inventors have developed an antibody-drug conjugate, containing masked PBD as cytotoxic payload, which show no or negligible aggregation propensity. The ACDs according to the invention were found to display significant in vivo efficacy. Furthermore, the therapeutic index of the antibody-conjugate according to the invention is increased with respect to conventional PBD dimer-based antibody-drug conjugates, showing a beneficial effect of the application of masked PBD and connection of the linker-drug via the glycan of a cell-binding agent based on metal-free click chemistry.

The invention first and foremost concerns the antibody-drug conjugate of general structure (1):

AB-[L-(D)ₓ]_{y} (1)

In a second aspect, the invention concerns a process for the synthesis of the antibody-drug conjugate according to the invention. In a third aspect, the invention concerns a linker-drug construct which is suitable to be used in the process according to the invention. In a fourth aspect, the invention concerns the medical use of the antibody-drug conjugate according to the invention, as well as a pharmaceutical composition comprising the antibody-drug conjugate according to the invention. The skilled person understands that all aspects are linked, and that everything said for the antibody-drug conjugate according to the invention equally applies to the process according to the invention, the linker-drug construct according to the invention, the uses according to the invention and the composition according to the invention, and vice versa.

### The antibody-drug conjugate

The antibody-drug conjugate according to the invention has structure (1):

AB-[L-(D)ₓ]_{y} (1)

wherein
- AB is a cell-binding agent;
- x is 1 or 2;
- y is 1 or 2;
- D is a masked PBD payload.
- L is a linker that connects AB to D, wherein L comprises at least one -L⁶-Z¹- fragment, wherein Z¹ is a connecting group, said connecting group comprising the product of a cycloaddition reaction, and wherein L⁶ is -GlcNAc(Fuc)_{w}-(G)ⱼ-S-(L7)w'-, wherein L⁶ is connected to AB via GlcNAc(Fuc)_{w} and:
   ∘ G is a monosaccharide,
   ∘ j is an integer in the range of 0 - 6,
   ∘ S is a sugar or a sugar derivative,
   ∘ GlcNAc is N-acetylglucosamine,
   ∘ Fuc is fucose,
   ∘ w is 0 or 1,
   ∘ w' is 0 or 1, and
   ∘ L⁷ is -N(H)C(O)CH₂-, -N(H)C(O)CF₂- or -CH₂-.

AB represents a cell-binding agent. Preferably, the cell-binding agent is an antibody. The skilled person understands that the present invention can be applied to any antibody. More preferably, the conjugate according to the present invention comprises an antibody that interacts with a receptor that is over-expressed in cancer cells.

The antibody-drug conjugate may be a DAR1, DAR2 or DAR4 conjugate. For the DAR1 conjugate x and y in structure (1) are both 1. The DAR2 conjugate is represented by structure (1), wherein x = 1 and y = 2 or x = 2 and y = 1, preferably wherein x = 1 and y = 2. The DAR4 conjugate is represented by structure **(1)** in which x is 2 and y is also 2. Preferably, the antibody-drug conjugate is a DAR1 conjugate or a DAR2 conjugate, more preferably the antibody-drug conjugate is a DAR2 conjugate.

L is a linker that connects the antibody to the payload, D. The linker L comprises at least one -L⁶-Z¹- fragment, in which L⁶ is connected to the antibody. The structure of the linker is determined by whether the conjugate is a DAR1, DAR2 or a DAR4 conjugate. If the conjugate is a DAR1 conjugate with y = 1 and x = 1, L is preferably according to structure **(2):** 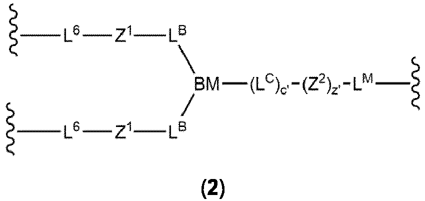 wherein both L⁶ are connected to a single AB and L^{M} to D, wherein (Z²)_{z'} represents an optional Z-fragment that may be present (z' = 1) or absent (z' = 0).

For the DAR2 and DAR4 conjugates with y = 2, L is preferably represented by structure **(3):** 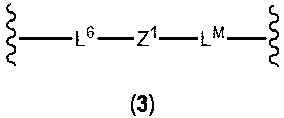 wherein L⁶ is connected to AB and L^{M} to D.

### Mode of conjugation - linker L⁶

The conjugates according to the invention may be prepared by reacting a modified antibody comprising a sugar or a sugar derivative with click-probe F¹ with a compound comprising the payload and click-probe Q¹.

The mode of conjugation involves conjugations via the glycan of the antibody, preferably via an N-glycosylation site. L⁶ is represented by -GIcNAc(Fuc)_{w-}(G)ⱼ-S-(L⁷)w'-, wherein j is an integer in the range of 0-6, w is 0 or 1, w' is 0 r 1, GIcNAc is an N-acetylglycosamine moiety, Fuc is a fucose moiety, G is a monosaccharide moiety, S is a sugar or sugar derivative. In other words, L⁶ is represented by -GlcNAc(Fuc)_{w}-(G)ⱼ-S-(L⁷)_{w'}-, wherein GlcNAc(Fuc)_{w}- is attached to the peptide part of the antibody and S is attached to Z¹ or F¹. Herein, GlcNAc is the core N-acetylglucosamine moiety which is typically present in the glycan structure of antibodies. Herein, the core N-acetylglucosamine moiety refers to the N-acetylglucosamine moiety that is directly attached to the peptide chain of the antibody. This core N-acetylglucosamine moiety is optionally fucosylated (d is 0 or 1), which is a common feature of antibodies.

(G)ⱼ represents the glycoform of the antibody. The present invention may be applied to antibodies of any glycoform. Typical monosaccharides that are present in glycans, and from among which G may be selected, include glucose, galactose, mannose, fucose, N-acetylglucosamine, N-acetylgalactosamine, N-acetylneuraminic acid and xylose. (G)ⱼ may thus be a linear or branched oligosaccharide, comprising j monosaccharide moieties. Typical glycans have j in the range of 4 - 16, preferably 6 - 10. In a preferred embodiment, the antibody is trimmed and j = 0. Such trimming may be performed by an endoglycosidase enzyme such as EndoS. Conjugation via the glycan preferably employs an N-glycosylation site, more preferably an N-glycosylation site connected to an asparagine amino acid of the antibody, most preferably to the conserved glycosylation site at amino acid N297 of the antibody. Typically, L⁶ is at least partly formed by the glycan of an antibody. All recombinant antibodies, generated in mammalian host systems, contain the conserved N-glycosylation site at the asparagine residue at or close to position 297 of the heavy chain (Kabat numbering), which is modified by a glycan of the complex type. This naturally occurring glycosylation site of antibodies is preferably used, but other glycosylation sites, including artificially introduced ones, may also be used for the connection of linker L⁶. Thus, in a preferred embodiment, L⁶ is connected to an amino acid of the antibody which is located at a position in the range of 250 - 350 of the heavy chain, preferably in the range of 280 - 310 of the heavy chain, more preferably in the range of 295 - 300 of the heavy chain, most preferably at position 297 of the heavy chain.

The -GIcNAc(Fuc)_{w}-(G)ⱼ- of L⁶ is the glycan, or part thereof. The -GIcNAc(Fuc)_{w}-(G)ⱼ- of the glycan thus typically originates from the original antibody, wherein GIcNAc is an N-acetylglucosamine moiety and Fuc is a fucose moiety. Fuc is typically bound to GIcNAc via an [β-1,6-glycosidic bond. Normally, antibodies may (w = 1) or may not be fucosylated (w = 0). In the context of the present invention, the presence of a fucosyl moiety is irrelevant, and similar effects are obtained with fucosylated (w = 1) and non-fucosylated (w = 0) antibody conjugates. The GicNAc residue may also be referred to as the core-GicNAc residue and is the monosaccharide that is directly attached to the peptide part of the antibody.

S may be directly connected to the core-GIcNAc(Fuc)_{w} moiety, i.e. j = 0, meaning that the remainder of the glycan is removed from the core-GIcNAc(Fuc)_{w} moiety before S is attached. Such trimming of glycans is well-known in the art and can be achieved by the action of an endoglycosidase. Alternatively, there are one or more monosaccharide residues present in between the core-GIcNAc(Fuc)_{w} moiety and S, wherein j typically is an integer in the range of 1 - 10, preferably j = 2 - 5. In a preferred embodiment, (G)ⱼ is an oligosaccharide fraction comprising j monosaccharide residues G, wherein j is an integer in the range of 2 - 5. (G)ⱼ is connected to the GicNAc moiety of GIcNAc(Fuc)_{w}, typically via a β-1,4 bond. In a preferred embodiment, j is 3, 4 or 5. Although any monosaccharide that may be present in a glycan may be employed as G, each G is preferably individually selected from the group consisting of galactose, glucose, *N-*acetylgalactosamine, N-acetylglucosamine, mannose and N-acetylneuraminic acid. More preferred options for G are galactose, N-acetylglucosamine, mannose. Antibodies and antibody conjugates having j = 0 show no or significantly reduced binding to Fc-gamma receptors, while antibodies and antibody conjugates having j in the range of 4 - 10 do bind to Fc-gamma receptors. Thus, by selecting a certain value for j, the desired extent of binding to Fc-gamma receptors can be obtained. It is thus preferred that j = 0, 4, 5, 6, 7, 8, 9 or 10, more preferably j = 0, 4 or 5, most preferably the antibody is trimmed and j = 0.

S is a sugar or sugar derivative. The term "sugar derivative" is herein used to indicate a derivative of a monosaccharide sugar, i.e. a monosaccharide sugar comprising substituents and/or functional groups. Suitable examples for S include glucose (Glc), galactose (Gal), mannose (Man), fucose (Fuc), amino sugars and sugar acids, e.g. glucosamine (GIcNHz), galactosamine (GaINHz), N-acetylglucosamine (GIcNAc), N-acetylgalactosamine (GaINAc), sialic acid (SiaIA) which is also referred to as N-acetylneuraminic acid (NeuNAc), and N-acetylmuramic acid (MurNAc), glucuronic acid (GlcA) and iduronic acid (IdoA). In the present invention S is a monosaccharide that was modified to contain a click probe F¹, that is transformed into a connecting group Z¹ upon conjugation with the cell-binding moiety. Preferably, S is selected from Gal, GIcNAc, GalNAc and NeuNAc. In an especially preferred embodiment, S is GaINAc. Most preferably S is a N-acetylgalactosamine that has been modified with F¹ (or after conjugation with Z¹) at the 2-position or 6-position.

Connecting group Z¹ or reactive group F¹ may be attached directly to S, or there may be a linker L⁷ present in between S and Z¹ or F¹. Thus, L⁷ may be present (w' = 1 or 2) or absent (w' = 0). Typically, each moiety Z¹ may be connected to S via a linker L⁷, thus in one embodiment w' = 0 of x. Preferably, L⁷ is absent and each connecting moiety Z¹ is directly attached to S. If present, L⁷ may be selected from -N(H)C(O)CH₂-, -N(H)C(O)CF₂- or -CH₂-. In a preferred embodiment, x = 1 and w' = 0 or 1, most preferably x = 1 and w' = 0.

y is an integer that denotes the number of sugar(s) (derivative(s)) S, each having x reactive groups F¹ or connected to x connecting groups Z¹, that are connected to AB. y is 1 or 2, preferably y = 2. Thus, the antibody contains y moieties S, each of which comprises x reactive moieties F. Each of these reactive moieties F¹ are reacted with reactive moiety Q of the linker-toxin construct, such that x × y connecting groups Z¹ are formed and x × y payloads are attached to a single AB. Each linker-toxin construct may contain multiple payloads, e.g. by virtue of a branching moiety BM in L. It is preferred that each linker-toxin construct contains 1 or 2 occurrences of D, most preferably 1 occurrence of D. In an especially preferred embodiment, linker L¹ contains a branching moiety to which a second occurrence of D is connected.

x is an integer that denotes the number of connecting groups Z¹ or reactive groups F¹ that are attached to sugar (derivative) S. Thus, the antibody preferably contains a moiety S comprising x reactive moieties F¹. Each of these reactive moieties F¹ are reacted with a reactive moiety Q¹ of the linker-toxin construct, such that x connecting groups Z¹ are formed and x payloads are attached to a single occurrence of S. x is 1 or 2, preferably x = 1.

### The connecting group Z

Z is a connecting group. The term "connecting group' refers to a structural element connecting one part of the conjugate and another part of the same bioconjugate. In the present invention connects antibody AB with the masked PBD payload, via a linker. Connecting group Z¹ is a moiety obtainable by a cycloaddition, preferably a metal-free click reaction, the reactants for this a cycloaddition being click probe F¹ and click probe Q¹. As the skilled person understands, the exact nature of Z¹ depends on the nature of F¹ and Q¹. Preferred embodiments for Q¹ and F¹ are defined below.

Z¹ is formed by a cycloaddition. Conjugation reactions via cycloadditions are known to the skilled person, and the skilled person is capable of selecting appropriate reaction partners F¹ and Q¹, and will understand the nature of the resulting connecting group Z¹. Preferred cycloadditions are a (4+2)-cycloaddition (e.g. a Diels-Alder reaction) or a (3+2)-cycloaddition (e.g. a 1,3-dipolar cycloaddition). Preferably, the conjugation is the Diels-Alder reaction or the 1 ,3-dipolar cycloaddition. The preferred Diels-Alder reaction is the inverse electron-demand Diels-Alder cycloaddition. In another preferred embodiment, the 1,3-dipolar cycloaddition is used, more preferably the alkyne-azide cycloaddition, and most preferably wherein Q¹ is or comprises an alkyne group and F¹ is an azido group. Cycloadditions, such as Diels-Alder reactions and 1,3-dipolar cycloadditions are known in the art, and the skilled person knowns how to perform them.

Preferably, Z¹ contains a moiety selected from the group consisting of a triazole, a cyclohexene, a cyclohexadiene, a [2.2.2]-bicyclooctadiene, a [2.2.2]-bicyclooctene, an 20sooxazoline, an isoxazolidine, a pyrazoline, a piperazine, a thioether, an amide or an imide group. Triazole moieties are especially preferred to be present in Z¹. In one embodiment, Z¹ comprises a (hetero)cycloalkene moiety, i.e. formed from Q¹ comprising a (hetero)cycloalkyne moiety. In an alternative embodiment, Z¹ comprises a (hetero)cycloalkane moiety, i.e. formed from Q¹ comprising a (hetero)cycloalkene moiety. Herein, aromatic rings such as a triazole ring are considered a heterocycloalkane ring, since it is formed by reaction of an alkyne moiety and an azide moiety. In a preferred embodiment, Z¹ has the structure (Z1):

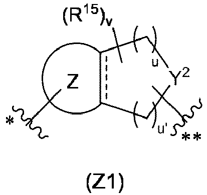

Herein, the bond depicted as - - - is a single bond or a double bond. Furthermore:
- ring Z is obtained by a cycloaddition, preferably ring Z is selected from (Za) - (Zj) defined below, wherein the carbon atoms labelled with ** correspond to the two carbon atoms of the bond depicted as - - - of (Z1) to which ring Z is fused;
- R¹⁵ is independently selected from the group consisting of hydrogen, halogen, -OR¹⁶, -NO₂, -CN, -S(O)₂R¹⁶, -S(O)₃⁽⁻⁾, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y² is C(R³¹)₂, O, S, S⁽⁺⁾R³¹, S(O)R³¹, S(O)=NR³¹ or NR³¹, wherein S⁽⁺⁾ is a cationic sulphur atom counterbalanced by B⁽⁻⁾, wherein B⁽⁻⁾ is an anion, and wherein each R³¹ individually is R¹⁵ or a connection with D, connected via L;
- u is 0, 1, 2, 3, 4 or 5;
- u' is 0, 1, 2, 3, 4 or 5, wherein u + u' = 0, 1, 2, 3, 4, 5, 6, 7 or 8;
- v = an integer in the range 8 - 16;
- Ring Z is formed by the cycloaddition, and is preferably selected from (Za) - (Zj).

In a preferred embodiment, u + u' = 0, 4, 5, 6, 7 or 8, more preferably 0, 4 or 5. In case the bond depicted as - - - is a double bond, it is preferred that u + u' = 4, 5, 6, 7 or 8, more preferably u + u' = 4 or 5. In case the bond depicted as - - - is a single bond, it is preferred that u + u' = 0 or 5. Preferably, the wavy bond labelled with * is connected to AB, optionally via L⁶, and the wavy bond labelled with ** is connected to L.

It is especially preferred that Z¹ comprises a (hetero)cycloalkene moiety, i.e. the bond depicted as - - - is a double bond . In a preferred embodiment, Z¹ is selected from the structures (Z2)-(Z20) or (Z38a):

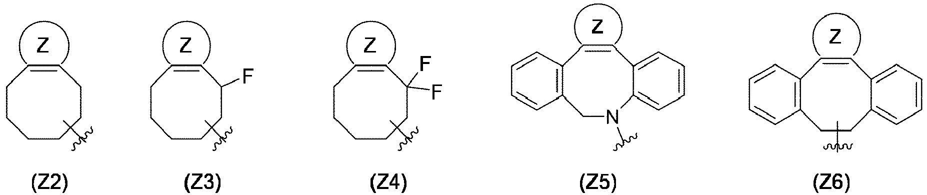

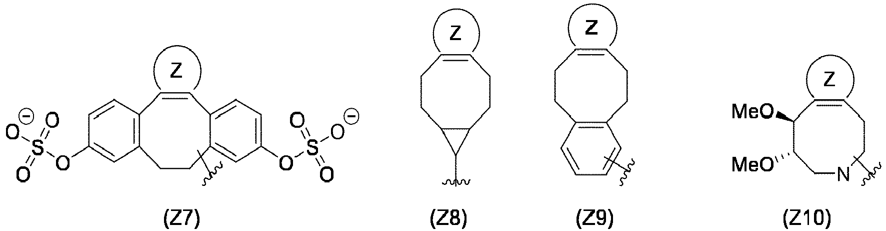

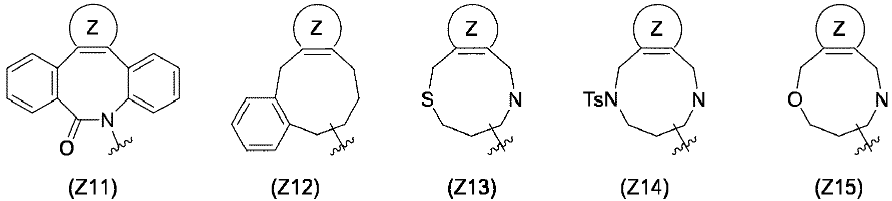

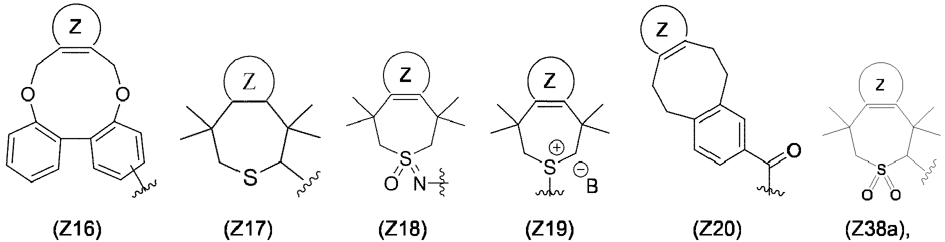

Herein the connection to the payload, via a linker, is represented by the wavy bond, B⁽⁻⁾ is an anion, preferably a pharmaceutically acceptable anion. Ring Z is formed by the cycloaddition reaction, and preferably is selected from a triazole, a cyclohexene, a cyclohexadiene, a [2.2.2]-bicyclooctadiene, a [2.2.2]-bicyclooctene, an iso-oxazoline, an isoxazolidine, a pyrazoline or a piperazine. Most preferably, ring Z is a triazole ring. Ring Z may have the structure selected from (Za)-(Zj) depicted below, wherein the carbon atoms labelled with ** correspond to the two carbon atoms of the (hetero)cycloalkane ring of (Z2)-(Z20), (Z38a) to which ring Z is fused. Since the connecting group Z is formed by the reaction with a (hetero)cycloalkyne in the context of the present embodiment, the bond depicted above as - - - is a double bond.

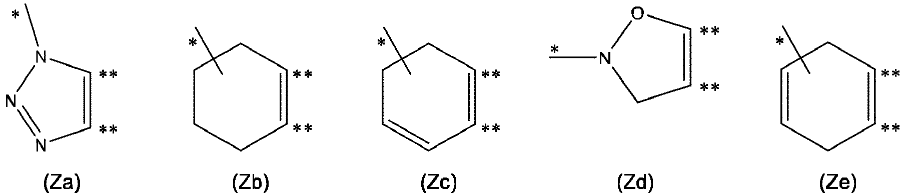

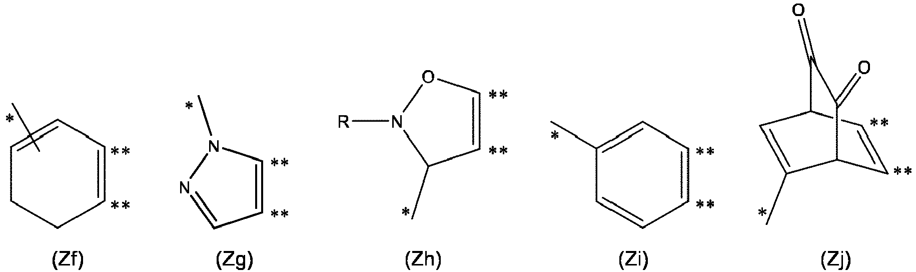

In a further preferred embodiment, Z¹ is selected from the structures (Z21) - (Z38a), depicted here below:

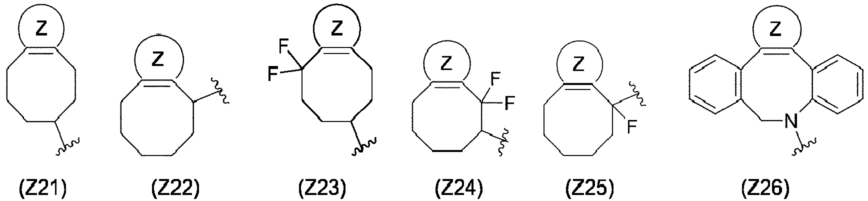

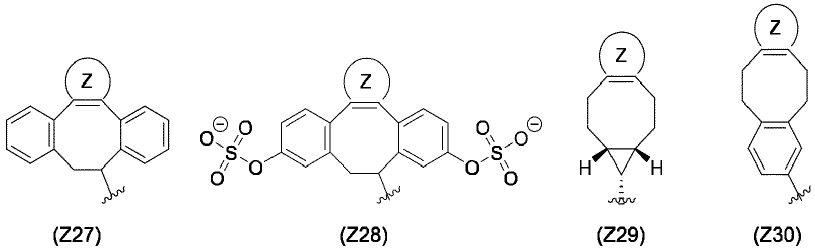

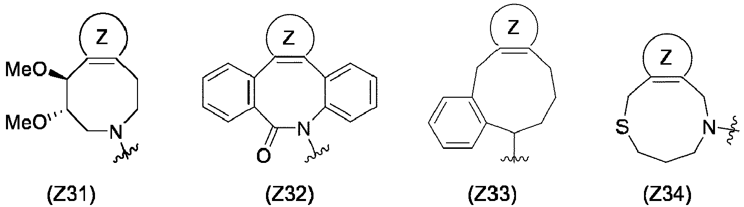

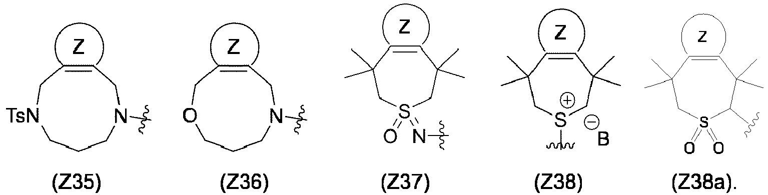

Herein, the connection to L is depicted with the wavy bond. Structure (Z29) can be in endo or exo configuration, preferably it is in endo configuration. In structure (Z38), B⁽⁻⁾ is an anion, preferably a pharmaceutically acceptable anion. Ring Z is selected from structures (Za) - (Zj), as defined above.

In a preferred embodiment, Z¹ comprises a (hetero)cyclooctene moiety or a (hetero)cycloheptene moiety, preferably according to structure (Z8), (Z26), (Z27), (Z28) or (Z37), which are optionally substituted. Each of these preferred options for Z¹ are further defined here below.

Thus, in a preferred embodiment, Z¹ comprises a heterocycloheptene moiety according to structure (Z37), which is optionally substituted. Preferably, the heterocycloheptene moiety according to structure (Z37) is not substituted.

In a preferred embodiment, Z¹ comprises a (hetero)cyclooctene moiety according to structure (Z8), more preferably according to (Z29), which is optionally substituted. Preferably, the cyclooctene moiety according to structure (Z8) or (Z29) is not substituted. In the context of the present embodiment, Z¹ preferably comprises a (hetero)cyclooctene moiety according to structure (Z39) as shown below, wherein V is (CH₂)₁ and I is an integer in the range of 0 to 10, preferably in the range of 0 to 6. More preferably, I is 0, 1, 2, 3 or 4, more preferably I is 0, 1 or 2 and most preferably I is 0 or 1. In the context of group (Z39), I is most preferably 1. Most preferably, Z¹ is according to structure (Z42), defined further below.

In an alternative preferred embodiment, Z¹ comprises a (hetero)cyclooctene moiety according to structure (Z26), (Z27) or (Z28), which are optionally substituted. In the context of the present embodiment, Z¹ preferably comprises a (hetero)cyclooctene moiety according to structure (Z40) or (Z41) as shown below, wherein Y¹ is O or NR¹¹, wherein R¹¹ is independently selected from the group consisting of hydrogen, a linear or branched C₁ - C₁₂ alkyl group or a C₄- C₁₂ (hetero)aryl group. The aromatic rings in (Z40) are optionally O-sulfonylated at one or more positions, whereas the rings of (Z41) may be halogenated at one or more positions. Preferably, the (hetero)cyclooctene moiety according to structure (Z40) or (Z41) is not further substituted. Most preferably, Z¹ is according to structure (Z43), defined further below.

In an alternative preferred embodiment, Z¹ comprises a heterocycloheptenyl group and is according to structure (Z37):

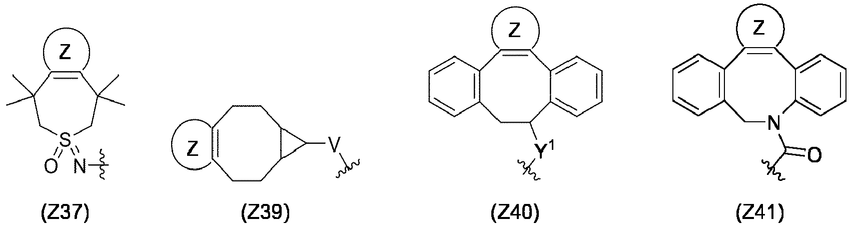

In an especially preferred embodiment, Z¹ comprises a cyclooctenyl group and is according to structure (Z42):

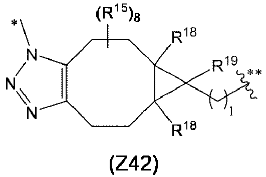

Herein: the bond labelled with * is connected to AB and the wavy bond labelled with ** is connected to L;
R¹⁵ is independently selected from the group consisting of hydrogen, halogen, -OR¹⁶, -NO₂, -CN, -S(O)₂R¹⁸, -S(O)₃⁽⁻⁾,C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
R¹⁸ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
R¹⁹ is selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted, or R¹⁹ is a second occurrence of Z¹ (or Q) or D connected via a spacer moiety; and I is an integer in the range 0 to 10.

In a preferred embodiment of the group according to structure (Z42), R¹⁵ is independently selected from the group consisting of hydrogen, halogen, -OR¹⁶, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and C₁ - C₆ alkyl, most preferably all R¹⁵ are H. In a preferred embodiment of the group according to structure (Z42), R¹⁸ is independently selected from the group consisting of hydrogen, C₁ - C₆ alkyl groups, most preferably both R¹⁸ are H. In a preferred embodiment of the group according to structure (Z42), R¹⁹ is H. In a preferred embodiment of the group according to structure (Z42), I is 0 or 1, more preferably I is 1.

In an especially preferred embodiment, Z¹ comprises a (hetero)cyclooctenyl group and is according to structure (Z43):

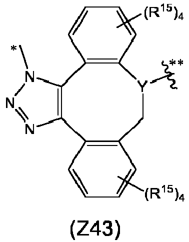

Herein: the bond labelled with * is connected to AB and the wavy bond labelled with ** is connected to L; R¹⁵ is independently selected from the group consisting of hydrogen, halogen, -OR¹⁶, -NO₂, -CN, - S(O)₂R¹⁶, -S(O)₃⁽⁻⁾, C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups; Y is N or CR¹⁵.

In a preferred embodiment of the group according to structure (Z43), R¹⁵ is independently selected from the group consisting of hydrogen, halogen, -OR¹⁶, -S(O)₃⁽⁻⁾, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and -S(O)₃⁽⁻⁾. In a preferred embodiment of the group according to structure (Z43), Y is N or CH, more preferably Y = N.

In an especially preferred embodiment, Z¹ comprises a heterocycloheptenyl group and is according to structure (Z37) or (Z38a), wherein ring Z is a triazole.

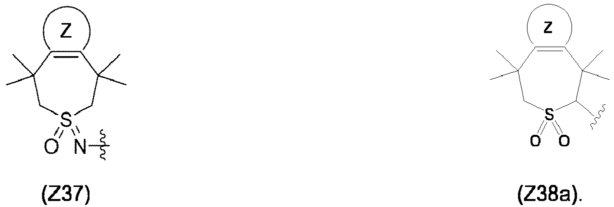

In an alternative preferred embodiment, Z¹ comprises a (hetero)cycloalkane moiety, i.e. the bond depicted as - - - is a single bond. The (hetero)cycloalkane group may also be referred to as a heterocycloalkyl group or a cycloalkyl group, preferably a cycloalkyl group, wherein the (hetero)cycloalkyl group is optionally substituted. Preferably, the (hetero)cycloalkyl group is a (hetero)cyclopropyl group, a (hetero)cyclobutyl group, a norbornyl group, a norbornenyl group, a (hetero)cycloheptyl group, a (hetero)cyclooctyl group, which may all optionally be substituted. Especially preferred are (hetero)cyclopropyl groups, (hetero)cycloheptyl group or (hetero)cyclooctyl groups, wherein the (hetero)cyclopropyl group, the (hetero)cycloheptyl group or the (hetero)cyclooctyl group is optionally substituted. Preferably, Z¹ comprises a cyclopropyl moiety according to structure (Z44), a hetereocyclobutane moiety according to structure (Z45), a norbornane or norbornene group according to structure (Z46), a (hetero)cycloheptyl moiety according to structure (Z47) or a (hetero)cyclooctyl moiety according to structure (Z48). Herein, Y³ is selected from C(R²³)₂, NR²³ or O, wherein each R²³ is individually hydrogen, C₁ - C₆ alkyl or is connected to L, optionally via a spacer, and the bond labelled --- is a single or double bond. In a further preferred embodiment, the cyclopropyl group is according to structure (Z49). In another preferred embodiment, the (hetero)cycloheptane group is according to structure (Z50) or (Z51). In another preferred embodiment, the (hetero)cyclooctane group is according to structure (Z52), (Z53), (Z54), (Z55) or (Z56).

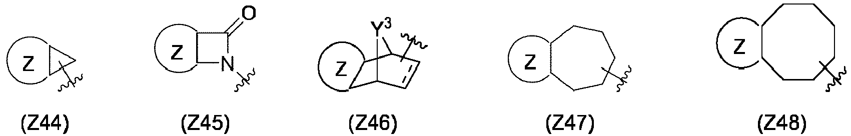

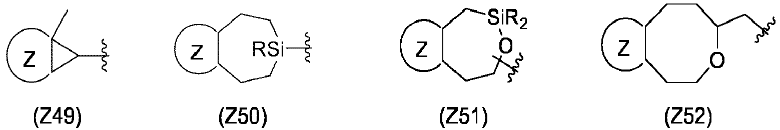

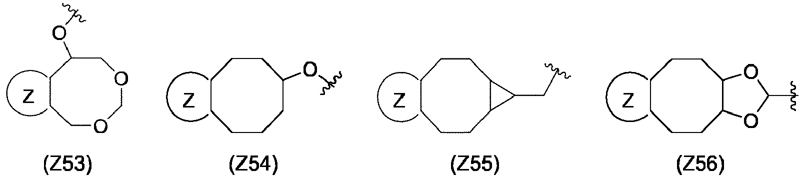

Herein, the R group(s) on Si in (Z50) and (Z51) are typically alkyl or aryl, preferably C₁-C₆ alkyl. Ring Z is selected from structures (Zk) - (Zn), wherein the carbon atoms labelled with ** correspond to the two carbon atoms of the (hetero)cycloalkane ring of (Z44) - (Z56) to which ring Z is fused, and the carbon a carbon labelled with * is connected to AB. Since the connecting group Z¹ is formed by reaction with a (hetero)cycloalkene in the context of the present embodiment, the bound depicted above as --- is a single bond.

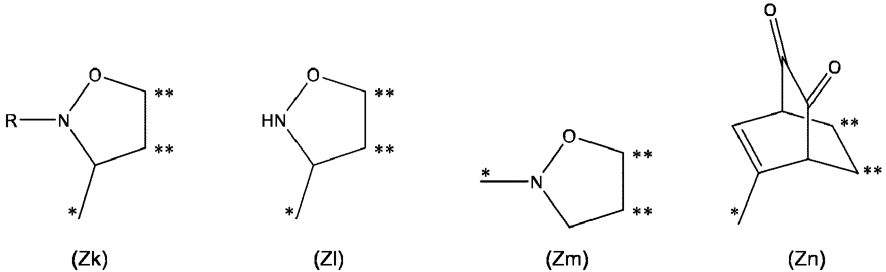

Z² is a further connecting group that may be present when y = 1, typically in a DAR1 conjugate. Z² is formed by reacting the precursor containing the payload with the precursor containing the other part of the linker. The nature of Z² thus depends on the nature of the reactive moieties and the type of reaction that is performed to connect those parts, and may take any form. Preferably, connecting group Z² is selected from the group consisting of an amide moiety, an ester moiety, a carbamate moiety, a carbonate moiety or a (hetero)aryl moiety, (hetero)cycloalkene moiety, heterocycloalkane moiety, more preferably an amide moiety or a carbamate moiety. Z² may be formed by a nucleophilic reaction, preferably a nucleophilic substitution or a Michael addition. In an especially preferred embodiment Z² is formed by a click reaction, in which case Z² comprises a (hetero)aryl moiety, (hetero)cycloalkene moiety or heterocycloalkane moiety, most preferably Z² is individually selected from the same structures as defined for Z¹, including preferred embodiments thereof. In this embodiment, Z¹ and Z² may be individually selected, wherein it is preferred that both occurrences of Z¹ are identical.

### Branching moiety

In a preferred embodiment, the linker of the conjugate according to the invention contains a branching moiety. A "branching moiety" in the context of the present invention refers to a moiety that is embedded in a linker connecting three moieties. In other words, the branching moiety comprises at least three bonds to other moieties. In one embodiment, the conjugate is a DAR4 conjugate, wherein BM comprises one bond to antibody AB, one bond to a PBD dimer payload and one bond to a second PBD dimer payload. Alternatively the conjugate is a DAR1 conjugate, BM comprises one bond to the PBD dimer payload, one bond the antibody AB and another bond to the same antibody AB. Any moiety that contains at least three bonds to other moieties is suitable as branching moiety in the context of the present invention. In a preferred embodiment, the branching moiety BM is selected from a carbon atom, a nitrogen atom, a phosphorus atom, a (hetero)aromatic ring, a (hetero)cycle or a polycyclic moiety. Most preferably, the branching moiety is a nitrogen atom.

### Linkers L^{B}and L^{c}

L^{B} is a chain of at least 2, preferably 5 to 100, atoms selected from C, N, O, S and P that may be present when y = 1, typically in DAR1 conjugates and connects the branching moiety BM with the Z¹ adjacent to L⁶. L^{B} may for example be selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups, C₉-C₂₀₀ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S(O)_{y'} and NR²¹, wherein y' is 0, 1 or 2, preferably y' = 2, and R²¹ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups.

In a preferred embodiment, both occurrences of L^{B} are identical. Preferred linkers L^{B} have structure (LB1), (LB2) or (LB3):

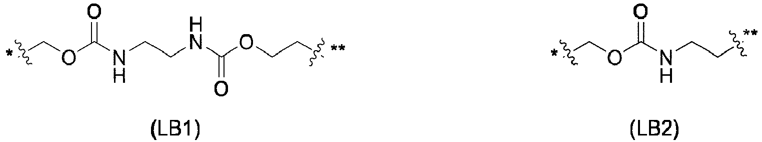

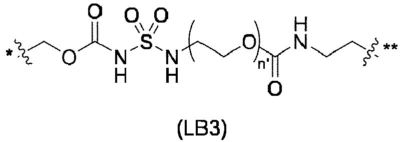

Herein, n' is an integer in the range of 1 - 10, preferably in the range of 1 - 4, most preferable n' = 2. The wavy bond labelled * is connected to the Z¹ and the wavy bond labelled ** is connected to BM.

L^{C} is a chain of at least 2, preferably 5 to 100, atoms selected from C, N, O, S and P that may be present when y = 1, typically in DAR1 conjugates, L^{C} connects the branching moiety with L^{M} or the (Z)_{z}. L^{C} may for example be selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups, C₉-C₂₀₀ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S(O)_{y'} and NR²¹, wherein y' is 0, 1 or 2, preferably y' = 2, and R²¹ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups.

In a preferred embodiment, the antibody-conjugate is a DAR1 conjugate according to structure (2), wherein BM =N, in which the conjugate has structure AB-[L-D], i.e. x = y = 1, and L has a structure selected from (L5) - (L7):

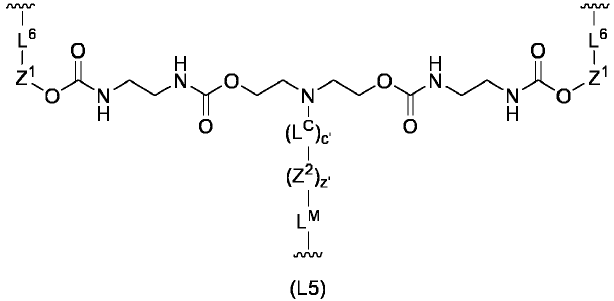

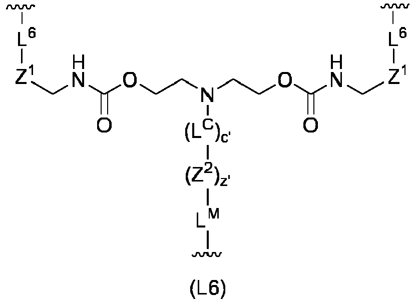

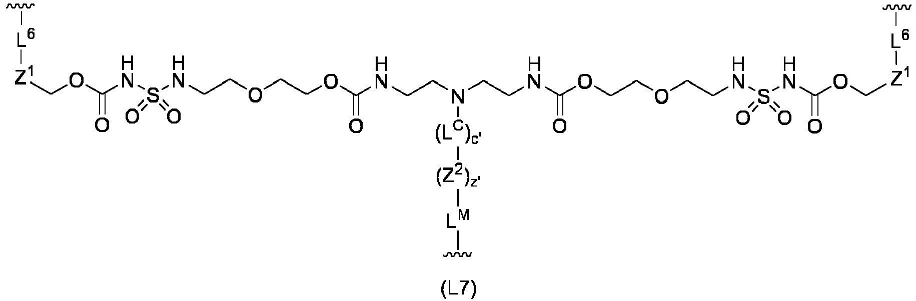

Herein, both occurrences of L⁶ are connected to a single AB, and L^{M} is connected to payload D.

### Linker L^{M}

Linker L^{M} has structure -(L¹)ₙ-(L²)ₒ-(L³)ₚ-(L⁴)_{q}-, wherein L¹, L², L³ and L⁴ are each individually linkers that together link Z, L^{C} or BM to D. Herein L¹, L², L³ and L⁴ are linkers or linking units and each of n, o, p and q are individually 0 or 1, wherein n + o + p + q is at least 1; p + q is 1 or 0 and o = p. In a preferred embodiment linkers L¹, L² and L³ are present and L⁴ is not (i.e. n = 1; o = 1; p = 1; q = 0). In another preferred embodiment, linkers L¹ and L⁴ are present (i.e. n = 1; o = 0; p = 0; q = 1). In another preferred embodiment, only linkers L¹ is present (i.e. n = 1; o = 0; p = 0; q = 0).

### Linker L ¹

Linker L¹ is either absent (n = 0) or present (n = 1). Preferably, linker L¹ is present and n = 1. L¹ may for example be selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups, C₉-C₂₀₀ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S(O)_{y'} and NR²¹, wherein y' is 0, 1 or 2, preferably y' = 2, and R²¹ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups.

In a preferred embodiment, linker L¹ contains a polar group. Such a polar group may be selected from (poly)ethylene glycol diamines (e.g. 1,8-diamino-3,6-dioxaoctane or equivalents comprising longer ethylene glycol chains), (poly)ethylene glycol or (poly)ethylene oxide chains, (poly)propylene glycol or (poly)propylene oxide chains and 1,z'-diaminoalkanes (wherein z' is the number of carbon atoms in the alkane, preferably z' = 1 - 10), -(O)ₐ-C(O)-NH-S(O)₂-NR¹³- (as further defined below, see structure (L1a)), -C(S(O)₃⁽⁻⁾)-, -C(C(O)₂⁽⁻⁾)-, -S(O)₂-, -P(O)₂⁽⁻⁾-, -O(CH₂CH₂O)ₜ-, -NR³⁰(CH₂CH₂NR³⁰)ₜ-, and the following two structures:

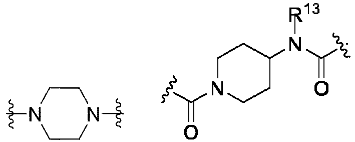

For the polar groups defined here above, it is irrelevant which end is connected to Z¹ and which end to (L²)ₒ.

The polar group may also contain an amino acid, preferably selected from Arg, Glu, Asp, Ser and Thr. Herein, a and R¹³ are further defined below for structure (L1a). t is an integer in the range of integer in the range of 0 - 15, preferably 1 - 10, more preferably 2 - 5, most preferably t = 2 or 4. Each R³⁰ is individually H, C₁₋₁₂ alkyl, C₁₋₁₂ aryl, C₁₋₁₂ alkaryl or C₁₋₁₂ aralkyl. Linker L¹ may contain more than one such polar group, such as at least two polar groups. The polar group may also be present in a branch of linker L¹, which branches off a branching moiety as defined elsewhere. Preferable, a nitrogen or carbon atom is used as branching moiety. It is especially preferred to have a -O(CH₂CH₂O)ₜ- polar group present in a branch.

In a preferred embodiment, Linker L¹ is or comprises a sulfamide group, preferably a sulfamide group according to structure (L1a):

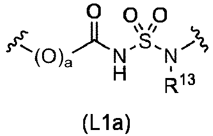

The wavy lines represent the connection to the remainder of the compound, typically to Q and L², L³ or D, preferably to Q and L². Preferably, the (O)ₐC(O) moiety is connected to Q and the NR¹³ moiety to L², L³ or D, preferably to L².

In structure (L1a), a = 0 or 1, preferably a = 1, and R¹³ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups. Alternatively, R¹³ is D connected to N optionally via a spacer moiety, preferably via Sp² as defined below, in one embodiment D is connected to N via -(B)ₑ-(A)_{f}-(B)_{g}-C(O)-. Alternatively, R¹³ is connected to elsewhere in the linker, optionally via a spacer moiety, to form a cyclic structure. For example, R¹³ may be connected to the linker via a CH₂CH₂ spacer moiety to form a piperazinyl ring, where the connection to D is via the second nitrogen of the piperazinyl ring.

In a preferred embodiment, R¹³ is hydrogen, a C₁ - C₂₀ alkyl group, preferably a C₁-C₁₆ alkyl group, more preferably a C₁ - C₁₀ alkyl group, or connected to a further occurrence of D or to elsewhere in the linker optionally via a spacer moiety. Herein, the alkyl group is optionally substituted and optionally interrupted by one or more heteroatoms selected from O, S and NR¹⁴, preferably O, wherein R¹⁴ is independently selected from the group consisting of hydrogen and C₁ - C₄ alkyl groups. In another preferred embodiment, R¹³ is a C₁ - C₂₀ alkyl group, more preferably a C₁-C₁₆ alkyl group, even more preferably a C₁ - C₁₀ alkyl group, wherein the alkyl group is optionally interrupted by one or more O-atoms, and wherein the alkyl group is optionally substituted with an -OH group, preferably a terminal -OH group. In this embodiment it is further preferred that R¹³ is a (poly)ethylene glycol chain comprising a terminal -OH group. In another preferred embodiment, R¹³ is selected from the group consisting of hydrogen, methyl, ethyl, n-propyl, i-propyl, n-butyl, s-butyl and t-butyl, or connected to a further occurrence of D or to elsewhere in the linker optionally via a spacer moiety, more preferably from the group consisting of hydrogen, methyl, ethyl, n-propyl and i-propyl, or connected to a further occurrence of D or to elsewhere in the linker optionally via a spacer moiety, and even more preferably from the group consisting of hydrogen, methyl and ethyl, or connected to a further occurrence of D or to elsewhere in the linker optionally via a spacer moiety. Yet even more preferably, R¹³ is hydrogen or connected to a further occurrence of D or to elsewhere in the linker optionally via a spacer moiety, and most preferably R¹³ is hydrogen.

In a preferred embodiment, L¹ is according to structure (L1b):

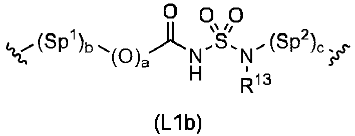

Herein, a and R¹³ are as defined above, Sp¹ and Sp² are independently spacer moieties and b and c are independently 0 or 1. Preferably, b = 0 or 1 and c = 1, more preferably b = 0 and c = 1. In one embodiment, spacers Sp¹ and Sp² are independently selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups and C₉-C₂₀₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted. When the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups are interrupted by one or more heteroatoms as defined above, it is preferred that said groups are interrupted by one or more O-atoms, and/or by one or more S-S groups.

More preferably, spacer moieties Sp¹ and Sp², if present, are independently selected from the group consisting of linear or branched C₁-C₁₀₀ alkylene groups, C₂-C₁₀₀ alkenylene groups, C₂-C₁₀₀ alkynylene groups, C₃-C₁₀₀ cycloalkylene groups, C₅-C₁₀₀ cycloalkenylene groups, C₈-C₁₀₀ cycloalkynylene groups, C₇-C₁₀₀ alkylarylene groups, C₇-C₁₀₀ arylalkylene groups, C₈-C₁₀₀ arylalkenylene groups and C₉-C₁₀₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂- C₂₄ alkenyl groups, C₂-C₂₄ alkynyl groups and C₃ - C₂₄ cycloalkyl groups, the alkyl groups, alkenyl groups, alkynyl groups and cycloalkyl groups being optionally substituted.

Even more preferably, spacer moieties Sp¹ and Sp², if present, are independently selected from the group consisting of linear or branched C₁-C₅₀ alkylene groups, C₂-C₅₀ alkenylene groups, C₂-C₅₀ alkynylene groups, C₃-C₅₀ cycloalkylene groups, C₅-C₅₀ cycloalkenylene groups, C₈-C₅₀ cycloalkynylene groups, C₇-C₅₀ alkylarylene groups, C₇-C₅₀ arylalkylene groups, C₈-C₅₀ arylalkenylene groups and C₉-C₅₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted.

Yet even more preferably, spacer moieties Sp¹ and Sp², if present, are independently selected from the group consisting of linear or branched C₁-C₂₀ alkylene groups, C₂-C₂₀ alkenylene groups, C₂-C₂₀ alkynylene groups, C₃-C₂₀ cycloalkylene groups, C₅-C₂₀ cycloalkenylene groups, C₈-C₂₀ cycloalkynylene groups, C₇-C₂₀ alkylarylene groups, C₇-C₂₀ arylalkylene groups, C₈-C₂₀ arylalkenylene groups and C₉-C₂₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted.

In these preferred embodiments it is further preferred that the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups are unsubstituted and optionally interrupted by one or more heteroatoms selected from the group of O, S and NR¹⁶, preferably O, wherein R¹⁶ is independently selected from the group consisting of hydrogen and C₁ - C₄ alkyl groups, preferably hydrogen or methyl.

Most preferably, spacer moieties Sp¹ and Sp², if present, are independently selected from the group consisting of linear or branched C₁-C₂₀ alkylene groups, the alkylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted. In this embodiment, it is further preferred that the alkylene groups are unsubstituted and optionally interrupted by one or more heteroatoms selected from the group of O, S and NR¹⁶, preferably O and/or S-S, wherein R¹⁶ is independently selected from the group consisting of hydrogen and C₁ - C₄ alkyl groups, preferably hydrogen or methyl.

Preferred spacer moieties Sp¹ and Sp² thus include -(CH₂)ᵣ-, -(CH₂CH₂)ᵣ-, -(CH₂CH₂O)ᵣ-, -(OCH₂CH₂)ᵣ-, -(CH₂CH₂O)ᵣCH₂CH₂-, -CH₂CH₂(OCH₂CH₂)ᵣ-, -(CH₂CH₂CH₂O)ᵣ-, -(OCH₂CH₂CH₂)ᵣ-, -(CH₂CH₂CH₂O)ᵣCH₂CH₂CH₂- and -CH₂CH₂CH₂(OCH₂CH₂CH₂)ᵣ-, wherein r is an integer in the range of 1 to 50, preferably in the range of 1 to 40, more preferably in the range of 1 to 30, even more preferably in the range of 1 to 20 and yet even more preferably in the range of 1 to 15. More preferably n is 1, 2, 3, 4, 5, 6, 7, 8, 9 or 10, more preferably 1, 2, 3, 4, 5, 6, 7 or 8, even more preferably 1, 2, 3, 4, 5 or 6, yet even more preferably 1, 2, 3 or 4.

In a particularly preferred embodiment, L¹ is a linker that for DAR1 and DAR2 conjugates, x of structure (1) being 1, is represented by -(W)ₖ-(A)_{d}-(B)ₑ-(A)_{f}-(B)_{g}-(C(O))_{g}-, wherein
- d = 0 or 1, preferably d = 1;
- e = an integer in the range 0 - 10, preferably e = 0, 1, 2, 3, 4, 5 or 6, preferably an integer in the range 1 - 10, most preferably e = 1, 2, 3 or 4;
- f = 0 or 1, preferably f = 0;
- wherein d + e + f is at least 1, preferably in the range 1 - 5; and preferably wherein d + f is at least 1, preferably d + f = 1.
- g = 0 or 1, preferably g = 1;
- k = 0 or 1, preferably k = 1;
- A is a sulfamide group according to structure (L1a);
- B represents a spacer such as a PEG spacer. B is individually selected from L², a -(CH₂)ₓ-O- or a -O-(CH₂)ₓ- moiety, or (B)ₑ is a -((CH₂)ₓ-O)ₑ₁-(CH₂)ₓ- moiety, wherein e1 is an integer in the range 1-10, each x is individually an integer in the range 1 - 10.
- W is -OC(O)-, -C(O)O-, -C(O)NH-, -NHC(O)-, -OC(O)NH-, -NHC(O)O-, -C(O)(CH₂)ₘC(O)-, -C(O)(CH₂)ₘC(O)NH- or -(4-Ph)CH₂NHC(O)(CH₂)ₘC(O)NH-, preferably wherein W is -OC(O)NH-, -C(O)(CH₂)ₘC(O)NH- or -C(O)NH-, and wherein m is an integer in the range 0 - 10, preferably m = 0, 1, 2, 3, 4, 5 or 6, most preferably m = 2 or 3;
- preferably wherein L¹ is connected to Q via (W)ₖ and to L², L³ or D, preferably to L², via (C(O))_{g}, preferably via C(O).

In the context of the present embodiment, the wavy lines in structure (L1a) represent the connection to the adjacent groups such as (W)ₖ, (B)ₑ and (C(O))_{g}. It is preferred that A is according to structure (L1a), wherein a = 1 and R¹³ = H or a C₁ - C₂₀ alkyl group, more preferably R¹³ = H or methyl, most preferably R¹³ = H.

Preferred linkers L¹ have structure -(W)ₖ-(A)_{d}-(B)ₑ-(A)f-(C(O))_{g}-, wherein:
(a) k = 0; d = 1; g = 1; f = 0; B = -CH₂-CH₂-O-; e = 1, 2, 3 or 4, preferably e = 2.
(b) k = 1; W = -C(O)(CH₂)ₘC(O)NH-; m = 2; d = 0; (B)ₑ = -(CH₂-CH₂-O)ₑ₁-CH₂-CH₂-; f = 0; g = 1; e1 = 1, 2, 3 or 4, preferably e = 1.
(c) k = 1; W = -OC(O)NH-; d = 0; B = -CH₂-CH₂-O-; g = 1; f = 0; e = 1, 2, 3 or 4, preferably e = 2.
(d) k = 1; W = -C(O)(CH2)mC(O)NH-; m = 2; d = 0; (B)ₑ = -(CH₂-CH₂-O)ₑ₁-CH₂-CH₂-; f = 0; g = 1; e1 = 1, 2, 3 or 4, preferably e1 = 4.
(e) k = 1; W = -OC(O)NH-; d = 0; (B)ₑ = -(CH₂-CH₂-O)ₑ₁-CH₂-CH₂-; g = 1; f = 0; e1 = 1, 2, 3 or 4, preferably e1 = 4.
(f) k = 1; W = -(4-Ph)CH₂NHC(O)(CH₂)ₘC(O)NH-, m = 3; d = 0; (B)ₑ = -(CH₂-CH₂-O)ₑ₁-CH₂-CH₂-; g = 1; f = 0; e1 = 1, 2, 3 or 4, preferably e1 = 4.
(g) k = 0; d = 0; g = 1; f = 0; B = -CH₂-CH₂-O-; e = 1, 2, 3 or 4, preferably e = 2.
(h) k = 1; W = -C(O)NH-; d = 0; g = 1; f = 0; B = -CH₂-CH₂-O-; e = 1, 2, 3 or 4, preferably e = 2.

Herein, it is preferred that when d and/or f = 1, than a = 1 and R¹³ = H. Most preferred is the linker is structure (a).

Alternatively, for DAR4 conjugates, x of structure (1) being 2, it is particularly preferred that L¹ is represented by:

-(W)ₖ-(A)_{d}-(B)ₑ-(A)_{f}-(C(O))_{g}-BM[-(A)_{d'}-(B)_{e'}-(A)_{f'}-(C(O))_{g'}-]₂.

wherein A, B, BM, W, d, e, f, g, and k are defined as above and individually selected for each occurrence.

Preferred linkers L¹ comprising a branching moiety have structure -(W)ₖ-(A)_{d}-(B)ₑ-(A)_{f}-(C(O))_{g}-BM[-(A')_{d'}-(B')_{e'}-(A')_{f'}-(C(O))_{g'}-]₂ wherein:
- k = d = g = e' = 1; f = d' = g' = 0; W = -C(O)-; B = B' = -CH₂-CH₂-O-; A is according to structure (L1a) with a = 0 and R¹³ = H; e = 1, 2, 3 or 4, preferably e = 2.
- k = d = g = e' = g' = 1; f = d' = 0; W = -C(O)-; B = B' = -CH₂-CH₂-O-; A is according to structure (L1a) with a = 0 and R¹³ = H; e = 1, 2, 3 or 4, preferably e = 2.

### Linker L²

Linker L² is a peptide spacer. Linker L² may be absent (o = 0) or present (o = 1). Linker L² may also be present in the capping group of masked PBD dimer payload. The combination of a peptide spacer L² and a cleavable linker L³ is well-known in the art. The peptide spacer may be defined by (N(H)-HCR¹⁷-CO)ₙ, wherein R¹⁷ represents an amino acid side chain. Herein, the amino acid may be a natural or a synthetic amino acid. If the amino acid is proline R¹⁷ is connected to nitrogen and (H) is not present. Preferably, linker L² functions as recognition and cleave site for cleaving-enzymes, more preferably the peptides are recognized by specific cleaving enzymes. This allows for cleavage at specific environments in which these cleaving enzymes are expressed such as specific tumors. Since different peptide sequences are cleaved by different enzymes, the L² group also allows customizing the ADC for specific treatments. The peptide sequences may be cleaved by intracellular enzymes and/or extracellular enzymes.

Preferably, the amino acid(s) are all in their L-configuration. n is an integer in the range of 1 - 5, preferably in the range of 2 - 5. Thus, the peptide spacer contains 1 - 5 amino acids. Preferably, the peptide is a dipeptide (n = 2), tripeptide (n = 3) or tetrapeptide (n = 4), most preferably the peptide spacer is a dipeptide. Although any peptide spacer may be used, preferably the peptide spacer is selected from Val-Cit, Val-Ala, Val-Lys, Val-Arg, AcLys-Val-Cit, AcLys-Val-Ala, Glu-Val-Ala, Asp-Val-Ala, iGlu-Val-Ala, Glu-Val-Cit, Glu-Gly-Cit, Glu-Gly-Val, Asp-Val-Cit, iGlu-Val-Cit, Phe-Cit, Phe-Ala, Phe-Lys, Phe-Arg, Ala-Lys, Leu-Cit, Ile-Cit, Trp-Cit, Asn-Asn, Ala-Ala-Asn, Ala-Asn, Asn-Ala, Phe-Phe, Gly, Gly-Gly, Gly-Gly-Gly, Gly-Gly-Gly-Gly, Leu-Gly, Tyr-Gly, Ala-Gly, Pro-Gly, Phe-Gly, Phe-Gly, Ser-Gly, Gly-Phe-Gly, Gly-Gly-Phe-Gly, Gly-Phe-Gly-Gly, Phe-Gly-Gly-Gly, Gly-Gly-Gly-Phe, Phe-Phe-Gly-Gly, Gly-Gly-Phe-Phe, Gly-Gly-Gly-Phe-Gly and Lys, more preferably Val-Cit, Val-Ala, Glu-Val-Ala, Val-Lys, Phe-Cit, Phe-Ala, Phe-Lys, Ala-Ala-Asn, more preferably Glu-Gly-Cit, Val-Cit, Val-Ala, Asn-Asn, Ala-Ala-Asn, Asn-Ala most preferably Glu-Gly-Cit, Val-Cit, Val-Ala or Asn-Ala. Herein, AcLys is e-N-acetyllysine and iGlu is isoglutamate. In one embodiment, L² = Val-Cit. In another embodiment, L² = Val-Ala. In another embodiment, L² = Asn-Ala. In another embodiment, L² = Glu-Gly-Cit.

R¹⁷ represents the amino acid side chain, preferably selected from the side chains of alanine, cysteine, aspartic acid, glutamic acid, phenylalanine, glycine, histidine, isoleucine, lysine, acetyllysine, leucine, methionine, asparagine, pyrrolysine, proline, glutamine, arginine, serine, threonine, selenocysteine, valine, tryptophan, tyrosine and citrulline. Preferred amino acid side chains are those of Val, Cit, Ala, Lys, Arg, AcLys, Phe, Leu, IIe, Trp, Glu, Asp and Asn, more preferably from the side chains of Val, Cit, Ala, Glu and Lys. Alternatively worded, R¹⁷ is preferably selected from CH₃ (Ala), CH₂CH(CH₃)₂ (Leu), CH₂CH₂CH₂NHC(O)NH₂ (Cit), CH₂CH₂CH₂CH₂NH₂ (Lys), CH₂CH₂CH₂NHC(O)CH₃ (AcLys), CH₂CH₂CH₂NHC(=NH)NH₂ (Arg), CH₂Ph (Phe), CH(CH₃)₂ (Val), CH(CH₃)CH₂CH₃ (Ile), CH₂C(O)NH₂ (Asn), CH₂CH₂C(O)OH (Glu), CH₂C(O)OH (Asp) and CH₂(1*H*-indol-3-yl) (Trp). Especially preferred embodiments of R¹⁷ are CH₃ (Ala), CH₂CH₂CH₂NHC(O)NH₂ (Cit), CH₂CH₂CH₂CH₂NH₂ (Lys), CH₂CH₂C(O)OH (Glu) and CH(CH₃)₂ (Val). Most preferably, R¹⁷ is CH₃ (Ala), CH₂CH₂CH₂NHC(O)NH₂ (Cit), CH₂CH₂CH₂CH₂NH₂ (Lys), or CH(CH₃)₂ (Val).

In an especially preferred embodiment, the peptide spacer may be represented by general structure (L2):

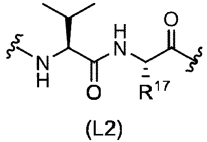

Herein, R¹⁷ is as defined above, preferably R¹⁷ is CH₃ (Ala) or CH₂CH₂CH₂NHC(O)NH₂ (Cit). The wavy lines indicate the connection to (L¹)ₙ and (L³)ₚ, preferably L² according to structure (L3) is connected to (L¹)ₙ via NH and to (L³)ₚ via C(O).

L² may comprise a certain peptide sequence that is cleavable by specific enzymes. If those enzymes are exclusively expressed or overexpressed in the tumor microenvironment or in the endosomal/lysosomal compartment of cancer cells there is an increased probability of targeted release of the PBD dimer in the tumor and reduced release in healthy tissue.

### Linker L³

Linker L³ is a self-cleavable spacer, also referred to as self-immolative spacer. Linker L³ is either absent (p = 0) or present. Linker L³ may also be present in the capping group of a masked PBD. Preferably, linker L³ is present and p = 1. Linker L³ is a self-cleavable spacer, also referred to as self-immolative spacer. L³ is para-aminobenzyloxycarbonyl (PABC) derivative, more preferably a PABC derivative according to structure (L3):

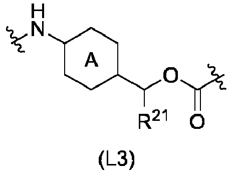

Herein, the wavy lines indicate the connection to L² and to D, wherein the PABC derivative is connected via NH to L².

Ring A is a 5- or 6-membered aromatic or heteroaromatic ring, preferably a 6-membered aromatic or heteroaromatic ring. Suitable 5-membered rings are oxazole, thiazole and furan. Suitable 6-membered rings are phenyl and pyridyl. Ring A may be substituted with a substituent selected from halogen, X²R⁴, N(R⁴)₂, C₁₋₄ alkyl and NO₂. Herein, X² and R⁴ are as defined above, including preferred embodiments thereof. In a preferred embodiment, the optional substituent is selected from F, Ci, Br, OH, OR⁴, SH, NH₂, Et, Me and NO₂. In an especially preferred embodiment, ring A comprises 0 - 2 substituents, more preferably 0 or 1 substituent, most preferably ring A is not substituted. In a preferred embodiment, ring A is 1,4-phenyl, 1,2-phenyl, 2,5-pyridyl or 3,6-pyridyl. Most preferably, A is 1,4-phenyl.

R²¹ is selected from H, R²⁶, C(O)OH and C(O)R²⁶, wherein R²⁶ is C₁ - C₂₄ (hetero)alkyl groups, C₃ -C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²⁸ wherein R²⁸ is independently selected from the group consisting of hydrogen and C₁ - C₄ alkyl groups. Preferably, R²⁶ is C₃ - C₁₀ (hetero)cycloalkyl or polyalkylene glycol. The polyalkylene glycol is preferably a polyethylene glycol or a polypropylene glycol, more preferably - (CH₂CH₂O)_{S}H or -(CH₂CH₂CH₂O)ₛH. The polyalkylene glycol is most preferably a polyethylene glycol, preferably -(CH₂CH₂O)ₛH, wherein s is an integer in the range 1 - 10, preferably 1 - 5, most preferably s = 1, 2, 3 or 4. More preferably, R²¹ is H or C(O)R²⁶, wherein R²⁶ = 4-methyl-piperazine or morpholine. Most preferably, R²¹ is H.

Cleavage of L² results in 1,6-β elimination in linker L³ resulting in decarboxylation and the release of the payload, D. This advantageously allows for increased probability of release of the payload in regions wherein enzymes that are able to cleave L² are overexpressed. In addition, release of a payload can in some cases induce bystander killing which is advantageous for tumours in which not all cancer cells have overexpression of the targeted receptor.

### Linker L⁴

Linker L⁴ is either absent (q = 0) or present (q = 1). Linker L⁴ may be present in the capping group. Linker L³ and linker L⁴ are typically not simultaneously present in linker L^{M} (p + q = 1 or 0). Linker L⁴ is a self-cleavable spacer, also referred to as a self-immolative spacer. L⁴ is a para-glucuronide-meta-amide-benzyloxycarbonyl derivative, more preferably a derivative according to structure (L4):

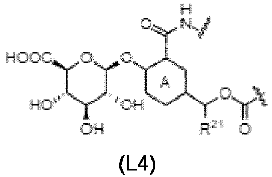

Herein, the wavy lines indicate the connection to Q¹ or Z¹ or L¹, and to D. Typically, the moiety is connected via NH to Q, Z¹ or L¹, preferably to L¹.

A is a 5- or 6-membered aromatic or heteroaromatic ring, preferably a 6-membered aromatic or heteroaromatic ring. Suitable 5-membered rings are oxazole, thiazole and furan. Suitable 6-membered rings are phenyl and pyridyl. In a preferred embodiment, A is 1,3,4-phenyl, 2,4,5-pyridyl or 2,5,6-pyridyl. Most preferably, A is 1,3,4-phenyl.

R²¹ is selected from H, R²⁶, C(O)OH and C(O)R²⁶, wherein R²⁶ is C₁ - C₂₄ (hetero)alkyl groups, C₃ -C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²⁸ wherein R²⁸ is independently selected from the group consisting of hydrogen and C₁ - C₄ alkyl groups. Preferably, R²⁶ is C₃ - C₁₀ (hetero)cycloalkyl or polyalkylene glycol. The polyalkylene glycol is preferably a polyethylene glycol or a polypropylene glycol, more preferably - (CH₂CH₂O)_{S}H or -(CH₂CH₂CH₂O)ₛH. The polyalkylene glycol is most preferably a polyethylene glycol, preferably -(CH₂CH₂O)ₛH, wherein s is an integer in the range 1 - 10, preferably 1 - 5, most preferably s = 1, 2, 3 or 4. More preferably, R²¹ is H or C(O)R²⁶, wherein R²⁶ = 4-methyl-piperazine or morpholine. Most preferably, R²¹ is H.

Linker L⁴ is cleavable by β-glucuronidase, similar to the mechanism in L³ this results in decarboxylation and the release of the payload. An ADC comprising Linker L⁴ is especially useful for treating cancers having an overexpression of β-giucuronidase. β Glucuronidase concentrations in many solid tumors, including lung, breast, and gastrointestinal cancers, as well as in the tumor microenvironment, are reported to be higher than those in normal tissues, and the enzyme is not found in the general circulation. Thus, it is preferred that the conjugates according to the invention comprising L⁴ are used to treat patients suffering from lung, breast, and gastrointestinal cancers.

### The payload

The payload (D) of the present invention is masked PBD dimer, wherein removal of the "mask" (capping group or cap) is required to release active payload. Preferably, D is according to any one of structures (D1) - (D4):

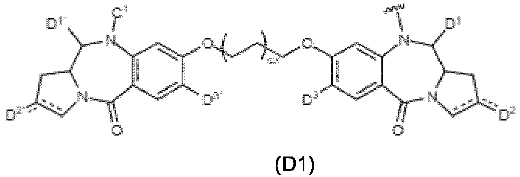

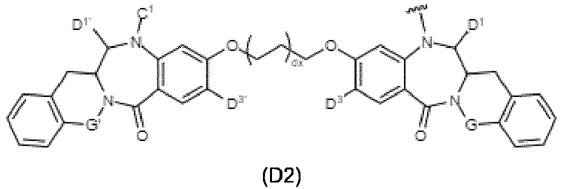

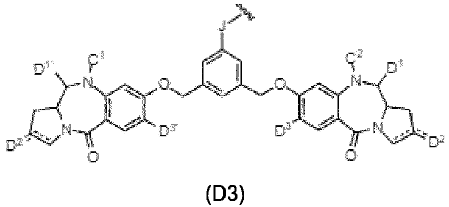

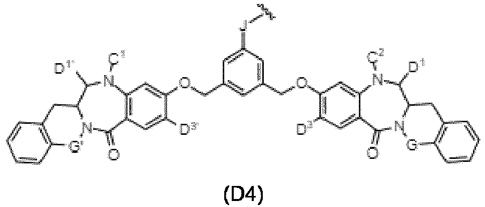

Herein the wavy-lines represent the connection to linker L, - - - represents a single or a double bond within the pyrrolidine ring or between the pyrrolidine ring and D²; dx is an integer in the range of 1-10, preferably dx is an integer in the range of 1-5, more preferably dx is 1 or 2, most preferably dx is 1; D¹ and D¹' are each selected from H, OH or SO₂X; wherein X is a halogen selected from fluorine, chlorine, bromine or iodine; D² and D²' are each selected from -H, -OH, =CH₂ and -CH₃; D³ and D³' are each selected from H, OH, OMe, SMe, NMe₂, NO₂, F, Cl, Br or I, preferably both D³ and D³' are selected from OH, OMe or NMe₂, most preferably D³ and D³' are both OMe; G and G' each represents a single bond or CH₂; J represents a single bond, CH₂, NH or O; and C¹ and C² are capping groups.

Preferably D is according to structure (D2), more preferably (D2) is according to structure (D21):

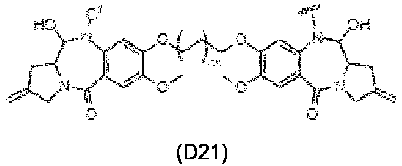

Even more preferably, D is according to structure (D21), wherein dx = 1 or dx = 2, most preferably dx = 1.

In another preferred embodiment, D is according to structure (D22):

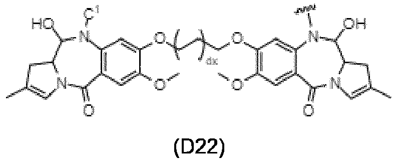

Even more preferably, D is according to structure (D22), wherein dx = 1 or dx = 2, most preferably dx = 2.

### The capping group

The conjugate according to the present invention comprises at least one capping group. In structures (D1) - (D4), (D21) and (D22), C¹ and C² represent capping groups. The capping groups comprise a self immolative spacer. More preferably, C¹ and C² are individually selected from the structures -L⁴-HM, -L³-L²-HM, (C1), (C2) and (C3):

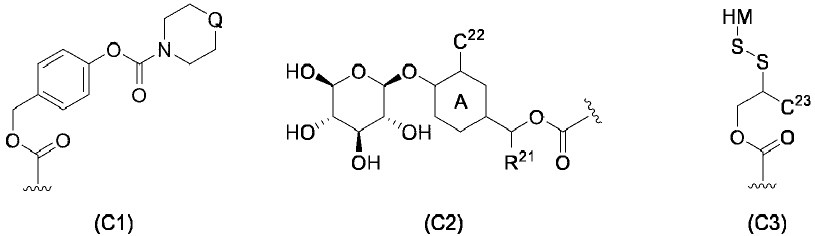

Herein, HM is a hydrophilic moiety. A hydrophilic moiety is a moiety that increases the solubility in aqueous solutions. Preferably, the hydrophilic moiety is selected from sulfonated side-chains, carbamoyl sulfamide side-chains or polyethylene glycol-containing side-chains, more preferably the hydrophilic moiety is selected from carbamoyl sulfamide side-chains or polyethylene glycol side-chains, most preferably the hydrophilic moiety is selected from polyethylene glycol side-chains. Alternatively defined, the hydrophilic moiety is a chain of at least 2 atoms individually selected from C, O, S, and P, more preferably, the hydrophilic moiety is selected from -(L¹)_{c'}-R²³, -C(C²³)₂-(L¹)_{c'}-R²³, most preferably the hydrophilic moiety is selected from the structures (HM1), (HM2), (HM3) and (HM4):

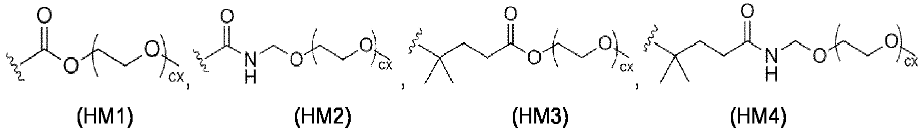

Herein, L¹, C²³ and c' are as defined above, each R²³ is individually hydrogen and C₁ - C₆ alkyl, cx is an integer 1-5, more preferably cx is 1 or 2, and c' is 0 or 1, preferably c' = 1.

It is especially preferred that HM in (C3) is connected to the disulfide via a carbon atom, wherein said carbon atom is connected to at least one C²³ group, more preferably HM in structure (C3) is structure (HM4).

Q is selected from O, NH, NMe, or N-HM, preferably Q is O or NMe, most preferably Q is NMe.

A is a 5- or 6-membered aromatic or heteroaromatic ring, preferably a 6-membered aromatic or heteroaromatic ring. Suitable 5-membered rings are oxazole, thiazole and furan. Suitable 6-membered rings are phenyl and pyridyl. In a preferred embodiment, A is 1,4-phenyl, 2,5-pyridyl or 3,6-pyridyl. Most preferably, A is 1,4-phenyl, wherein C²² is connected to the 2-position.

C²¹ is selected from H, C²⁶, C(O)OH and C(O)C²⁶, wherein C²⁶ is C₁ - C₂₄ (hetero)alkyl groups, C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NC²⁸ wherein C²⁸ is independently selected from the group consisting of hydrogen and C₁ - C₄ alkyl groups. Preferably, C²⁶ is C₃ - C₁₀ (hetero)cycloalkyl or polyalkylene glycol. The polyalkylene glycol is preferably a polyethylene glycol or a polypropylene glycol, more preferably -(CH₂CH₂O)ₛH or -(CH₂CH₂CH₂O)ₛH. The polyalkylene glycol is most preferably a polyethylene glycol, preferably -(CH₂CH₂O)ₛH, wherein s is an integer in the range 1 - 10, preferably 1-5, most preferably s = 1, 2, 3 or 4. More preferably, C²¹ is H or C(O)C²⁶, wherein C²⁶ = 4-methyl-piperazine or morpholine. Most preferably, C²¹ is H.

C²² is selected from HM, NO₂ or H, more preferably C²² is selected from NO₂ or H.

C²³ is H or a C₁-C₃ alkyl group, preferably C²³ is H or methyl, more preferably C²³ is H.

### Preferred embodiments

In one embodiment, the linker does not comprise a self-immolative spacer. The advantage of using a conjugate without a self-immolative spacer is that when the conjugate is decomposed in the lysosome, the payload remains connected to a charged moiety and is thus unable to escape the cell. Contrary to the linker, the cap is cleavable, so that the payload has sufficient potency. This embodiment is particularly advantageous for treating cancers in which very low bystander killing is desired, since bystander killing also affects healthy cells. Thus, conjugates comprising a non-self immolative linkers are preferred for use of treating cancers selected from liquid cancers or solid cancers that homogeneously express the target receptor, more preferably the conjugates comprising a non-cleavable linker are for the use of treating liquid cancers.

In a preferred embodiment, the conjugate according to the present invention comprises a linker comprising a self-immolative spacer. The advantage of using a cleavable linker is that bystander killing may be induced. The conjugate according to the present invention may have the same cleaving mechanism for the cap(s) as for the linker. This advantageously allows for immediate release of the payload in its most potent form. Alternatively the cleaving mechanism differs for the cap(s) and the linker. The advantage of having a different cleaving mechanism for the cap as the linker is that the cap is released at different stage than the payload. This may increase the tolerability of the conjugate. In a preferred embodiment, the cap is cleaved under extracellular conditions, while the linker is cleaved under intracellular conditions.

In a preferred embodiment, the conjugate comprises a payload is according to structures (D1) or (D2), wherein the conjugate comprises L^{M} that comprises a self-immolative spacer. More preferably, the self-immolative linker in L^{M} is cleaved by another mechanism than the self-immolative spacer in the cap. Even more preferably, C¹ is selected from -L⁴-HM, (C2) or (C3) and L^{M} comprises (L6) and (L7), most preferably C¹ is (C3).

In another preferred embodiment, L^{M} comprises a self-immolative linker which is cleaved by the same mechanism as C¹ and if present as C². More preferably, L^{M}, C¹ and if present C₂ comprise L² and L³, even more preferably , L^{M}, C¹ and if present C² comprise L² and L³, wherein L² is selected from Glu-Gly-Cit, Glu-Gly-Val, Ala-Asn, Asn-Ala, Pro-Leu-Gly, Asn-Asn, most preferably L² is Glu-Gly-Cit or Asn-Ala.

In a preferred embodiment, the conjugate is a DAR1 or DAR2 conjugate, L^{M}-D has a structure selected from the group consisting of (LM1) - (LM4): 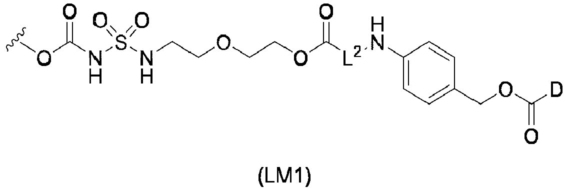 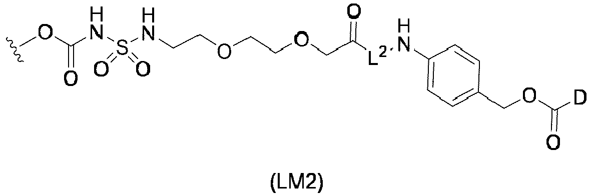 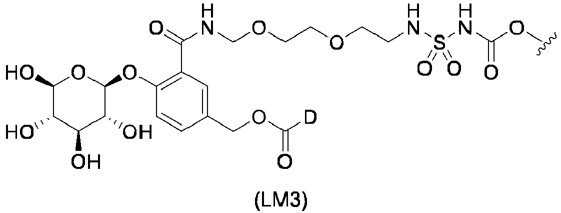 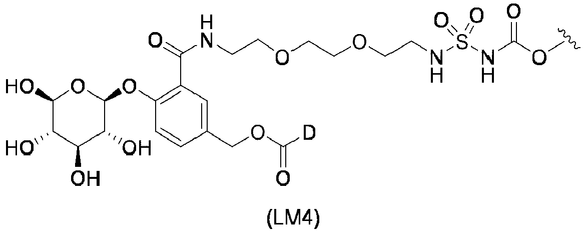 wherein the wavy line indicates the connection to Z¹.

In another preferred embodiment antibody-conjugate is a DAR4 conjugate, having a linker-payload moiety according to structure (L5) or (L6): 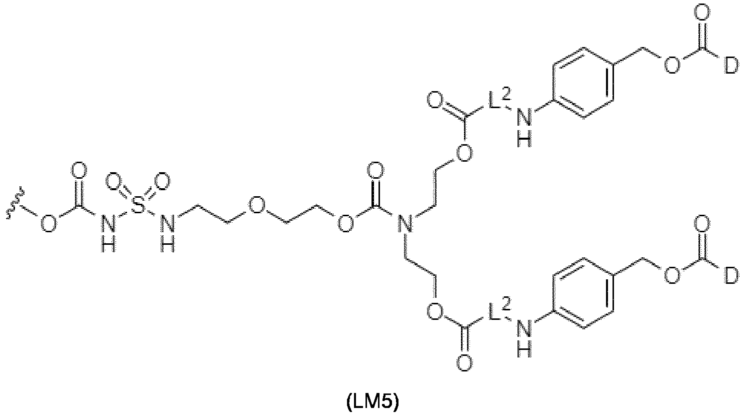 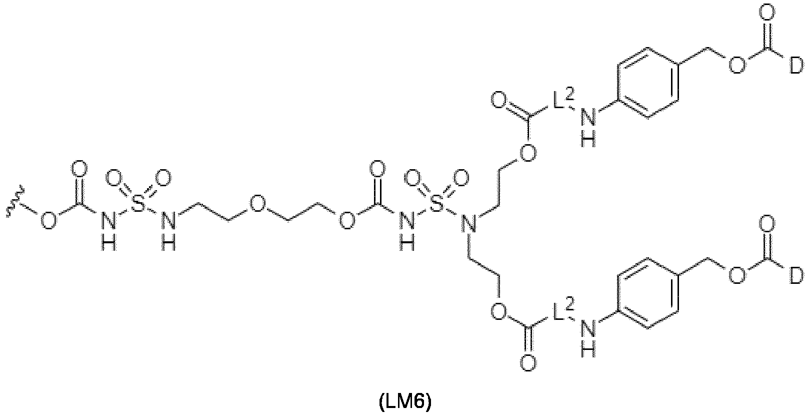 wherein:
- the wavy lines indicates the connection to Z¹;
- L², L⁴, o, q and D are as defined above.

More preferably, the linker-payload moiety is according to (LM7) or (LM8):

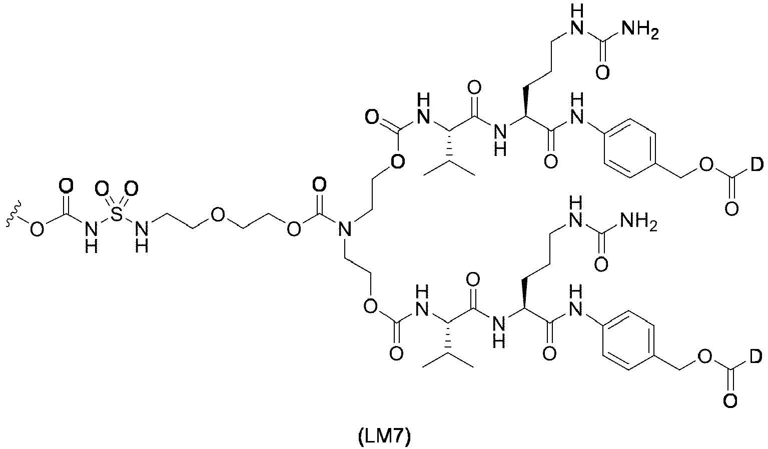

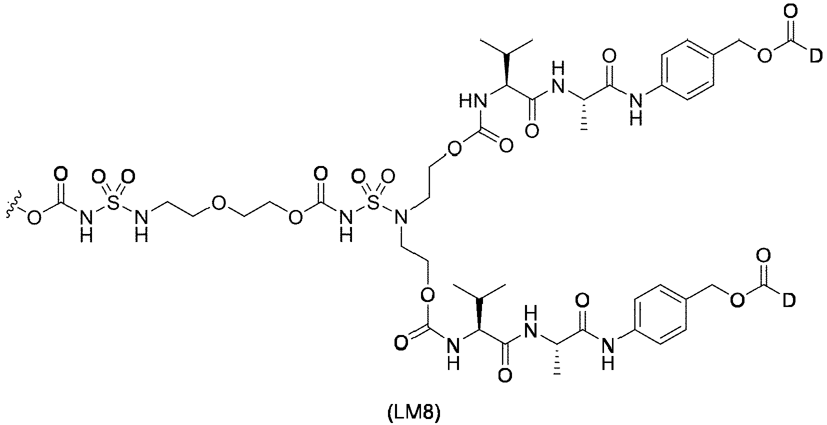

In a preferred embodiment, the conjugate according to the invention is selected from structure **(4) - (7):**

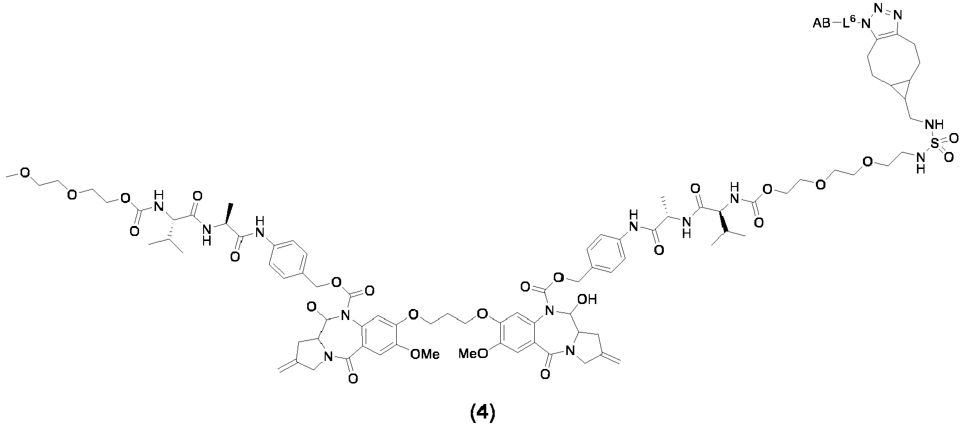

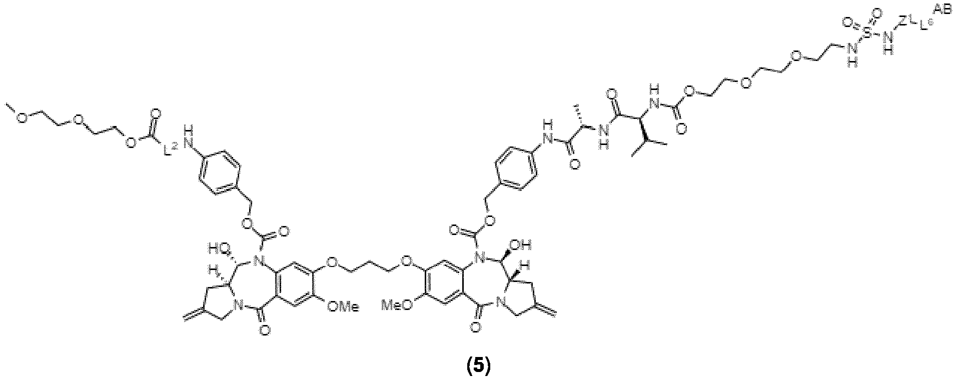

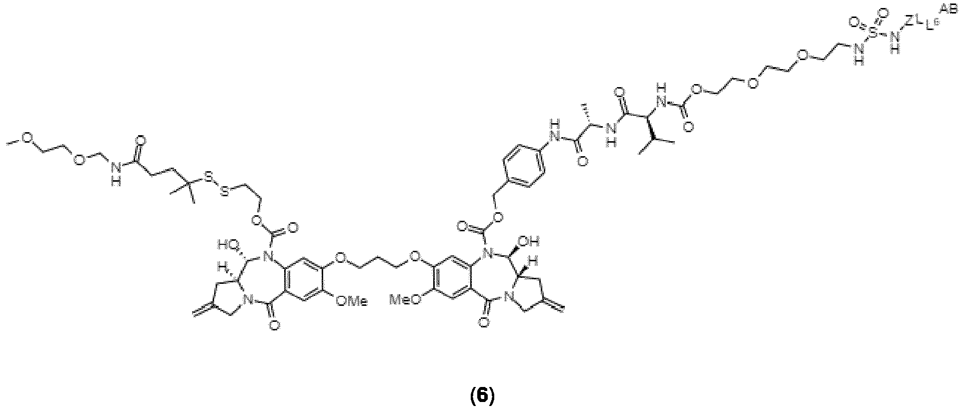

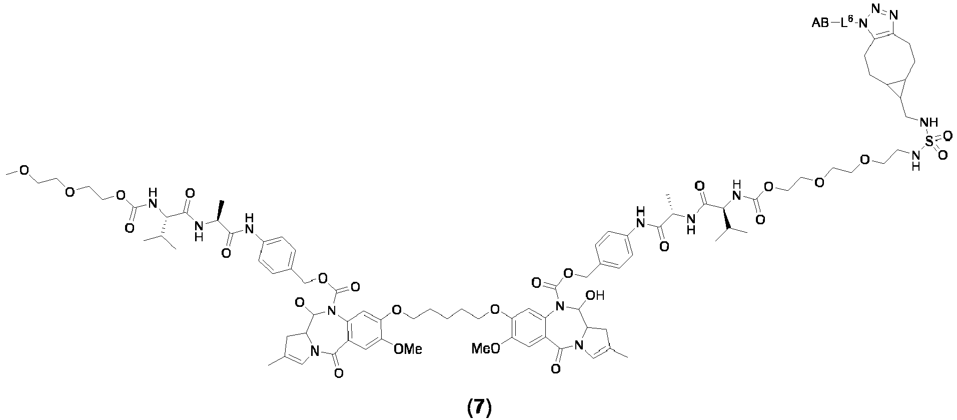

Preferably, L² is chosen so that the cap contains a peptide spacer selected from from Glu-Gly-Cit, Glu-Gly-Val, Val-Cit, Val-Ala, Val-Lys, Val-Arg, Phe-Cit, Phe-Ala, Phe-Lys, Phe-Arg, Ala-Lys, Leu-Cit, Ile-Cit, Trp-Cit, Ala-Ala-Asn, Ala-Asn, Asn-Ala, Phe-Phe, Gly-Gly, Leu-Gly, Tyr-Gly, Ala-Gly, Pro-Gly, Phe-Gly, Phe-Gly, Ser-Gly, Gly-Phe-Gly, more preferably Val-Cit, Val-Ala, Val-Lys, Phe-Cit, Phe-Ala, Phe-Lys, Asn-Asn, Asn-Ala or Glu-Gly-Cit more preferably Val-Cit, Val-Ala, Asn-Ala or Glu-Gly-Cit, most preferably Val-Cit or Val-Ala. Herein, AcLys is e-N-acetyllysine and iGlu is isoglutamate. In one embodiment, L² = Val-Cit. In one embodiment, L² = Val-Ala.

In a preferred embodiment, the conjugate is according to structure **(8):**

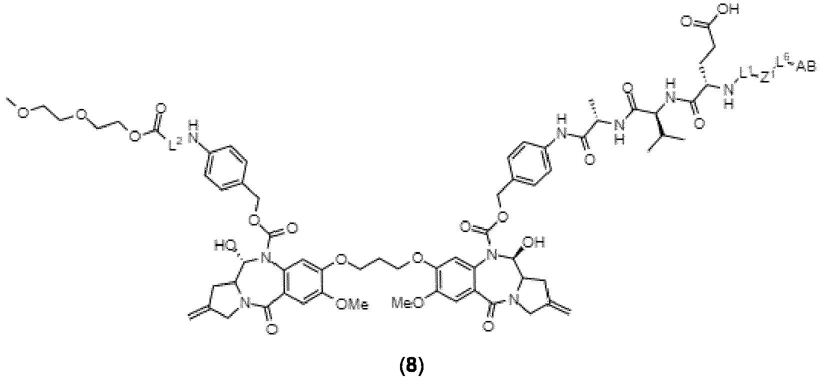

More preferably, the conjugate is according to structure **(8),** wherein linker L² in the cap is selected from Glu-Gly-Cit, Glu-Gly-Val, Ala-Asn, Asn-Ala, Pro-Leu-Gly or Asn-Asn.

### Medical treatment

In a second aspect, the invention concerns the conjugate according to present invention for the use as medicament. More preferably, the conjugate is used for the treatment of cancer.

The invention further concerns a pharmaceutical composition comprising the antibody-payload conjugate according to the invention and a pharmaceutically acceptable carrier. Preferably, the pharmaceutical composition is used for medical treatment, more preferably the pharmaceutical composition is used for the treatment of cancer.

The inventors have surprisingly found that the antibody-drug conjugates according to the invention are superior to conventional masked PBD conjugates in term of safety and/or efficacy, such that the therapeutic index of the antibody-conjugate according to the invention is increased with respect to conventional masked PBD antibody-conjugates. Preferably, increasing the therapeutic index of an antibody-conjugate is selected from:
a) increasing the therapeutic efficacy of the antibody-conjugate; and/or
b) increasing the tolerability of the antibody-conjugate.

Increase in therapeutic efficacy of the antibody-conjugates according to the invention may take the form of a reduction in tumour size and/or a prolonged period of regression, when compared to conventional masked PBD ADC. Increase in tolerability of the antibody-conjugates according to the invention may take the form of a reduction in signs of toxicity, compared to administration of a masked PBD ADC made with a conventional technology. The reduction in signs may also be referred to as a reduction in symptoms or side-effects of cancer treatment, and may involve one or more clinical signs such as reduced reduction in body weight, reduced reduction in mobility, reduced reduction in food intake and/or one or more toxicity parameters, such as improved blood chemistry, hematology, and/or histopathology.

### Process for the synthesis of the antibody-drug conjugate

In a third aspect, the invention concerns a process for preparing the antibody-drug conjugate according to the invention. The process according to the invention comprises reacting a modified antibody of structure AB-(L⁶-F¹)ₓ with a linker-drug construct. The reaction is a conjugation reaction, which forms a covalent attachment between the masked PBD payload and the antibody. The reaction is a metal-free click reaction, and forms an antibody-drug conjugate wherein the drug is covalently attached to the antibody via connecting group Z¹ that is formed by a metal-free click reaction between Q¹ and F¹. Preferably, the metal-free click reaction is a 1 ,3-dipolar cycloaddition.

In the process according to the invention, the modified antibody of structure AB-(L⁶-F¹)ₓ, wherein AB is an antibody, L¹ is a linker, F¹ is a click probe capable of reacting with Q¹ in a metal-free click reaction and x is an integer in the range of 1 - 8. Upon reacting F¹ with Q¹ of the linker-drug construct according to structure (9) or (10) via a metal-free click reaction, connecting group Z¹ is formed. In one embodiment, the modified antibody may be referred to as AB-(F¹)ₓ. Methods of preparing modified antibody are known in the art, e.g. from WO 2014/065661, WO 2016/170186 and WO 2016/053107, which are incorporated herein by reference. From the same documents, the conjugation reaction between the modified glycoprotein and a linker-drug construct comprising a cytotoxin and a click probe is known to the skilled person.

The process according the invention comprises the following steps:
i) contacting an antibody comprising y core N-acetylglucosamine (GlcNAc) moieties, with a compound of the formula S(F¹)ₓ-P in the presence of a catalyst, wherein S(F¹)ₓ is a sugar derivative comprising x reactive groups F¹ capable of reacting with a reactive group Q, and P is a nucleoside mono- or diphosphate, and wherein the catalyst is capable of transferring the S(F¹)ₓ moiety to the core-GlcNAc moiety, to obtain a modified antibody according to Formula **(26):**

   AB-[GlcNAc(Fuc)_{w}-S{F¹}_{x]y} **(26)**

   wherein
   - Fuc is fucose;
   - wis0or1;
   - x is 1 or 2, preferably x is 1; and
   - y is 1 or 2, preferably y is 2;
ii) optionally reacting the modified antibody according to formula AB-[GlcNac(Fuc)w-S{F¹}]_{y}, wherein y = 2, with a compound represented by formula **(27):** 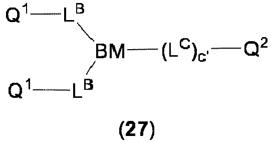 to obtain a modified antibody according to Formula **(28):** 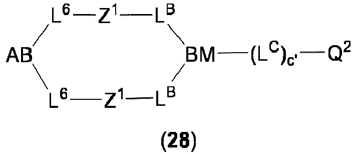
iii) reacting the modified antibody according to Formula **(26)** or **(28)** with the compound selected from structures **(9), (10)** and **(11),** to obtain the antibody-conjugate according to structure **(1)**

   Q¹-L^{M}-D (9)

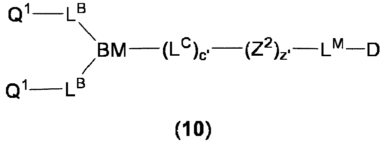

   F²-L^{M}-(D)ₓ **(11)**

   wherein if optional step ii) is performed, the compound used in step iii) is represented by structure **(11),** and if optional step ii) is not performed, the compound used in step iii) is represented by structure **(9)** or **(10).**

### Metal-free click reaction

Metal-free click reactions are well-known in the art (see e.g. from WO 2014/065661 and Nguyen and Prescher, Nature rev. 2020, doi: 10.1038/s41570-020-0205-0, both incorporated by reference) and may typically take the form of a 1,3-dipolar cycloaddition or (4+2) cycloaddition. The alkyne-azide cycloaddition may be strain-promoted (e.g. a strain-promoted alkyne-azide cycloaddition, SPAAC). In a preferred embodiment, the bioconjugation reaction is a metal-free strain-promoted cycloaddition, most preferably metal-free strain-promoted alkyne-azide cycloaddition. In a preferred embodiment, conjugation is accomplished via a cycloaddition, such as a (4+2) cycloaddition or a 1,3-dipolar cycloaddition, preferably the 1,3-dipolar cycloaddition.

A typical (4+2) cycloaddition is the Diels-Alder reaction, wherein Q¹ is a diene or a dienophile. As appreciated by the skilled person, the term "diene" in the context of the Diels-Alder reaction refers to 1,3-(hetero)dienes, and includes conjugated dienes (R₂C=CR-CR=CR₂), imines (e.g. R₂C=CR-N=CR₂ or R₂C=CR-CR=NR, R₂C=N-N=CR₂) and carbonyls (e.g. R₂C=CR-CR=O or O=CR-CR=O). Hetero-Diels-Alder reactions with N- and O-containing dienes are known in the art. Any diene known in the art to be suitable for (4+2) cycloadditions may be used as reactive group Q¹. Preferred dienes include tetrazines, 1,2-quinones and triazines. Although any dienophile known in the art to be suitable for (4+2) cycloadditions may be used as reactive group Q¹, the dienophile is preferably an alkene or alkyne group as described above, most preferably an alkyne group. For conjugation via a (4+2) cycloaddition, it is preferred that Q¹ is a dienophile (and F¹ is a diene), more preferably Q¹ is or comprises an alkynyl group.

For a 1,3-dipolar cycloaddition, Q¹ is a 1,3-dipole or a dipolarophile. Any 1,3-dipole known in the art to be suitable for 1,3-dipolar cycloadditions may be used as reactive group Q¹. Preferred 1,3-dipoles include azido groups, nitrone groups, nitrile oxide groups, nitrile imine groups and diazo groups. Although any dipolarophile known in the art to be suitable for 1,3-dipolar cycloadditions may be used as reactive groups Q¹, the dipolarophile is preferably an alkene or alkyne group, most preferably an alkyne group. For conjugation via a 1,3-dipolar cycloaddition, it is preferred that Q¹ is a dipolarophile (and F¹ is a 1,3-dipole), more preferably Q¹ is or comprises an alkynyl group.

Thus, in a preferred embodiment, Q¹ is selected from dipolarophiles and dienophiles.

### Click probe Q¹

Click probe Q¹ is used in the conjugation reaction to connect the linker-drug construct to the antibody of structure AB-(F¹)ₓ. Q¹ is reactive towards click probe F¹ in a metal-free click reaction. Such click probes are known in the art and include cyclic alkene, cyclic alkyne, azide, tetrazine, triazine, nitrone, nitrile oxide, nitrile imine, diazo compound, *ortho*-quinone, dioxothiophene and sydnone. Preferably, Q¹ is a cyclic alkene or a cyclic alkyne moiety, most preferably Q¹ is a cyclic alkyne moiety.

Thus, in an especially preferred embodiment, Q¹ comprises a cyclic (hetero)alkyne moiety. The alkynyl group may also be referred to as a (hetero)cycloalkynyl group, i.e. a heterocycloalkynyl group or a cycloalkynyl group, wherein the (hetero)cycloalkynyl group is optionally substituted. Preferably, the

(hetero)cycloalkynyl group is a (hetero)cycloheptynyl group, a (hetero)cyclooctynyl group, a (hetero)cyclononynyl group or a (hetero)cyclodecynyl group. Herein, the (hetero)cycloalkynes may optionally be substituted. Preferably, the (hetero)cycloalkynyl group is an optionally substituted (hetero)cycloheptynyl group or an optionally substituted (hetero)cyclooctynyl group. Most preferably, the (hetero)cycloalkynyl group is a (hetero)cyclooctynyl group, wherein the (hetero)cyclooctynyl group is optionally substituted.

In an especially preferred embodiment, Q¹ comprises a (hetero)cycloalkynyl or (hetero)cycloalkenyl group and is according to structure (Q1):

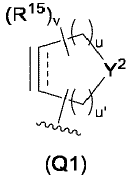

Herein:
- the bond depicted as 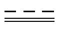 is a double bond or a triple bond; R¹⁵ is independently selected from the group consisting of hydrogen, halogen, -OR¹⁶, -NO₂, -CN, -S(O)₂R¹⁶, -S(O)₃⁽⁻⁾, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y² is C(R³¹)₂, O, S, S⁽⁺⁾R³¹, S(O)R³¹, S(O)=NR³¹ or NR³¹, wherein S⁽⁺⁾ is a cationic sulphur atom counterbalanced by B⁽⁻⁾, wherein B⁽⁻⁾ is an anion, and wherein each R³¹ individually is R¹⁵ or a connection with D, connected via L;
- u is 0, 1, 2, 3, 4 or 5;
- u' is 0, 1, 2, 3, 4 or 5, wherein u + u' = 0, 1, 2, 3, 4, 5, 6, 7 or 8;
- v = an integer in the range 0 - 16;

Typically, v = (u + u') × 2 (when the connection to L, depicted by the wavy bond, is via Y²) or [(u + u') × 2] - 1 (when the connection to L, depicted by the wavy bond, is via one of the carbon atoms).

In a preferred embodiment of structure (Q1), reactive group Q comprises a (hetero)cycloalkynyl group and is according to structure (Q1a): 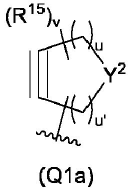 Herein,
- R¹⁵ and Y² are as defined above;
- u is 0, 1, 2, 3, 4 or 5;
- u' is 0, 1, 2, 3, 4 or 5, wherein u + u' = 4, 5, 6, 7 or 8;
- v = an integer in the range 8 - 16.
In a preferred embodiment, u + u' = 4, 5 or 6, more preferably u + u' = 5. In a preferred embodiment, v = 8, 9 or 10, more preferably v = 9 or 10, most preferably v = 10.

In a preferred embodiment, Q¹ is a (hetero)cycloalkynyl group selected from the group consisting of (Q2) - (Q20), (Q38a) depicted here below.

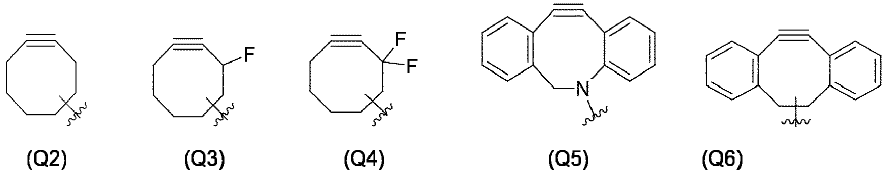

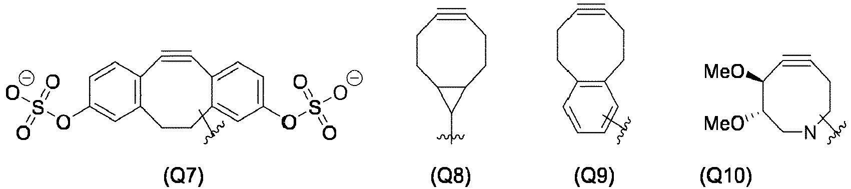

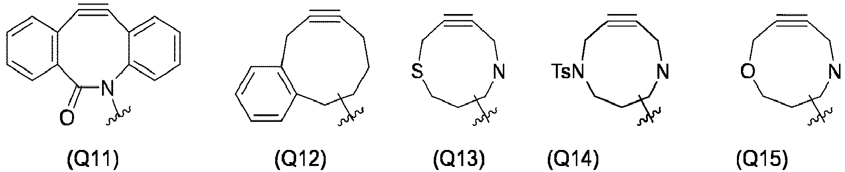

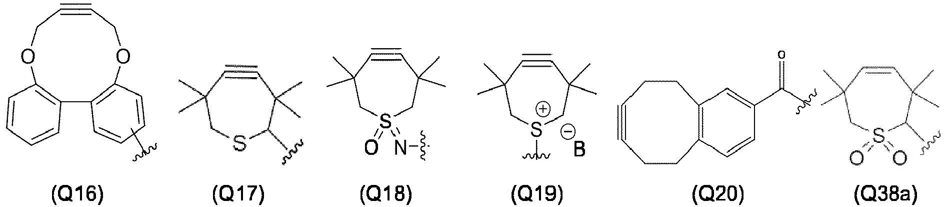

Herein, the connection to L, depicted with the wavy bond, may be to any available carbon or nitrogen atom of Q¹. The nitrogen atom of (Q10), (Q13), (Q14) and (Q15) may bear the connection to L, or may contain a hydrogen atom or be optionally functionalized. B⁽⁻⁾ is an anion, which is preferably selected from ⁽⁻⁾OTf, Cl⁽⁻⁾, Br⁽⁻⁾ or I⁽⁻⁾, most preferably B⁽⁻⁾ is ⁽⁻⁾OTf. In the conjugation reaction, B⁽⁻⁾ does not need to be a pharmaceutically acceptable anion, since B⁽⁻⁾ will exchange with the anions present in the reaction mixture anyway. In case (Q19) is used for Q¹, the negatively charged counter-ion is preferably pharmaceutically acceptable upon isolation of the conjugate according to the invention, such that the conjugate is readily useable as medicament.

In a further preferred embodiment, Q¹ is a (hetero)cycloalkynyl group selected from the group consisting of (Q21) - (Q38a) depicted here below.

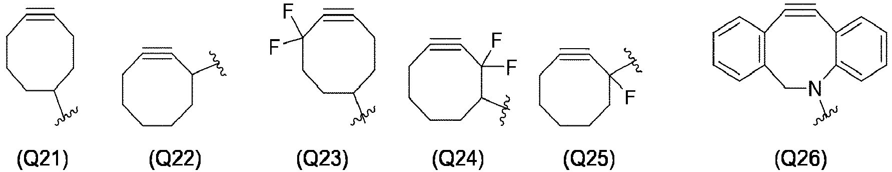

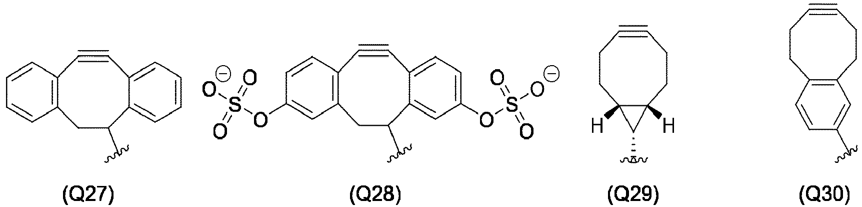

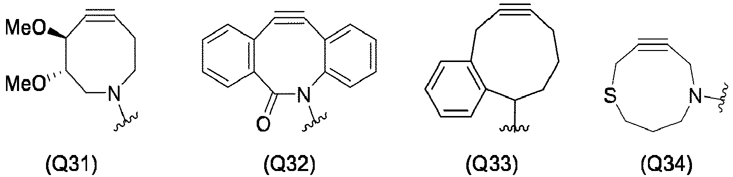

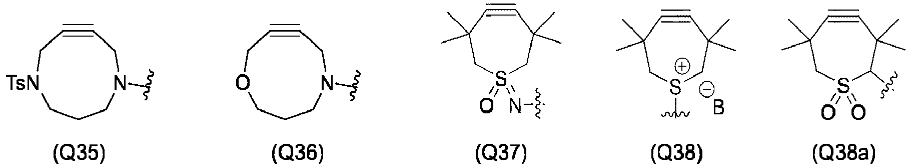

In structure (Q38), B⁽⁻⁾ is an anion, which is preferably selected from ⁽⁻⁾OTf, Cl⁽⁻⁾, Br⁽⁻⁾ or I⁽⁻⁾, most preferably B⁽⁻⁾ is ⁽⁻⁾OTf.

In a preferred embodiment, Q¹ comprises a (hetero)cyclooctyne moiety or a (hetero)cycloheptyne moiety, preferably according to structure (Q8), (Q26), (Q27), (Q28) or (Q37), which are optionally substituted. Each of these preferred options for Q are further defined here below.

Thus, in a preferred embodiment, Q¹ comprises a heterocycloheptyne moiety according to structure (Q37), also referred to as a TMTHSI, which is optionally substituted. Preferably, the heterocycloheptyne moiety according to structure (Q37) is not substituted.

In an alternative preferred embodiment, Q¹ comprises a cyclooctyne moiety according to structure (Q8), more preferably according to (Q29), also referred to as a bicyclo[6.1.0]non-4-yn-9-yl] group (BCN group), which is optionally substituted. Preferably, the cyclooctyne moiety according to structure (Q8) or (Q29) is not substituted. In the context of the present embodiment, Q¹ preferably is a (hetero)cyclooctyne moiety according to structure (Q39) as shown below, wherein V is (CH₂)_{I} and I is an integer in the range of 0 to 10, preferably in the range of 0 to 6. More preferably, I is 0, 1, 2, 3 or 4, more preferably I is 0, 1 or 2 and most preferably I is 0 or 1. In the context of group (Q39), I is most preferably 1. Most preferably, Q¹ is according to structure (Q42), defined further below.

In an alternative preferred embodiment, Q¹ comprises a (hetero)cyclooctyne moiety according to structure (Q26), (Q27) or (Q28), also referred to as a DIBO, DIBAC, DBCO or ADIBO group, which are optionally substituted. In the context of the present embodiment, Q¹ preferably is a (hetero)cyclooctyne moiety according to structure (Q40) or (Q41) as shown below, wherein Y¹ is O or NR¹¹, wherein R¹¹ is independently selected from the group consisting of hydrogen, a linear or branched C₁ - C₁₂ alkyl group or a C₄ - C₁₂ (hetero)aryl group. The aromatic rings in (Q40) are optionally O-sulfonylated at one or more positions, whereas the rings of (Q41) may be halogenated at one or more positions. Preferably, the (hetero)cyclooctyne moiety according to structure (Q40) or (Q41) is not further substituted. Most preferably, Q¹ is according to structure (Q43), defined further below.

In an alternative preferred embodiment, Q¹ comprises a heterocycloheptynyl group and is according to structure (Q37).

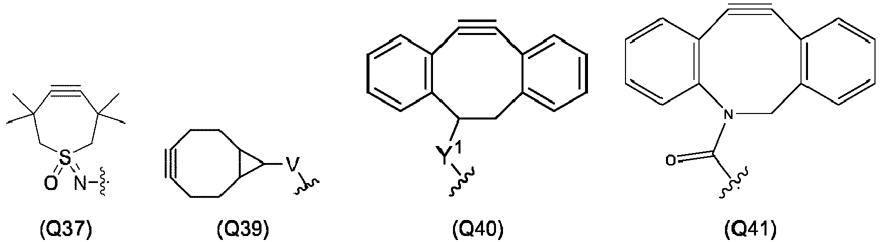

In an especially preferred embodiment, Q¹ comprises a (hetero)cyclooctynyl group and is according to structure (Q43):

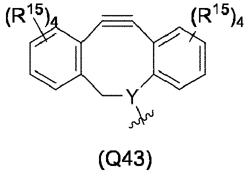

In an especially preferred embodiment, Q¹ comprises a cyclooctynyl group and is according to structure (Q42):

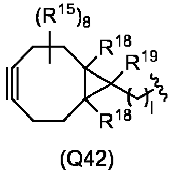

Herein:
- R¹⁵ is independently selected from the group consisting of hydrogen, halogen, -OR¹⁶, -NO₂, -CN, -S(O)₂R¹⁶, -S(O)₃⁽⁻⁾,C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁸ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁹ is selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted, or R¹⁹ is a second occurrence of Q or D connected via a spacer moiety; and
- I is an integer in the range 0 to 10.

In a preferred embodiment of the reactive group according to structure (Q42), R¹⁵ is independently selected from the group consisting of hydrogen, halogen, -OR¹⁶, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and C₁ - C₆ alkyl, most preferably all R¹⁵ are H. In a preferred embodiment of the reactive group according to structure (Q42), R¹⁸ is independently selected from the group consisting of hydrogen, C₁ - C₆ alkyl groups, most preferably both R¹⁸ are H. In a preferred embodiment of the reactive group according to structure (Q42), R¹⁹ is H. In a preferred embodiment of the reactive group according to structure (Q42), I is 0 or 1, more preferably I is 1.

In an especially preferred embodiment, Q comprises a (hetero)cyclooctynyl group and is according to structure (Q43):

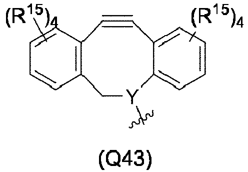

Herein:
- R¹⁵ is independently selected from the group consisting of hydrogen, halogen, -OR¹⁶, -NO₂, -CN, - S(O)₂R¹⁶, -S(O)₃⁽⁻⁾, C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y is N or CR¹⁵.

In a preferred embodiment of the reactive group according to structure (Q43), R¹⁵ is independently selected from the group consisting of hydrogen, halogen, -OR¹⁶, -S(O)₃⁽⁻⁾, - C₆ alkyl groups, C₅ - C₆ (hetero)aryl groups, wherein R¹⁶ is hydrogen or - C₆ alkyl, more preferably R¹⁵ is independently selected from the group consisting of hydrogen and -S(O)₃⁽⁻⁾. In a preferred embodiment of the reactive group according to structure (Q43), Y is N or CH, more preferably Y = N.

In an alternative preferred embodiment, Q¹ comprises a cyclic alkene moiety. The alkenyl group Q¹ may also be referred to as a (hetero)cycloalkenyl group, i.e. a heterocycloalkenyl group or a cycloalkenyl group, preferably a cycloalkenyl group, wherein the (hetero)cycloalkenyl group is optionally substituted. Preferably, the (hetero)cycloalkenyl group is a (hetero)cyclopropenyl group, a (hetero)cyclobutenyl group, a norbornene group, a norbornadiene group, a *trans*-(hetero)cycloheptenyl group, a *trans-*(hetero)cyclooctenyl group, a *trans*-(hetero)cyclononenyl group or a *trans*-(hetero)cyclodecenyl group, which may all optionally be substituted. Especially preferred are (hetero)cyclopropenyl groups, *trans-*(hetero)cycloheptenyl group or *trans*-(hetero)cyclooctenyl groups, wherein the (hetero)cyclopropenyl group, the *trans*-(hetero)cycloheptenyl group or the *trans*-(hetero)cyclooctenyl group is optionally substituted. Preferably, Q¹ comprises a cyclopropenyl moiety according to structure (Q44), a hetereocyclobutene moiety according to structure (Q45), a norbornene or norbornadiene group according to structure (Q46), a *trans*-(hetero)cycloheptenyl moiety according to structure (Q47) or a *trans-*(hetero)cyclooctenyl moiety according to structure (Q48). Herein, Y³ is selected from C(R²³)₂, NR²³ or O, wherein each R²³ is individually hydrogen, - C₆ alkyl or is connected to L, optionally via a spacer, and the bond labelled - - - is a single or double bond. In a further preferred embodiment, the cyclopropenyl group is according to structure (Q49). In another preferred embodiment, the *trans*-(hetero)cycloheptene group is according to structure (Q50) or (Q51). In another preferred embodiment, the *trans-*(hetero)cyclooctene group is according to structure (Q52), (Q53), (Q54), (Q55) or (Q56).

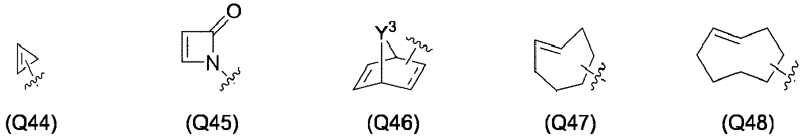

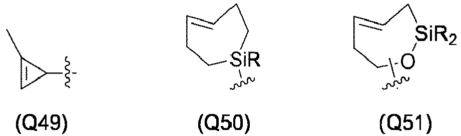

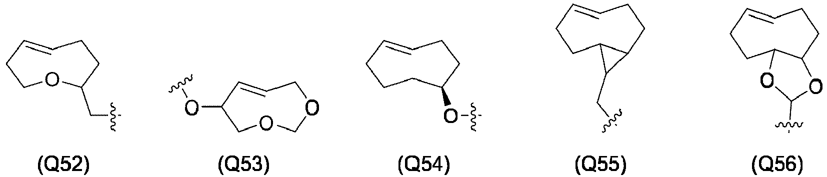

Herein, the R group(s) on Si in (Q50) and (Q51) are typically alkyl or aryl, preferably C₁-C₆ alkyl.

Q² is a group that reacts with F² to form a further connecting group Z². Preferred options for Z² are defined above. In an especially preferred embodiment, Q² is selected from the same preferred embodiments as Q¹, wherein Q¹ and Q² are individually selected. In case the compound comprises two groups Q¹, e.g. for **(10)** or **(27),** it is preferred that both groups are identical.

### Reactive moiety F¹

F¹ is reactive towards Q¹ in the conjugation reaction, wherein the conjugation reaction is a cycloaddition. As the skilled person will understand, the options for F¹ are the same as those for Q¹, provided that F¹ and Q¹ are reactive towards each other. Thus, F¹ comprises a click probe. The click probe is reactive in a cycloaddition (click reaction) and is preferably selected from an azide, a tetrazine, a triazine, a nitrone, a nitrile oxide, a nitrile imine, a diazo compound, an *ortho*-quinone, a dioxothiophene, a sydnone, an alkene moiety and an alkyne moiety. Preferably, the click probe comprises or is an azide, a tetrazine, a triazine, a nitrone, a nitrile oxide, a nitrile imine, a diazo compound, an *ortho*-quinone, a dioxothiophene or a sydnone, most preferably an azide. In a preferred embodiment, F¹ is an azide.

More than one reactive group F¹ may be present in the antibody. The reactive group F¹ in the antibody may be naturally present or may be placed in the antibody by a specific technique, for example a (bio)chemical or a genetic technique. The reactive group that is placed in the antibody is prepared by chemical synthesis, for example an azide or a terminal alkyne. Methods of preparing modified antibodies are known in the art, e.g. from WO 2014/065661, WO 2016/170186 and WO 2016/053107, which are incorporated herein by reference. From the same documents, the conjugation reaction between the modified antibody and a linker-toxin-construct is known to the skilled person.

Preferably, F¹ is a click probe reactive towards a (hetero)cycloalkene and/or a (hetero)cycloalkyne, and is typically selected from the group consisting of azide, tetrazine, triazine, nitrone, nitrile oxide, nitrile imine, diazo compound, *ortho*-quinone, dioxothiophene and sydnone. Preferred structures for the reactive group are structures (F1) - (F10) depicted here below.

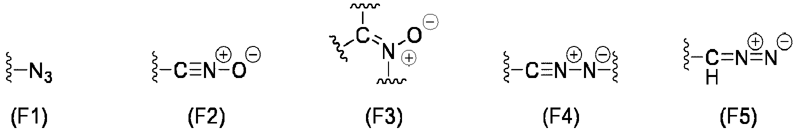

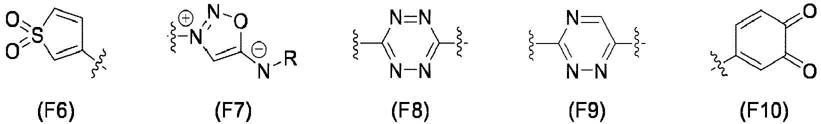

Herein, the wavy bond represents the connection to the payload. For (F3), (F4), (F8) and (F9), the payload can be connected to any one of the wavy bonds. The other wavy bond may then be connected to an R group selected from hydrogen, - C₂₄ alkyl groups, C₂ - C₂₄ acyl groups, C₃ - C₂₄ cycloalkyl groups, C₂ - C₂₄ (hetero)aryl groups, C₃ - C₂₄ alkyl(hetero)aryl groups, C₃ - C₂₄ (hetero)arylalkyl groups and - C₂₄ sulfonyl groups, each of which (except hydrogen) may optionally be substituted and optionally interrupted by one or more heteroatoms selected from O, S and NR³² wherein R³² is independently selected from the group consisting of hydrogen and - C₄ alkyl groups. The skilled person understands which R groups may be applied for each of the groups F¹. For example, the R group connected to the nitrogen atom of (F3) may be selected from alkyl and aryl, and the R group connected to the carbon atom of (F3) may be selected from hydrogen, alkyl, aryl, acyl and sulfonyl. Preferably, the reactive moiety F¹ is selected from azides or tetrazines. Most preferably, the reactive moiety F¹ is an azide.

F² is a group that reacts with Q² to form a further connecting group Z². Preferred options for Z² are defined above. In an especially preferred embodiment, F² is selected from the same preferred embodiments as F¹, wherein F¹ and F² are individually selected.

### Obtaining the modified antibody

The modified antibody is obtained by contacting an antibody comprising y core N-acetylglucosamine (GIcNAc) moieties, with a compound of the formula S(F)-P in the presence of a catalyst, wherein S(F¹) is a sugar derivative comprising reactive group F¹ capable of reacting with a reactive group Q, and P is a nucleoside mono- or diphosphate, and wherein the catalyst is capable of transferring the S(F¹) moiety to the core-GicNAc moiety, to obtain a modified antibody according to structure **(26):**

AB-[(L⁶)-(F¹)_{x]y} **(26)**

wherein
- AB is a cell-binding agent
- b is 0 or 1;
- L⁶ is -GlcNAc(Fuc)_{w}-(G)ⱼ-S-(L⁷)_{w}'-, wherein G is a monosaccharide, j is an integer in the range of 0 - 10, S is a sugar or a sugar derivative, GlcNAc is N-acetylglucosamine and Fuc is fucose, w is 0 or 1, w' is 0, 1 or 2 and L⁷ is -N(H)C(O)CH₂-, -N(H)C(O)CF₂- or -CH₂-;
- F¹ is a reactive moiety;
- x is 1 or 2; and
- y is 1 or 2.

In an embodiment, the modified antibody according to structure **(26)** is reacted with a compound according to structure **(27):** 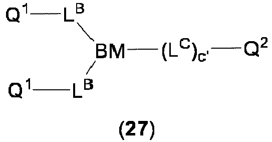 to obtain a modified antibody according to structure **(28):**

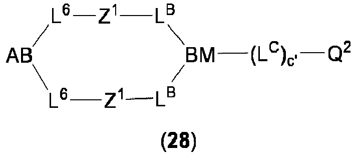

In a preferred embodiment, the modified antibody according to structure **(26)** is reacted with the compounds according to structure **(9)** to obtain a DAR2 or DAR4 conjugate or structure **(10)** to obtain a DAR1 conjugate.

Q¹-L^{M}-D (9)

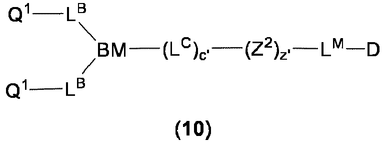

In another preferred embodiment, the modified antibody according to structure **(28)** is reacted with the compound according to structure **(11):**

F²-L^{M}-(D)ₓ **(11)**

A fourth aspect of the invention relates to the precursors that can be used to synthesize antibody-drug conjugate, the precursors are according to structure **(9), (10)** or **(11).**

Q¹-L^{M}-D (9)

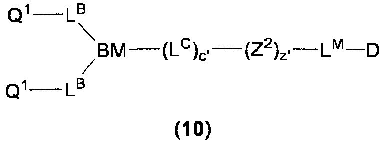

F²-L^{M}-(D)ₓ (11)

Herein F² is a moiety capable of reacting with Q² and wherein Q¹, L^{B}, BM, (L^{C})_{c'}, (Z)_{z'}, L^{M} and D are as defined above. The preferred embodiments of D, L^{M}, Z¹ and Z² described for conjugates are likewise preferred for D, L^{M} and Q¹, Q², F¹ and F² groups of the precursors.

In a preferred embodiment, the precursor of the present invention is according to structure **(9)** or **(10),** more preferably the precursor of the present invention is according to structure **(9),** wherein x = 1.

### Application

The conjugates according to the invention are especially suitable in the treatment of cancer. The compound according to structure **(1)** are furthermore suitable for the killing of cells. In that light, the invention also concerns the use of the conjugate according to the invention for the killing of cells, as well as a method for killing cells comprising the contacting of the cells with the conjugate of the present invention. The present use and method is typically *ex vivo* or *in vitro.*

The conjugates of the present invention are especially suitable in the treatment of cancer. In that light, the invention further concerns a method for the treatment of cancer, comprising administering to a subject in need thereof the conjugate according to the invention. The subject in need thereof is typically a cancer patient. The use of conjugates, such as antibody-drug conjugates, is well-known in the field of cancer treatment, and the conjugates according to the invention are especially suited in this respect. The method as described is typically suited for the treatment of cancer. In the method according to this aspect, the antibody-conjugate is typically administered in a therapeutically effective dose. The present aspect of the invention can also be worded as a conjugate according to the invention for use in the treatment of cancer. In other words, this aspect concerns the use of a conjugate according to the invention for the preparation of a medicament or pharmaceutical composition for use in the treatment of cancer. In the present context, treatment of cancer is envisioned to encompass treating, imaging, diagnosing, preventing the proliferation of, containing and reducing tumours.

This aspect of the present invention may also be worded as a method for targeting a tumour cell expressing a specific extracellular receptor, comprising contacting the conjugate according to the invention with cells that may possibly express the extracellular receptor, and wherein the antibody specifically targets the extracellular receptor. The method according to this aspect is thus suitable to determine whether the cells are expressing the desired extracellular receptor. These tumour cells may be present in a subject, in which case the method comprises administering to a subject in need thereof the conjugate according to the invention. Alternatively, the method occurs *ex vivo* or *in vitro.* In a preferred embodiment, the cells that may possibly express the extracellular receptor are cells that express the extracellular receptor. The targeting of tumour cells preferably includes one or more of treating, imaging, diagnosing, preventing the proliferation of, containing and reducing the tumour cells.

In the context of diagnosis, it is typically unknown whether the cells that are being contacted in fact express the specific extracellular receptor that is being investigated. For example, in the diagnosis of HER2-positive breast cancer, a conjugate containing an antibody that targets HER2, such as trastuzumab, may be contacted with the cells. In case the tumour cells are in fact HER2-expressing, the conjugate will target the cells, while in case the tumour cells are not HER2-expressing, the conjugate will not target the cells. Likewise, in the treatment of a cancer cells specifically expressing an extracellular receptor, the skilled person will understand that a cell-binding agent, such as an antibody, is to be used that targets that specific extracellular receptor.

In the methods of the present invention, it is preferred that the extracellular receptor is selected from the group consisting of 5T4, ADAM-9, AMHRII, ASCT2, ASLG659, ASPHD1, av-integrin, Axl, B7-H3, B7-H4, BAFF-R, BCMA, BMPR1B, Brevican, c-KIT, c-Met, C4.4a, CA-IX, cadherin-6, CanAg, CD123, CD13, CD133, CD138/syndecan-1, CD166, CD19, CD20, CD203c, CD205, CD21, CD22, CD228, CD25, CD30, CD324, CD33, CD37, CD38, CD45, CD46, CD48a, CD56, CD70, CD71, CD72, CD74, CD79a, CD79b, CEACAM5, claudin-18.2, claudin-6, CLEC12A, CLL-1, Cripto, CRIPTO, CS1, CXCR5, DLK-1, DLL3, DPEP3, E16, EGFR, ENPP3, EpCAM, EphA2, EphB2R, ETBR, FAP, FcRH1, FcRH2, FcRH5, FGFR2, fibronectin, FLT3, folate receptor alpha, Gal-3BP, GD3, GDNF-Ra1, GEDA, GFRA1, Globo H, gpNMB, GPR172A, GPR19, GPR54, guanyl cyclase C, HER2, HER3, HLA-DOB, IGF-1R, IL13R, IL20Rα, Lewis Y, LGR5, LIV-1, LRRC15, LY64, Ly6E, Ly6G6D, LY6K, MDP, MFI2, MICA/B, MOSPD2, MPF, MSG783, MUC1, MUC16, NaPi2b, NCA, nectin-4, Notch3, P-cadherin, P2X5, PD-L1, PMEL17, PRLR, PSCA, PSCA hlg, PSMA, PTK7, RET, RNF43, RON, ROR1, ROR2, Sema 5b, SLITRK6, SSTR2, STEAP1, STEAP2, TAG72, TENB2, TF, TIM-1, TM4SF, TMEFF, TMEM118, TMEM46, transferrin, TROP-2, TrpM4, TWEAKR, receptor tyrosine kinases (RTK), tenascin. Likewise, it is preferred that the tumour cells express an extracellular receptor selected from the same group. The skilled person is capable of matching the desired extracellular receptor with a suitable cell-binding agent capable of targeting that extracellular receptor.

In this light, the invention also concerns a pharmaceutical composition comprising the conjugate according to the invention and a pharmaceutically acceptable carrier. The pharmaceutical composition typically contains the conjugate according to the invention in a pharmaceutically effective dose. In a preferred embodiment, the conjugate according to the invention is present in the pharmaceutical composition in a dose of 100 µg to 10 mg per kg body weight of the subject to be treated.

The inventors have surprisingly found that the conjugates according to the invention are superior to conventional conjugates having a toxin derived from PBD, in terms of safety and/or efficacy, such that the therapeutic index of the antibody-conjugate according to the invention is increased with respect to conventional PBD-containing conjugates. In view of the reduced toxicity of the compounds according to structure (1), especially the safety of the conjugates according to the present invention is improved. As such, higher doses of the conjugate may be administered to the subject in need thereof, which in turn has further benefits in the treatment. Conventional conjugates of PBD with cell-binding agents such as antibodies need to be administered in very low doses, such that administering a too high dose is not uncommon. This may lead to aspecific cell death and thus unwanted side-effects of cancer treatment. Furthermore, administration of PBD-antibody conjugates at their conventional low doses negatively affects the biodistribution, such that the targeting of the tumour is less efficient. Hence, the inventors have found that masked PBD dimers conjugates according to the invention have a reduced toxicity, such that the therapeutic index, in particular the safety or tolerability, of the conjugates therewith is improved. Improved therapeutic efficacy of the conjugates according to the invention may take the form of a reduction in tumour size and/or a prolonged period of regression, when compared to conventional conjugates. Increase in tolerability of the conjugates according to the invention may take the form of a reduction in signs of toxicity, compared to administration of a conventional conjugate. The reduction in sings may also be referred to as a reduction In symptoms or side-effects of cancer treatment, and may involve one or more clinical signs such as reduced reduction in body weight, reduced reduction in mobility, reduced reduction in food intake and/or one or more toxicity parameters, such as improved blood chemistry, hematology, and/or histopathology.

### Examples

The invention is illustrated by the following examples.

### BCN-linker synthesis

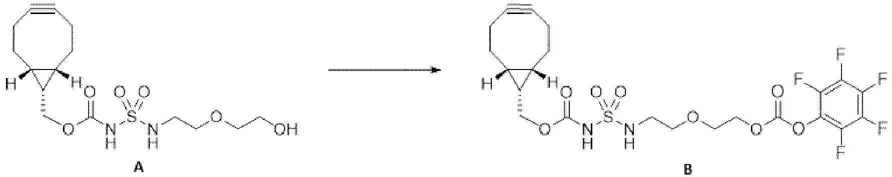

### Example 1. Synthesis of compound A

To a solution of BCN-OH (3.0 g, 20 mmol) in DCM (300 mL), under a N₂ atmosphere, was added chlorosulfonyl isocyanate (1.74 mL, 2.83 g, 20 mmol). After stirring for 15 min at ambient temperature, Et₃N (5.58 mL, 4.0 g, 40 mmol) and 2-(2-aminoethoxy)ethanol (2.2 mL, 2.31 g, 22 mmol) were added. The mixture was stirred for 15 min and quenched through addition of aqueous NH₄Cl (sat., 300 mL). After separation, the aqueous layer was extracted with DCM (200 mL). The combined organic layers were dried (Na₂SO₄), filtered and concentrated. The residue was purified with column chromatography over silicagel (0% → 10% MeOH in DCM) to give **A** as a slightly yellow oil (4.24 g, 11.7 mmol, 58%). ¹H NMR (400 MHz, CDCl₃) δ (ppm) 6.0 (bs, 1H), 4.30 (d, J = 8.2 Hz, 2H), 3.79 - 3.74 (m, 2H), 3.67 - 3.62 (m, 2H), 3.61 - 3.57 (m, 2H), 3.35 (t, J = 4.9 Hz, 2H), 2.37 - 2.15 (m, 6H), 1.63 - 1.49 (m, 2H), 1.40 (quintet, J = 8.7 Hz, 1H), 1.05-0.93 (m, 2H).

### Example 2. Synthesis of compound B

To a solution of **A** (708.4 g, 1.34 mmol, 1.0 eq) in anhydrous DMF (1.0 mL) were added bis(pentafluorophenyl) carbonate (583.4 g, 1.48 mmol, 1.1 eq) and DiPEA (0.7 mL, 519.0 mg, 4.01 mmol, 3.0 eq). After stirring for 3.5 h at ambient temperature, the reaction mixture was diluted with DCM (1.5 mL) and purified by flash column chromatograph over silicagel (0% -> 10% acetone in DCM) to give B as a clear orange oil (459.7 mg, 0.82 mmol, 61%). LCMS (ESI+) calculated for C₂₂H₂₄F₅N₂O₈S⁺ (M+H⁺) 571.49 found 571.40. ¹H NMR (400 MHz, CDCl₃) δ (ppm) 7.59 (s, 1H), 5.50 (t, *J* = 6.0 Hz, 1H), 4.51 - 4.45 (m, 2H), 4.29 (d, *J* = 8.3 Hz, 2H), 3.81 - 3.76 (m, 2H), 3.68 (dd, *J* = 5.5, 4.7 Hz, 2H), 3.34 (td, *J* = 5.7, 4.6 Hz, 2H), 2.37 - 2.16 (m, 6H), 1.63 - 1.49 (m, 2H), 1.45 - 1.37 (m, 1H), 1.00 (m, 2H).

### PBD-linker drug

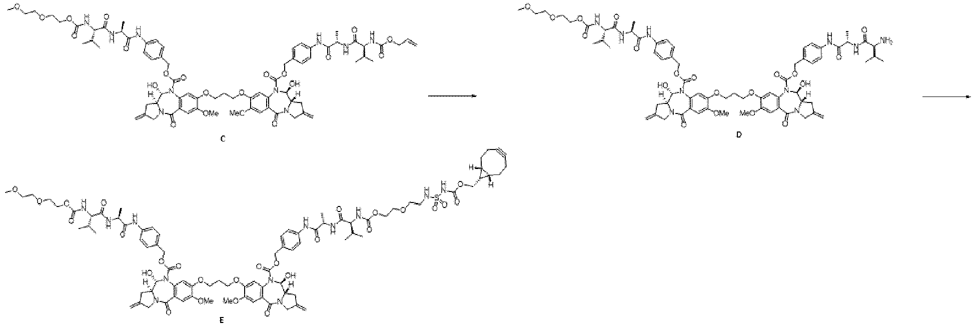

### Example 3. Synthesis of compound D

To a solution of Alloc-PBD derivative **C** (10.9 mg, 7.46 µmol, 1 eq, commercially obtained from MedChemExpress) in anhydrous DCM (500 µL, degassed with N₂) were added pyrrolidine (1.5 mg, 1.8 µL, 22.0 µmol, 2.9 eq) and Pd(PPh₃)₄ (22.5 µL, 9.95 mM, 0.22 µmol, 0.03 eq). After 35 min at ambient temperature, the reaction mixture was diluted with DCM (2.0 mL) and washed with aqueous NH₄Cl solution (sat., 1.5 mL). The two layers were separated and the aqueous layer was extracted with DCM (2x 2 mL). The combined organic layers were dried (Na₂SO₄), filtered and directly purified by flash column chromatography over silicagel (0% → 20% MeOH in DCM) to give D as a yellow solid (8.0 mg, 6.0 µmol, 80%). LCMS (ESI+) calculated for C₆₉H₈₉N₁₀O₂₀⁺ (M+H⁺) 1378.50 found 1378.01.

### Example 4. Synthesis of compound E

To a solution of **D** (8.0 mg, 6.0 µmol, 1.0 eq) in anhydrous DCM (300 µL) were added a stock of **B** in DCM (9.0 mg, 140 µL, 16.0 µmol, 116 mM, 2.7 eq) and Et₃N (2.0 mg, 2.0 µL, 20 µmol, 3.0 eq). After stirring for 6 h at ambient temperature, the reaction mixture was directly purified by flash column chromatography over silicagel (0% → 10% MeOH in DCM) to give E as an off-white solid (5.6 mg, 3.17 µmol, 54%). LCMS (ESI+) calculated for C₅₈H₁₁₀N₁₂O₂₇S⁺ (M+H⁺) 1764.92 found 1764.44.

### Preparation of antibody-drug conjugates

### General procedure for mass spectral analysis of monoclonal antibodies

Prior to mass spectral analysis, IgG was treated with IdeS, which allows analysis of the Fc/2 fragment. For analysis of the Fc/2 fragment, a solution of 20 µg (modified) IgG was incubated for 1 hour at 37 °C with IdeS/Fabricator^{™} (1.25 U/µL) in PBS Ph 7.4 in a total volume of 10 µL. Samples were diluted to 80 µL followed by analysis electrospray ionization time-of-flight (ESI-TOF) on a JEOL AccuTOF. Deconvoluted spectra were obtained using Magtran software.

### Example 6. Conjugation of azide-modified trastuzumab with compound E to obtain conjugate trast-E

A bioconjugate according to the invention was prepared by conjugation of compound E as linker-conjugate to trastuzumab-(6-N₃-GaINAc)₂ as biomolecule. To a solution of trastuzumab-(6-N₃-GaINAc)₂ (728 mL, 15.0 mg, 20.6 mg/mL in TBS Ph 7.4), prepared according to WO2016170186, was added TBS Ph 7.4 (122 mL), DMF (110 mL) compound E (40 mL, 10 mM solution in DMF). The reaction was incubated at rt overnight followed by purification on a Superdex200 Increase 10/300 GL (GE Healthcare) on an AKTA Purifier-10 (GE Healthcare). Mass spectral analysis of the fabricator-digested sample showed one major product (observed mass 26127 Da, approximately 90% of total Fc/2 fragment, calculated mass 26129 Da), corresponding to the conjugated Fc/2 fragment.

### Example 7. Conjugation of azide-modified B12 with compound E to obtain conjugate B12-E

A bioconjugate according to the invention was prepared by conjugation of compound E as linker-conjugate to B12-(6-N₃-GaINAc)₂ as biomolecule. To a solution of B12-(6-N₃-GaINAc)₂ (288 MI, 7.0 mg, 24.3 mg/mL in TBS Ph 7.4), prepared according to WO2016170186, was added TBS Ph 7.4 (108 mL), DMF (51 mL) compound E (18.7 mL, 10 mM solution in DMF). The reaction was incubated at rt overnight followed by purification on a Superdex200 Increase 10/300 GL (GE Healthcare) on an AKTA Purifier-10 (GE Healthcare). Mass spectral analysis of the fabricator-digested sample showed one major product (observed mass 26094 Da, approximately 70% of total Fc/2 fragment, calculated mass 26097 Da), corresponding to the conjugated Fc/2 fragment, and one minor product (observed mass 26223 Da, approximately 20% of total Fc/2 fragment, calculated mass 26325 Da), corresponding to the conjugated Fc/2 fragment with C-terminal lysine.

### In vivo evaluation

### Example 8. In vivo efficacy in JIMT-1 model

Female NOD/SCID mice (5- to 8-week-old at study initiation, obtained from GemPharmatech Co. Ltd., China) were inoculated subcutaneously it the right front flank region with 5 × 10⁶ JIMT-1 human breast cancer cells in 0.1 ml of PBS for tumor development. When the tumor volume was in the range of 100 to 150 mm³, groups of eight mice were injected i.v. with either vehicle, trast-E (at 1 mg/kg and 3 mg/kg), and B12-E (at 3 mg/kg). In all cases a single dose was administered on day 0. Tumor volume and body weight was measured twice per week after randomization (Figure 13). Anti-tumor efficacy was observed for trast-E at both 1 mg/kg and 3 mg/kg, but not for the non-targeting control ADC B12-E.

## Claims

1. A conjugate according to structure **(1):**
AB-[L-(D)ₓ]_{y} **(1)**
wherein
- AB is a cell-binding agent;
- x is 1 or 2;
- y is 1 or 2;
- D is a masked PBD dimer payload;
- L is a linker that connects AB to D, wherein L comprises at least one -L⁶-Z¹- fragment, wherein Z¹ is a connecting group comprising the product of a cycloaddition reaction, and wherein L⁶ is - GlcNAc(Fuc)_{w}-(G)ⱼ-S-(L⁷)_{w'}-, wherein L⁶ is connected to AB via GlcNAc(Fuc)_{w} and wherein
∘ G is a monosaccharide,
∘ j is an integer in the range of 0 - 6,
∘ S is a sugar or a sugar derivative,
∘ GicNAc is N-acetylglucosamine,
∘ Fuc is fucose,
∘ w is 0 or 1,
∘ w' is 0 or 1, and
∘ L⁷ is -N(H)C(O)CH₂-, -N(H)C(O)CF₂- or -CH₂-.

2. The conjugate according to claim 1, wherein:
- L for y = 1 and x = 1 is according to structure **(2),** and wherein L⁶ is connected to AB and L^{M} to D;
- L for y = 2 is according to structure **(3),** wherein L⁶ is connected to AB and L^{M} to D: 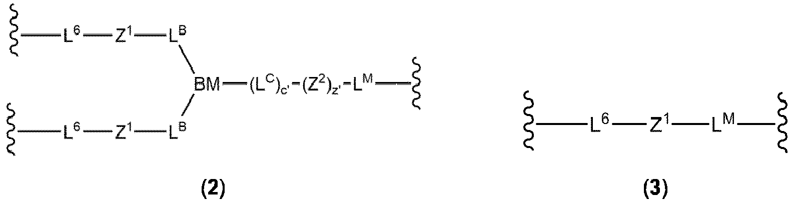
- BM is a branching moiety selected from is a carbon atom, a nitrogen atom, a phosphorus atom, a (hetero)aromatic ring, a (hetero)cycle or a polycyclic moiety;
- z' and c' are both individually 0 or 1;
- L^{B}, L^{C} and L^{M} are linkers, wherein L^{M} is of structure -(L¹)ₙ-(L²)ₒ-(L³)ₚ-(L⁴)_{q}-, wherein:
∘ L¹, L², L³ and L⁴ are each individually linkers that together link Z, L^{C} or BM to D,
∘ n, o, p and q are each individually 0 or 1, provided that p + q = 1 or 0 and o = p,
∘ linker L¹ is represented for x = 1 by: -(W)ₖ-(A)_{d}-(B)ₑ-(A)_{f}-(B)_{g}-(C(O))_{g}-,
or for x = 2 by: -(W)ₖ-(A)_{d}-(B)ₑ-(A)_{f}-(C(O))_{g}-BM[-(A)_{d'}-(B)_{e'}-(A)_{f'}-(C(O))_{g'}-]₂, wherein:
▪ d and d' are individually 0 or 1,
▪ I is 0 or 1,
▪ e and e' are individually an integer in the range 1 - 10,
▪ f and f' are individually 0 or 1,
▪ g and g' are individually an integer in the range 0 - 10,
▪ k = 0 or 1 with the proviso that if k = 1 then d = 0,
▪ preferably f + d = 1 or 2;
▪ A is a sulfamide group according to structure (L1a) 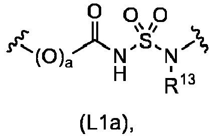 wherein a = 0 or 1, and R¹³ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups; W is -OC(O)-, -C(O)O-, -C(O)NH-, -NHC(O)-, -OC(O)NH-, -NHC(O)O-, -C(O)(CH2)ₘC(O)-, -C(O)(CH₂)ₘC(O)NH- or -(4-Ph)CH₂NHC(O)(CH₂)ₘC(O)NH-, wherein m is an integer in the range 0 - 10;
▪ B is individually selected from L², a -(CH₂)ₓ-O- or a -O-(CH₂)ₓ- moiety, or (B)ₑ is a -((CH₂)ₓ-O)ₑ₁-(CH₂)ₓ- moiety, wherein e1 is an integer in the range 1 - 10, each x is individually an integer in the range 1-10,
o linker L² is a peptide spacer;
o linker L³ is a para-aminobenzyloxycarbonyl (PABC) derivative according to structure (L3): 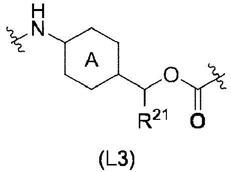 wherein
▪ ring A is an optionally substituted 5- or 6-membered aromatic or heteroaromatic ring;
▪ R²¹ is H, R²⁶ or C(O) R²⁶, wherein R²⁶ is C₁ - C₂₄ (hetero)alkyl groups, C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR₂₈ wherein R₂₈ is independently selected from the group consisting of hydrogen and C₁ - C₄ alkyl groups;
o linker L⁴ a moiety according to structure (L4): 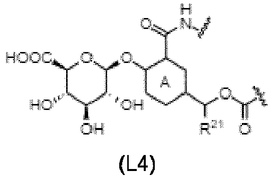 wherein ring A and R²¹ are as defined above.

3. The conjugate according to claim 1 or 2, wherein D is according to any one of structures (D1)-(D4): 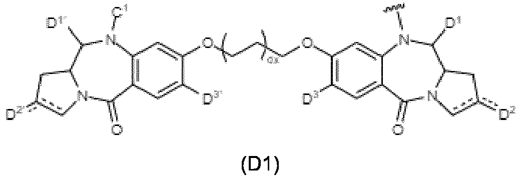 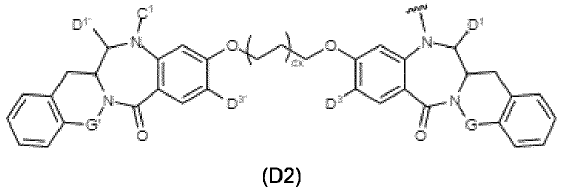 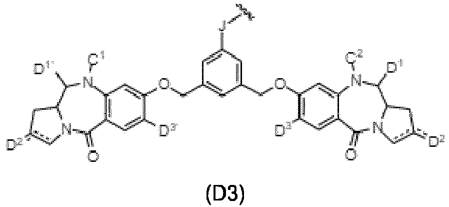 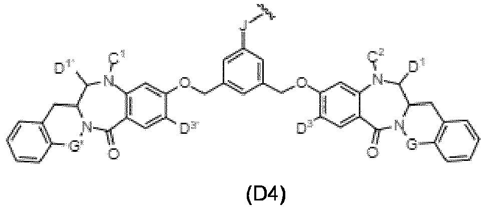 wherein:
- dx is an integer in the range of 1-10, preferably dx is 1 or 2, most preferably dx is 1;
- - - - represents a single or a double bond within the pyrrolidine ring or between the pyrrolidine ring and D²;
- D¹ and D¹' are each selected from H, OH or SO₂X, wherein X is a halogen selected from fluorine, chlorine, bromine or iodine;
- D² and D²' are each selected from -H, -OH, =CH₂ and -CH₃;
- D³ and D³' are each selected from H, OH, OMe, SMe, NMe₂, NO₂, F, Cl, Br or I, preferably D³ and D^{3'} are both OMe;
- G and G' each represents a single bond or CH₂;
- J represents a single bond, CH₂, NH or O; and
- and C² represent a capping group, preferably C¹ and C² are individually selected from the structures -L⁴-HM, -L³-L²-HM, (C1), (C2) or (C3): 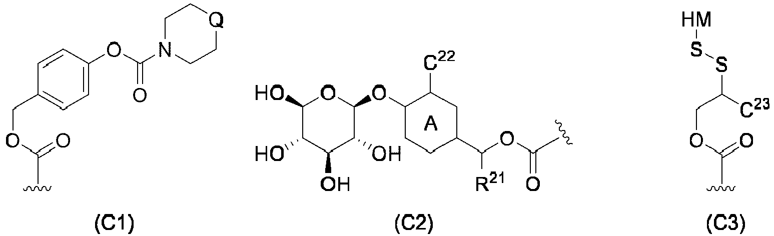 wherein
∘ L², L³ and L⁴ are as defined in claim 2, wherein L³ or L⁴ are connected to the nitrogen of D via a carbamate group and for (D3) and (D4) J = NH;
∘ HM is a hydrophilic moiety; C²² is selected from HM, NO₂ or H, more preferably C²² is selected from NO₂ or H;
∘ C²³ is H or a C₁-C₃ alkyl chain, preferably C²³ is H or methyl, more preferably C²³ is H;
∘ R²¹ is as defined in claim 2;
∘ Q is selected from O, NH, NMe or N-HM.

4. The conjugate according to any one of the preceding claims, wherein Z¹ comprises a structure selected from the structures (Z2) - (Z20) and (Z38a): 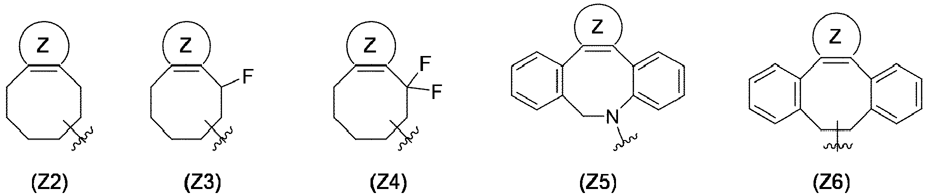 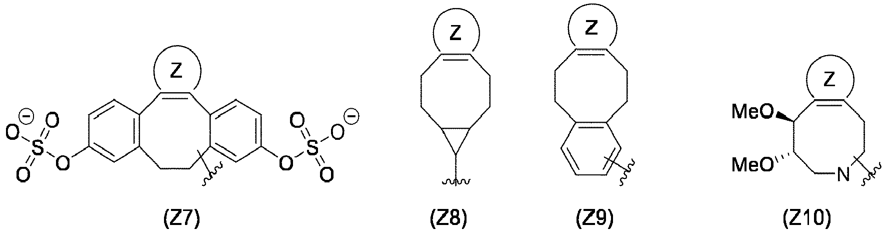 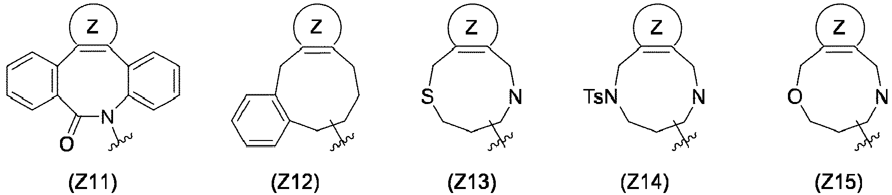 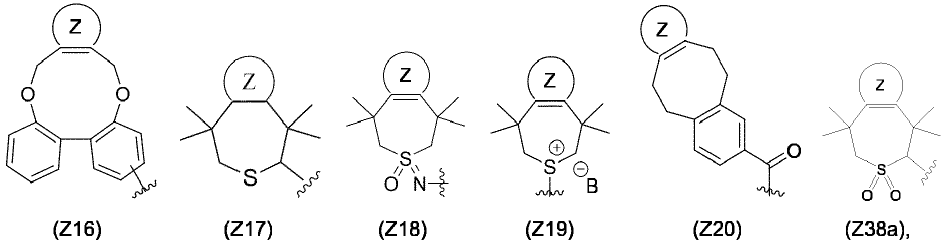 wherein the connection to L^{M} or BM is represented by the wavy bond, B⁽⁻⁾ is an anion, ring Z is selected from a triazole, a cyclohexene, a cyclohexadiene, a [2.2.2]-bicyclooctadiene, a [2.2.2]-bicyclooctene, an oxazoline, an isoxazolidine, a pyrazoline or a piperazine,
preferably Z¹ is selected from the structures (Z21) - (Z38a), more preferably Z¹ is (Z29):

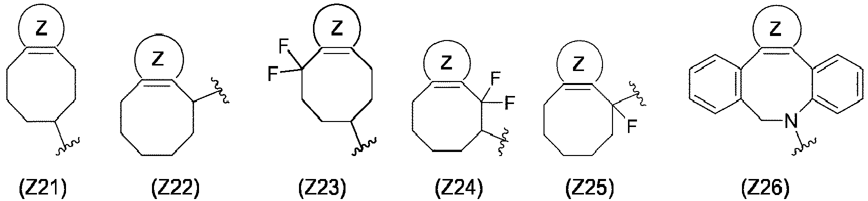

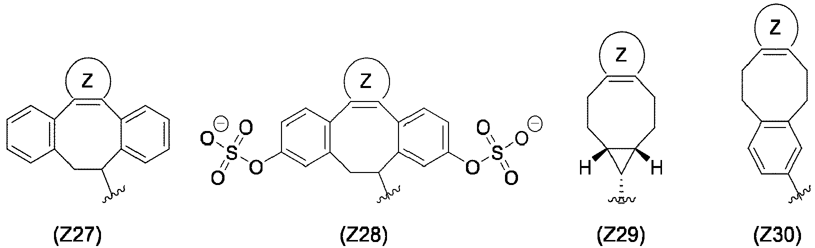

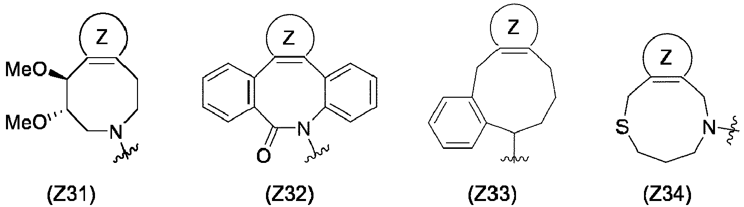

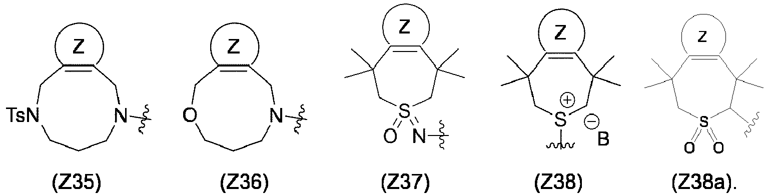

5. The conjugate according to any one of the preceding claims, wherein L^{M}-D has a structure selected from the group consisting of (LM1) - (LM4): 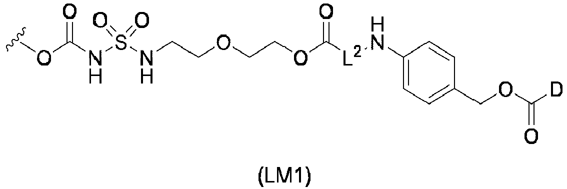 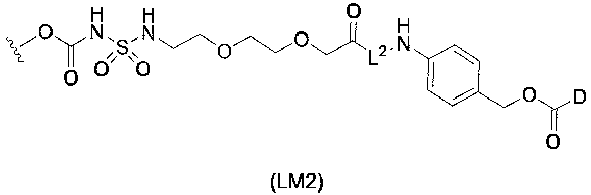 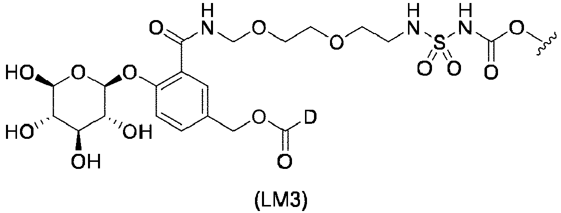 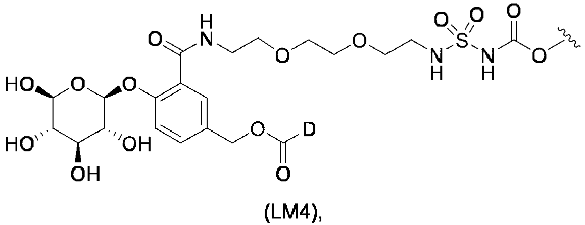 wherein the wavy line indicates the connection to Z¹, and L² is as defined in claim 2.

6. The conjugate according to any one of the preceding claims, wherein D has structure (D21):

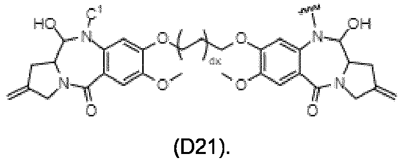

7. The conjugate according to any one of the preceding claims wherein p = 1 for linker L^{M}.

8. The conjugate according to any one of the preceding claims, wherein x = 1 and y = 2, and the conjugate has structure AB-[L-D]₂.

9. The conjugate according to any one of the preceding claims, wherein x = 1 and y = 1, and the conjugate has structure AB-[L-D], wherein L has a structure selected from (L5) - (L7):

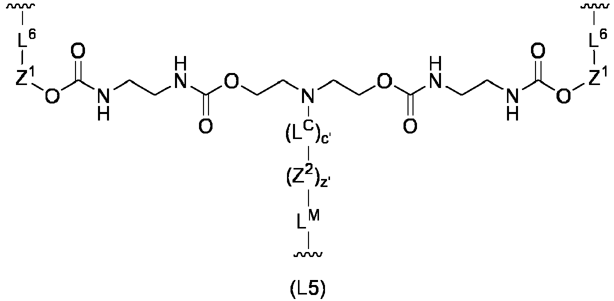

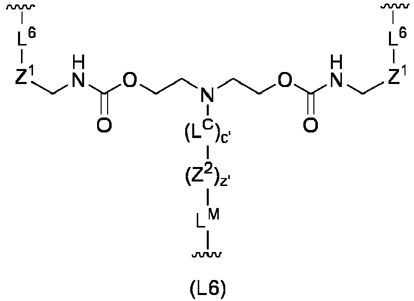

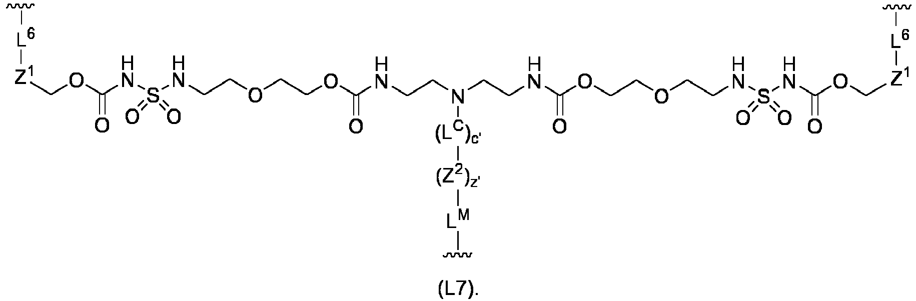

10. The conjugate according to any one of the preceding claims, wherein the linker comprises a self-immolative linker that is cleaved by a different mechanism than the self-immolative linker in the cap.

11. The conjugate according to any one of claims 1-13, wherein the conjugate has a structure selected from **(4)** - **(7):**

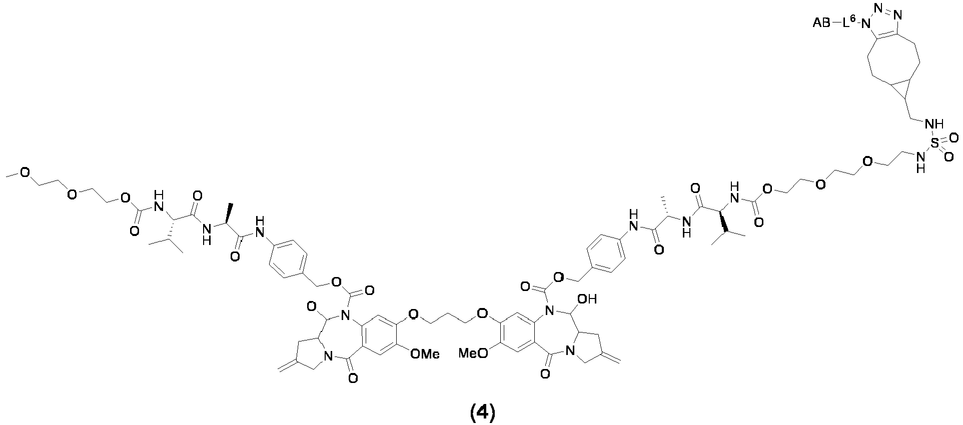

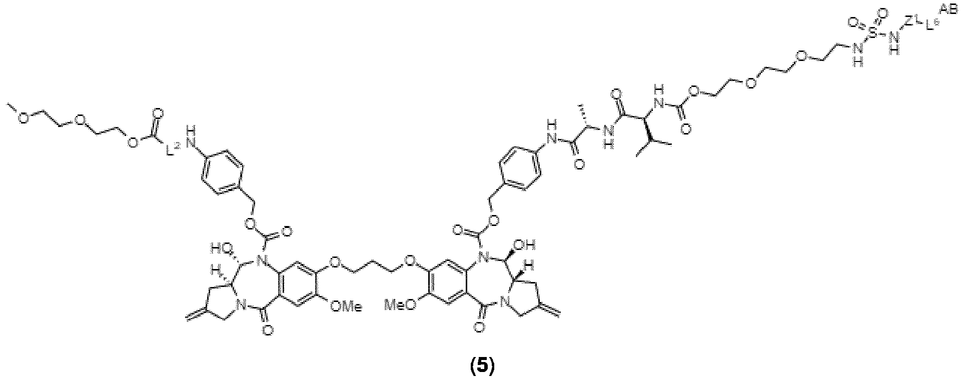

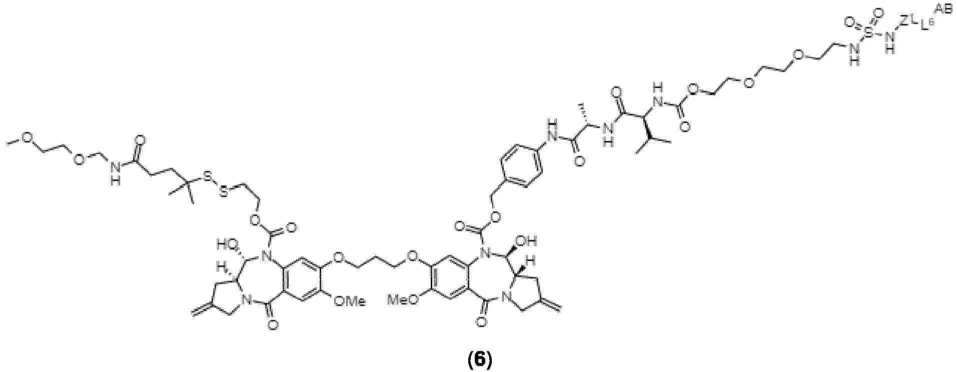

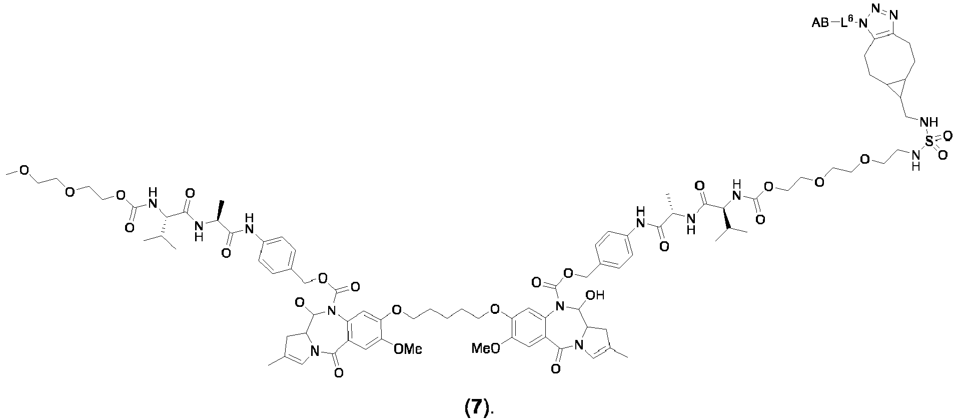

12. The conjugate according to any one of claims 1-13, wherein the conjugate has a structure **(8):** 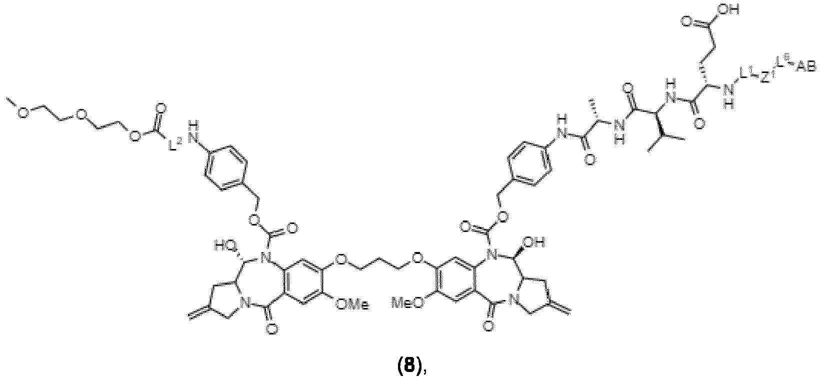 preferably wherein linker L² in the cap is selected from Glu-Gly-Cit, Glu-Gly-Val, Ala-Asn, Asn-Ala, Pro-Leu-Gly or Asn-Asn.

13. A pharmaceutical composition comprising 100 µg/kg to 10 mg/kg of a conjugate according to any one of the preceding claims and one or more pharmaceutically acceptable excipients.

14. A conjugate or pharmaceutical composition according to any one of the preceding claims for the use in medical treatment, preferably in cancer treatment.

15. A process for preparing the conjugate according to any one of claims 1-12, comprising:
i) contacting a cell-binding agent comprising y core N-acetylglucosamine (GlcNAc) moieties, with a compound of the formula S(F)ₓ-P in the presence of a catalyst, wherein S(F¹)ₓ is a sugar derivative comprising x reactive groups F¹ capable of reacting with a reactive group Q¹, and P is a nucleoside mono- or diphosphate, and wherein the catalyst is capable of transferring the S(F¹)ₓ moiety to the core-GIcNAc moiety, to obtain a modified antibody according to Formula **(26):**
AB-[(L₆)-S{F¹}ₓ]_{y} **(26)**
wherein
- AB is a cell-binding agent
- b is 0 or 1;
- L⁶ is -GlcNAc(Fuc)_{w}-(G)ⱼ-S-(L⁷)_{w'}-, wherein G is a monosaccharide, j is an integer in the range of 0 - 10, S is a sugar or a sugar derivative, GlcNAc is N-acetylglucosamine and Fuc is fucose, w is 0 or 1, w' is 0, 1 or 2 and L⁷ is -N(H)C(O)CH₂-, -N(H)C(O)CF₂- or -CH₂-;
- F¹ is a reactive moiety;
- x is 1 or 2; and
- y is 1 or 2;
ii) optionally reacting the modified antibody according to formula **(26)** with a compound represented by formula **(27):** 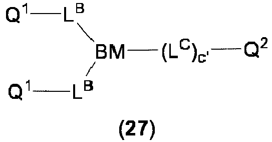 to obtain a modified antibody according to Formula **(28):** 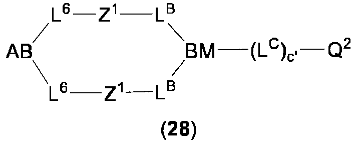
iii) reacting, preferably via a 1,3-dipolar cycloaddition, the modified antibody according to Formula **(26)** or **(28)** with the compound selected from structures **(9), (10)** and **(11),** to obtain the antibody-conjugate according to structure **(1)**
Q¹-L^{M}-D (9) 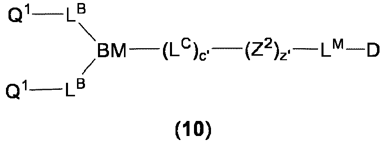
F²-L^{M}-(D)ₓ (11),
wherein if optional step ii) is performed, the compound used in step iii) is represented by structure **(11),** and if optional step ii) is not performed, the compound used in step iii) is represented by structure **(9)** or **(10).**
